(19) Europäisches Patentamt
European Patent Office
Office européen des brevets

(11) EP 0 953 357 A1

(12) **EUROPEAN PATENT APPLICATION**
published in accordance with Art. 158(3) EPC

(43) Date of publication:
**03.11.1999 Bulletin 1999/44**

(21) Application number: **97944099.7**

(22) Date of filing: **09.10.1997**

(51) Int. Cl.$^6$: **A61K 47/26**

(86) International application number:
**PCT/JP97/03642**

(87) International publication number:
**WO 98/31392 (23.07.1998 Gazette 1998/29)**

(84) Designated Contracting States:
**AT BE CH DE DK ES FI FR GB GR IE IT LI LU MC NL PT SE**

(30) Priority: **17.01.1997 JP 1971497**

(71) Applicants:
- **Drug Delivery System Institute, Ltd.**
  **Kawasaki-shi, Kanagawa-ken (JP)**
- **MEIJI SEIKA KAISHA LTD.**
  **Chuo-ku Tokyo (JP)**
- **Asahi Kasei Kogyo Kabushiki Kaisha**
  **Osaka-shi, Osaka 530-8205 (JP)**

(72) Inventors:
- **SUZUKI, Kokichi;**
  **Pharm. Res. Center, Meijii Seika**
  **Yokohama-shi; Kanagawa 222 (JP)**
- **ITO, Teruomi**
  **Asahi Kasei Kogyo Kabushiki Kaisha**
  **Shizuoka 410-23 (JP)**
- **ANDO, Takashi;**
  **Pharm. Res. Center, Meijii seika**
  **Yokohama-shi; Kanagawa 222 2 (JP)**
- **TOMA, Kazumori**
  **Asahi Kasei Kogyo Kabushiki Kaisha**
  **Shizuoka 416 (JP)**
- **SUSAKI, Hiroshi**
  **Tokyo R+D Center, Daiichi**
  **Edogawa-ku Tokyo 134 (JP)**
- **OKUNO, Satoshi;**
  **Lead Optim. Res. Lab.,Tanabe**
  **Toda-shi, Saitama 335 (JP)**
- **WATANABE, Hiroshi**
  **Meiji Seika Kaisha, Ltd.**
  **Tokyo 104 (JP)**

(74) Representative: **Kyle, Diana**
**Elkington and Fife**
**Prospect House**
**8 Pembroke Road**
**Sevenoaks, Kent TN13 1XR (GB)**

## (54) NEPHROTROPIC DRUGS

(57) Disclosed are a drug having renal selectivity and a drug carrier for specifically transporting a drug supported thereon to a kidney. A segment structure specifically recognizable in the kidney is utilized. More specifically, since a segment structure represented by formula (I) is kidney-oriented, introduction of a drug into a molecule having the segment structure can provide a renal targeting drug. A compound, which has the segment structure and can support a drug thereon, can be utilized as a drug carrier which can specifically transport a drug supported thereon to the kidney.

$$A\text{-}U\text{-}V\text{-} \qquad (I)$$

wherein A represents glycosyl, such as glucosyl, mannosyl, or 2-deoxy-glucosyl; U represents O, S, or NH; and V represents an aromatic hydrocarbon or a straight-chain or branched $C_{1\text{-}18}$ aliphatic hydrocarbon.

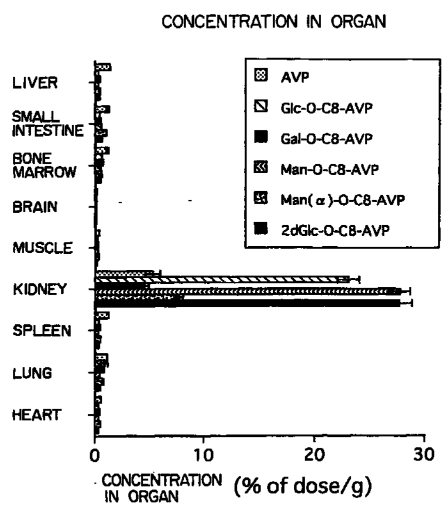


FIG. 1

EP 0 953 357 A1

## Description

### BACKGROUND OF THE INVENTION

#### Field of the Invention

The present invention relates to a renal targeting drug having a structure, recognizable in a kidney, comprising sugar and lipid, and a drug carrier having the structure recognizable in the kidney.

#### Background Art

The kidney is an organ which maintains the constancy of living bodies through transportation of various materials and production of hormones. Therefore, the hypofunction of the kidney is often causative of the lethal state of living bodies. This has lead to widespread development of diagnostic methods and therapies for renal diseases. Regarding diagnostic methods, for example, renal biopsy, biochemical examination, and image diagnosis have become widely used. On the other hand, regarding therapies, for example, pharmacotherapy, alimentary therapy, dialysis, and renal transplantation have been progressed. Despite the progress of diagnostic methods and therapies, the total number of patients suffering from renal failure has been increased. Further, an increase in the number of patients, which undergo kidney dialysis due to chronic renal failure, has also been reported. The dialysis disadvantageously imposes a heavy burden on patients. Further, at the present time, renal transplantation, which is recognized as the primary therapy, is carried out for limited patients due to problems such as biocompatibility.

Drugs, which are currently in use for the kidney, include steroid agents, anticancer agents, immunosuppressive agents, anticoagulants and antiplatelets, antiphlogistics, antihypertensive agents, diuretics, and antidiuretics. The above facts, however, clearly indicate that development of better drugs has been desired in the art.

Under these circumstances, an attempt to target drugs to the kidney by utilizing the physiological function of organ-specific metabolic enzyme systems and transportation system of the kidney has been made with a view to enhancing the efficacy of drugs in the kidney and alleviating side effect.

A representative method for targeting of drugs to the kidney is to utilize enzymes localized in the kidney, and there are a large number of reports on this method. For example, utilization of enzymes localized in the kidney, such as γ-glutamyltranspeptidase (Drieman, J.C., et al., Br. J. Pharmacol., 108, 204-208, 1993), phosphatase (Casagrande, C., et al., J. Cardiovasc. Pharmacol. 14 (Suppl. 8), S40-S59, 1989), and β-lyase (Elfarra, A. A., et al., J. Pharmacol. Exp. Ther., 274, 1289-1304, 1995), has been attempted.

Modification of a low-molecular weight protein carrier with a drug has been also reported. According to this method, a drug is accumulated in the kidney through such a mechanism that the low-molecular weight protein, after it is subjected to glomerular filtration and then excreted as primitive urine, is again absorbed in renal tubules (Franssen, E. J. F., et al., Adv. Drug Delivery Rev., 14, 67-88, 1994).

Further, regarding "drug carriers capable of recognizing the kidney," a method is also known wherein the surface charge of liposome/emulsion is regulated to allow the liposome to stay in the kidney (particularly in cortical glomerui) (Japanese Patent Laid-Open No. 27030/1996). The amount of liposome accumulated in the kidney, however, only about twice that in the case of the control. Further, in this publication, the amount of liposome accumulated in the kidney is not compared with the amount of liposome accumulated in other organs such as liver.

On the other hand, the elucidation of the mechanism, through which sugar is recognized in living bodies, has been advanced, and various studies have also been made on drug transportation utilizing the sugar recognition mechanism. For example, targeting to liver through an asialoglycoprotein receptor (Franssen, E. J. F. et al., Biochem. Pharmacol., 45, 1215-1226, 1994) and targeting to large intestine (Nolen III, H., et at., J. Pharm. Sci., 84, 677-681, 1995) have been reported. Among them, regarding the organ-specific drug transportation, at the present time, only the transportation to the liver utilizing the asialoglycoprotein receptor has been demonstrated to have a possibility that it is put to practical use (Fallon, R.J. and Schwartz, A.L., Adv. Drug Delivery Rev., 4, 49-63, 1989). Regarding the transportation of drugs to the kidney through the utilization of sugar, modification of enzymes with mannose has been reported although this is in under basic research. Specifically, the pharmacokinetics of sugar derivatives of superoxide dismutase, which is an enzyme capable of eliminating active oxygen and is a promising pharmaceutical, has been studied, and there is a report that the incorporation of a mannose-modified product in the kidney has been increased although the degree of the increase is small (Mihara, K., et al., Biol. Pharm. Bull., 17, 296-301, 1994 ). A mannose receptor is assumed to be involved in this incorporation. However, details thereof are unknown. Applicability thereof to other drugs are also not reported. Further, there is a report on sugar-modified peptides for targeting to organs and for prolongation of activity duration. Although the duration of antidiuretic activity could have been prolonged by the sugar-modified peptides, there is no description on the distribution in organs (Japanese Patent Laid-Open No. 135995/1994).

Drug transportation by targeting utilizing enzymes localized in the kidney has been reported on only limited

drugs, such as dopamine (Stella, V.J. & Himmelstein, K. J., J. Med. Chem., 23, 1275-1282, 1980), 6-mercaptopurine (Hwang, I. Y., et at., J. Pharmacol. Exp. Ther., 264, 41-46, 1993), sulfamethoxazole (Orlowski, M., et at., J. Pharmacol. Exp. Ther., 212, 167-172, 1979), and p-nitroaniline (Drieman, J. C., et al., Br. J. Pharmacol., 108, 204-208, 1993). This is considered attributable to strict structure requirement for compounds as the substrate for enzymes. Further, according to this method, after the compounds are mainly distributed through passive diffusion, a part thereof is activated. This disadvantageously results in very low proportion of the activator to the dose.

On the other hand, in the system utilizing the reabsorption of the low-molecular weight protein, it is difficult to prepare compounds having constant quality as protein modifiers, and the protein modifiers are foreign to the living body, posing a problem of antigenicity.

Thus, means for transporting a contemplated drug to the kidney in a stable and safe manner is still desired in the art.

## SUMMARY OF THE INVENTION

The present inventors have now found that a structure comprising sugar and lipid has kidney selectivity and that supporting a drug on this structure enables the drug to be specifically transported to the kidney. More specifically, they have found that the introduction of a specific structure recognizable in the kidney into the drug can vary the distribution of the drug conjugate in the body and can increase the amount of the drug transported to the kidney. The present invention has been made based on such finding.

Accordingly, it is an object of the present invention to provide a drug carrier which can specifically transport the drug to the kidney.

It is another object of the present invention to provide a drug to which kidney selectively has been imparted.

It is a further object of the present invention to provide a novel compound having kidney selectivity.

According to an aspect of the present invention, there is provided a drug carrier having in its molecule a segment structure represented by formula (I), and said drug carrier having function to specifically transport a drug supported thereon to a kidney:

$$A\text{-}U\text{-}V\text{-} \qquad (I)$$

wherein

A represents glycosyl represented by formula (II)

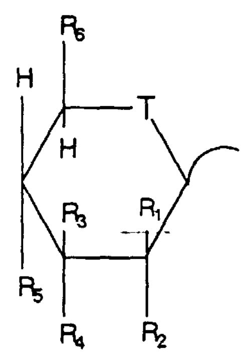

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH, and

V represents an optionally substituted $C_{6-18}$ aromatic hydrocarbon or a straight or branched, optionally substituted $C_{1-18}$ aliphatic hydrocarbon.

According to a preferred embodiment of the present invention, the drug carrier is represented by formula (III):

$$A\text{-}U\text{-}V\text{-}Y \qquad (III)$$

wherein

A, U, and V are as defined above in connection with formula (I); and
Y represents a segment structure which can support a drug thereon.

According to still another aspect of the present invention, there is provided a renal targeting drug having in its molecule a segment structure represented by formula (I):

$$A\text{-}U\text{-}V\text{-} \qquad (I)$$

wherein

A represents glycosyl represented by formula (II)

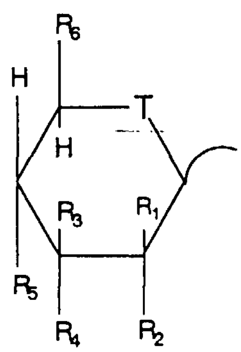

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight-chain or branched, optionally substituted $C_{1-18}$ aliphatic hydrocarbon.

According to a preferred embodiment of the present invention, the renal targeting drug is represented by formula (IV):

$$A\text{-}U\text{-}V\text{-}X\text{-}Z \qquad (IV)$$

wherein

A, U, and V are as defined above in connection with formula (I);
X represents a binding group; and

Z represents a drug.

According to a further aspect of the present invention, there is provided a novel compound represented by formula (III-a):

$$A^*\text{-}U\text{-}V\text{-}Y^* \qquad \text{(III-a)}$$

wherein

A* represents 2-deoxy-D-glucosyl, 5-deoxy-5-thio-D-glucosyl, or 5-amino-5-deoxy-D-glucosyl;
U represents O, S, or NH;
V represents a $C_{6-18}$ aromatic hydrocarbon or a straight-chain or branched $C_{1-18}$ aliphatic hydrocarbon; and
Y* represents carboxyl, methoxycarbonyl, hydroxyl, thiol, amino, aldehyde, sulfonic ester, or a halogen atom.

According to a still further aspect of the present invention, there is provided a novel compound represented by formula (IV-a):

$$A\text{-}U\text{-}V\text{-}X^*\text{-}Z^* \qquad \text{(IV-a)}$$

wherein

A, U, and V are as defined above in connection with formula (I);
X* represents -CONH-, -COO-, -OCOO-, or -CO-J-O-wherein J represents an amino acid residue; and
Z* represents a residue of arginine vasopressin, oxytocin, tryptamine, doxorubicin, dexamethasone, 4-(2-hydroxyethyl)amino-7-nitrobenz-2-oxa-1,3-diazole, or 4-(2-aminoethyl)amino-7-nitrobenz-2-oxa-1,3-diazole,
provided that compounds, wherein U represents O and Z represents arginine vasopressin or oxytocin, are excluded.

## BRIEF DESCRIPTION OF THE DRAWINGS

Fig. 1 is a graph showing the concentration of AVP supported on the drug carrier of the present invention in organs, obtained in Experiment Example 1; and
Fig. 2 is a graph showing organ clearance of AVP supported on the drug carrier of the present invention, obtained in Experiment Example 1.

## DETAILED DESCRIPTION OF THE INVENTION

### Drug carrier and renal targeting drug

The drug carrier according to the present invention has in its molecule a segment structure represented by formula (I), and can specifically transport a drug supported thereon to the kidney. Although the following mechanism is merely hypothetical and the present invention should not be limitedly interpreted thereby, a possible mechanism for the kidney selectivity is as follows. It is believed that the segment structure, represented by formula (I), comprising sugar and lipid is recognized by some recognition structure present in the kidney. As is apparent from the experiment examples described below, this kidney selectivity does not occur in a segment structure comprising any one of sugar and lipid, that is, without presence of both sugar and lipid. Up to now, the presence of any mechanism, through which the segment structure represented by formula (I) is specifically recognized in the kidney, has not been reported.

In formula (I), A represents glycosyl represented by formula (II). The glycosyl is particularly preferably D-glycosyl (in formula (II), $R_1$ represents H, $R_2$ represents OH, $R_3$ represents OH, $R_4$ represents H, $R_5$ represents OH, and $R_6$ represents $CH_2OH$), D-mannosyl (in formula (II), $R_1$ represents OH, $R_2$ represents H, $R_3$ represents OH, $R_4$ represents H, $R_5$ represents OH, and $R_6$ represents $CH_2OH$), or 2-deoxy-D-glucosyl (in formula (II), $R_1$ and $R_2$ represent H, $R_3$ represents OH, $R_4$ represents H, $R_5$ represents OH, and $R_6$ represents $CH_2OH$).

Further, in formula (I), U represents O, S, or NH with S and NH being preferred.

In formula (I), A is preferably in D form, and the glycoside linkage is preferably β linkage.

In formula (I), V represents an optionally substituted $C_{6-18}$ aromatic hydrocarbon, or a straight-chain or branched, optionally substituted $C_{1-18}$ aliphatic hydrocarbon.

According to a preferred embodiment of the present invention, V is preferably a $C_{1-18}$ aliphatic hydrocarbon, more preferably a $C_{3-15}$ straight-chain aliphatic hydrocarbon or arylene, such as phenylene. Preferred examples of straight-chain aliphatic hydrocarbons usable herein include pentamethylene, hexamethylene, heptamethylene, octamethylene, nonamethylene, decamethylene, and undecamethylene. One or more hydrogen atoms on the aromatic hydrocarbon (for example, on the aromatic ring) and the aliphatic hydrocarbon may be substituted. Substituents usable herein include: lower alkyl; lower alkenyl; lower alkynyl; hydroxyl; lower alkoxy; lower acyl; lower acyloxy; lower alkoxycarbonyl; amino optionally substituted by lower alkyl; thiol; lower alkylthio; halogen atom; halo lower alkyl (for example, trifluoromethyl); aryl; lower acylamino; nitro; cyano; and sulfonic ester (for example, methanesulfonyloxy and p-toluenesulfonyloxy). The lower alkyl as a group or a part of a group is preferably $C_{1-6}$ alkyl. The lower alkenyl and the lower alkynyl are preferably $C_{2-6}$ alkenyl and $C_{2-6}$ alkynyl, respectively.

The drug carrier according to the present invention specifically transports a drug supported thereon to the kidney. The term "supported" used herein refers to such a state that the segment structure represented by formula (I) and a drug are physically or chemically linked to each other, preferably linked to each other through a chemical bond, so that the drug can be specifically transported to the kidney.

Thus, one specific embodiment of the drug carrier according to the present invention is such that a segment structure, which can support a drug thereon, has been introduced into the segment structure represented by formula (I), that is, the drug carrier represented by formula (III).

In formula (III), Y represents a segment structure which makes it possible to support a drug on the segment structure represented by formula (I).

Examples of Y which makes it possible to physically support a drug on the segment structure represented by formula (I) include a liposoluble group which is introduced into liposome supporting a drug and permits this liposome to be physically linked to the segment structure represented by formula (I).

On the other hand, examples of Ys which make it possible to chemically support a drug on the segment structure represented by formula (I) include a segment structure having a functional group which is chemically linkable to the group present in the drug. Examples thereof include reactive groups, such as carboxyl, methoxycarbonyl, hydroxyl, thiol, amino, aldehyde, sulfonic ester, and halogen atom. Reactive derivatives thereof include, for example, in the case of carboxylic acid, conventional reactive derivatives, such as active ester derivatives, acid anhydride derivatives, halogen derivatives, and azide derivatives.

Drugs supportable on the drug carrier according to the present invention include physiologically active compounds, diagnostic agents, examination agents, and contrast media that are materials capable of exhibiting contemplated effect upon being transported to the kidney. Therefore, the term "drug" used herein refers to not only pharmaceuticals which have physiological activity and are used in prevention or treatment of diseases in animals including human beings, but also materials which, upon transportation to the kidney, can exhibit contemplated effects, for example, diagnosis of renal function, examination drugs, and contrast media. The present invention is especially advantageous in that the renal selectivity can be imparted to the conventional or novel drugs which, although exerting the contemplated effect in the kidney is expected, the distribution thereof in other organs lead to a problem in use due to a reduction in effect or development of side effects.

Examples of drugs usable herein include diagnostic drugs, examination drugs, contrast media, steroidal agents, carcinostatic agents, immunosuppressive agents, anticoagulants/antiplatelets, anti-inflammatory drugs, antihypertensive drugs, diuretics, antidiuretics, physiologically active peptides, useful genes, and antisense drugs. More specific examples of drugs usable herein include dexamethasone, prednisolone, cortisone, hydrocortisone, fludrocortisone, triamcinolone, triamcinolone acetonide, betamethasone, paramethasone, methylprednisolone, cortisone acetate, sodium hydrocortisone succinate, sodium hydrocortisone phosphate, fludrocortisone acetate, triamcinolone acetate, sodium dexamethasone phosphate, sodium dexamethasone metasulfobenzoate, dexamethasone acetate, dexamethasone palmitate, paramethasone acetate, sodium betamethasone phosphate, betamethasone acetate, halopredone acetate, sodium prednisolone succinate, prednisolone acetate, prednisolone butyl acetate, sodium prednisolone phosphate, prednisolone methyl acetate, sodium prednisolone methyl succinate, fluorometholone, doxorubisin, 5-fluorouracil, sulfamethoxazole, dopamine, naproxane, acetazolamide, furosemide, captopril, theophylline, dipyridamole, vasopressin, oxytocin, vitamin E, vitamin D, interferon β gene, tumor necrosis factor (TNF) gene, and antisense RNA of cytomegalo virus growth inhibitor.

Introduction of drugs into drug carriers according to the present invention can provide renal targeting drugs according to the present invention. At the outset, the renal targeting drug according to the present invention has in its molecule a segment structure represented by formula (I).

Further, the renal targeting drug according to a specific embodiment of the present invention is represented by formula (IV). In formula (IV), X represents a binding group which links the segment structure represented by formula (I) to the drugs Z through a chemical bond. The binding group X is not particularly limited so far as it is not substantially detrimental to the renal selectivity derived from the segment structure represented by formula (I).

X may be one which is chemically or enzymatically cleaved in vivo or alternatively may be one which is not

chemically or enzymatically cleaved. Specifically, selection of X can provide the so-called drug in the form of a prodrug (or drug conjugate) wherein the cleaved drug exhibits contemplated effect. On the other hand, when the segment structure represented by formula (I) is not cleaved from the drug, a drug can be provided which, in the form of a conjugate of the kidney recognition structure represented by formula (I) with the drug, can exhibit contemplated effect.

More specific examples of the binding group X include binding groups formed as a result of chemical bonding between Y in formula (III) and the functional group in the drug and binding groups formed as a result of binding thereof through formaldehyde, acetaldehyde or the like. Specific examples thereof include binding groups selected from the group consisting of -C(= O)O-, -OC(= O)-, -O(C = O)O-, -C(= O)S-, -SC(= O)-, -C(= O)NH-, -C(= O)N<, -NHC(= O)-, -OC(= O)NH-, -OC(= O)N<, -NHC(=O)O-, -CH = N-, -N = CH-, -O-, -NHC(=O)NH-, -NHC(= O)N<, -NH-, -N<, -OCH$_2$O-, -OCH(CH$_3$)O-, -SCH$_2$O-, - SCH(CH$_3$)O-, -OCH$_2$S-, -OCH(CH$_3$)S-, -OCH$_2$NH-, -OCH(CH$_3$)NH-, - OCH$_2$N<, -OCH(CH$_3$)N<, -NHCH$_2$O-, -NHCH(CH$_3$)O-, -SCH$_2$NH-, - SCH(CH$_3$)NH-, -SCH(CH$_3$)N<, -C(= O)OCH$_2$O-, -C(= O)OCH(CH$_3$)O-, -C(= O)OCH$_2$NH-, -C(= O)OCH(CH$_3$)NH-, -C(= O)OCH$_2$N<, -C(= O)OCH(CH$_3$)N<, -C(=O)OCH$_2$S-, -C(=O)OCH(CH$_3$)S-, -NHCH$_2$OC(=O)-, -NHCH(CH$_3$)OC(= O)-, -NHC(= O)OCH$_2$O-, -NHC(= O)OCH(CH$_3$)O-, -C(= O)OCH$_2$OC(= O)NH-, -C(= O)OCH(CH$_3$)OC(= O)NH-, and -C(= 0)-J-O- wherein J represents an amino acid residue. Examples of amino acid residues usable herein include, but are not limited to, alanine, glycine, leucine, and isoleucine.

According to another embodiment of the present invention, the binding group X may have a structure represented by formula -X,-$X_2$-$X_3$- wherein $X_1$ and $X_3$ each independently represent a binding group and $X_2$ represents a spacer structure. The spacer structure can further improve the control of dissolution, stability and other properties of the renal targeting drug, the control of liberation rate of the drug, and the recognition in the kidney. The binding group X capable of providing the spacer structure can be formed by binding Y in formula (III) to the functional group in the drug through a compound having two or more functional groups. Specifically, the binding group -$X_1$-$X_2$-$X_3$- may be formed by reacting and binding one functional group in the compound having two or more functional groups with Y in formula (III) and further reacting another functional group in the compound having two or more functional groups with the functional group in the drug. Further, according to the present invention, in the reaction of the functional group in the compound having two or more functional groups with Y in formula (III) or the functional group in the drug, binding may be further carried out through formaldehyde, acetaldehyde or the like to form binding groups $X_1$ and $X_3$.

Examples of compounds having two or more functional groups usable in the present invention include amino acids, such as glycine, alanine, leucine, isoleucine, proline, and alanine, peptides, preferably peptides comprising two to six amino acids, diamines, such as ethylenediamine, dihydric alcohols, such as ethylene glycol, diethylene glycol, and triethyleneglycol, dicarboxylic acids, amino alcohols, such as ethanolamine, and carboxyl alcohol.

The adoption of these compounds having two or more functional groups results in the formation of a binding group, selected from the group consisting of -C(= O)O-, -OC(= O)-, -OC(= O)O-, -C(= O)S-, -SC(= O)-, -C(= O)NH-, -C(= O)N<, -NHC(= O)-, -OC(= O)NH-, =OC(= O)N<, -NHC(= O)O-, -CH = N-, -N = CH-, -O-, -NHC(=O)NH-, -NHC(=O)N<, -NH-, -N<, -OCH$_2$O-, -OCH(CH$_3$)O-, -SCH$_2$O-, -SCH(CH$_3$)O-, -OCH$_2$S-, -OCH(CH$_3$)S-, - OCH$_2$NH-, -OCH(CH$_3$)NH-, -OCH$_2$N<, -OCH(CH$_3$)N<, -NHCH$_2$O-, - NHCH(CH$_3$)O-, -SCH$_2$NH-, -SCH(CH$_3$)NH-, -SCH(CH$_3$)N<, -C(= O)OCH$_2$O-, -C(= O)OCH(CH$_3$)O-, -C(= O)OCH$_2$NH-, -C(= O)OCH(CH$_3$)NH-, -C(=O)OCH$_2$N<, -C(=O)OCH(CH$_3$)N<, -C(=O)OCH$_2$S-, -C(=O)OCH(CH$_3$)S-, -NHCH$_2$OC(=O)-, -NHCH(CH$_3$)OC(=O)-, -NHC(= O)OCH$_2$O, -NHC(= O)OCH(CH$_3$)O-, -C(= O)OCH$_2$OC(= O)NH-, -C(= O)OCH(CH$_3$)OC(= O)NH-, and -C(= 0)-J-O- wherein J represents an amino acid residue, as a binding group represented by formula $X_1$ or $X_3$. Examples of amino acid residues usable herein include, but are not limited to, alanine, glycine, leucine, and isoleucine. The segment structure not involved in the formation of $X_1$ and $X_3$ in the compound having two or more functional groups forms the spacer structure represented by $X_2$. In this case, as described above, the spacer structure should be selected so that the control of dissolution, stability and other properties of the renal targeting drug, the control of liberation rate of the drug, and the recognition in the kidney are further improved. It is believed that the selection of this spacer structure, specifically the selection of the compound having two or more functional groups, can be easily accomplished by a person having ordinary skill in the art without necessitating any excessive experiment.

According to a preferred embodiment of the present invention, a preferred group of compounds are a group of compounds wherein the glycosyl represented by formula (II) is D-glucosyl, D-mannosyl, or 2-deoxy-D-glucosyl, U represents S, V represents a $C_{3-15}$ straight-chain aliphatic hydrocarbon, X represents -C-(O)O- or -CO-J-O- (wherein J represents an amino acid residue), and Z represents a steroidal agent bound to X through its OH.

The renal targeting drug according to the present invention may be administered to human beings and animals other than human beings by way of any one of routes including oral and parenteral administration, such as intravenous injection, intramuscular injection, subcutaneous administration, rectal administration, or percutaneous administration, according to the kind of the drug present in its molecule. The renal targeting drug according to the present invention as such may be administered in the form of the compound. However, the renal targeting drug is preferably administered in a suitable dosage form according to administration routes. Specifically, oral preparations includes tablets, capsules, powders, granules, and syrups. Parenteral preparations include injections, such as intravenous injections and intramuscular injections, rectal agents, oleosuppositories, and aqueous suppositories. These preparations may be prepared by

conventional methods with commonly employed components, such as excipients, disintegrators, binders, lubricants, and colorants. Excipients usable herein includes, for example, lactose, glucose, corn starch, sorbit, and crystalline cellulose. Disintegrators usable herein include, for example, starch, sodiumalginate, gelatin, calcium carbonate, calcium citrate, and dextrin. Binders usable herein include, for example, dimethylcellulose, polyvinyl alcohol, polyvinyl ether, methylcellulose, ethylcellulose, gum arabic, hydroxypropylcellulose, and polyvinylpyrrolidone. Lubricants usable herein include, for example, talc, magnesium stearate, polyethylene glycol, and hydrogenated oils. Injections may also be prepared by adding buffers, pH adjustors, or stabilizers, if necessary.

The dose of the renal targeting drug according to the present invention may be properly determined by taking into consideration the kind of the supported drug.

Thus, the present invention provides a drug carrier having renal selectivity. When the present invention is viewed from a different angle, the drug carrier according to the present invention may be understood as a chemical modifier for imparting renal selectivity to drugs. Therefore, according to another aspect of the present invention, there is provided a chemical modifier comprising the above drug carrier of the present invention, for imparting renal selectivity to drugs.

Further, the drug carrier according to the present invention may be used for modification of the conventional drugs and, in addition, may be used for the preparation of novel kidney-related drugs having renal selectivity. For example, novel drugs may be prepared which, as a result of conjugation with the segment structure represented by formula (I), have physiological activity and renal selectivity as the whole molecule.

<u>Production of drug carrier and renal targeting drug</u>

The drug carrier and the renal targeting drug according to the present invention may be synthesized preferably by the following process.

At the outset, the segment structure represented by formula (I) according to the present invention may be previously synthesized and then introduced into a functional group in the contemplated drug. According to a preferred embodiment of the present invention, Y in the drug carrier represented by formula (III) may be reacted with a functional group in the drug optionally through a compound having two or more functional groups capable of providing a spacer structure to obtain a renal targeting drug represented by formula (IV).

Alternatively, a functional group in the drug may be reacted with a compound capable of providing X and optionally a compound capable of providing X having a spacer structure, followed by introduction of a compound capable of providing the structure V as the lipid moiety and, further, introduction of glycosyl, thereby obtaining a renal targeting drug represented by formula (IV).

Selection of the method used may be properly determined by taking into consideration the structure of the drug, the segment structure represented by formula (I) to be introduced, specifically the kind of a functional group present in its molecule to be protected, the kind of the compound capable of providing the spacer structure and the like.

Preferred methods for synthesizing the drug carrier represented by formula (III) will be described.

At the outset, the drug carrier represented by formula (III) may be synthesized by binding glycosyl, which has been protected by a conventional method or unprotected, to a lipid residue and removing the protective group if any.

In the production of the drug carrier represented by formula (III), glycosyl represented by formula (II) may be produced by or according to a conventional method associated with sugars. Specific glycosyl structure according to the present invention may be produced as follows.

When glycosyl represented by formula (II) is 2-deoxy-D-glucosyl, it may be synthesized, for example, by glycosylation, described below, utilizing conventional 2-deoxy-D-glucose, or by reacting, in the presence of N-iodosuccinimide, D-glucal with a lipid derivative having at one end a functional group to be bound to the drug or a precursor easily convertible to the functional group and at the other end a hydroxyl group and then reducing the reaction product with tributyltin hydride. Alternatively, 2-deoxy-D-glucosyl may be produced by glycosylation using a 2-thiosugar derivative with phenylthio being regioselectively introduced at the 2-position or a 2-selenosugar derivative with phenylseleno being regioselectively introduced at the 2-position and then reducibly removing 2-phenylthio or 2-phenylseleno using tributyltin hydride or the like.

When glycosyl represented by formula (II) is 2-deoxy-2-fluoro-D-glucosyl or 4-deoxy-4-fluoro-D-glucosyl, it may be synthesized, for example, by the following glycosylation utilizing conventional 2-deoxy-2-fluoro-D-glucose or 4-deoxy-4-fluoro-D-glucose.

Further, when glycosyl represented by formula (II) is 4-deoxy-4-fluoro-D-glucosyl, it may be synthesized by fluorination of regioselectively protected galactose with a fluorinating agent, such as diethylaminosulfatrifluoride (DAST), or introducing a halogen atom or an eliminating group, for example, an alkylsulfonic ester, such as methanesulfonyloxy, or arylsulfonic ester, such as p-toluenesulfonyloxy, into the 4-position followed by conducting fluorination with tetrabutylammonium fluoride (TBAF), cesium fluoride or the like, and then removing the protective group.

When glycosyl represented by formula (II) is 5-amino-5-deoxy-D-glucosyl, it may be synthesized by providing

conventional 5-azido-5-deoxy-1,2-o-isopropylidene-α-D-glucofranose (Carbohydrate Research, 68, 391-395, 1979), selectively reducing azido, protecting amino with a protective group, which is not influenced by various reactions in the course of the process and is easily removable in the final stage, preferably 9-fluorenylmethyloxycarbonyl, benzyloxycarbonyl, t-butoxycarbonyl or the like, removing 1,2-o-isopropylidene to give a 5-amino-5-deoxyglucopyranose derivative, and glycosylating the 5-amino-5-deoxyglucopyranose derivative as a starting compound by a properly selected glycosylation method described below.

In the production of the drug carrier represented by formula (III), the glycosylation for forming a glycoside linkage U between glycosyl A and lipid residue V may be carried out by reacting glycosyl donor compound, such as a glycosyl halide, a thioglycoside, an acylated sugar, a 1-hydroxy sugar, glycosyl imidate, a phosphoric acid derivative or the like, of which the hydroxyl group has been properly protected, with a proper lipid derivative capable of providing a structure of V in formula (I) in an inert solvent (for example, methylene chloride, acetonitrile, benzene, or tetrahydrofuran) optionally in the presence of a dehydrating agent, such as molecular sieves.

Preferably, the lipid derivative, together with the lipid structure of V, provides Y in formula (III) or provides a group easily convertible to the functional group. Therefore, in the glycosylation, if necessary, the Y-donating portion may be protected by a protective group. Further, the terminal functional group obtained by the glycosylation may be converted to a different functional group to be used as Y.

In the segment structure represented by formula (I), when moiety A-U- is O-glycoside or S-glycoside, for example, the lipid derivative may be a compound having at one end an unprotected functional group, a functional group, which has been properly protected by a selectively removable protective group, or a precursor easily convertible to the functional group, the functional group being to be bound to the drug, and having hydroxyl or thiol at the other end. Specific examples thereof include methyl 9-hydroxynonanoate, methyl 9-mercaptononanoate, 8-azido-1-octanol, 8-S-acetylthio-1-octanol and other derivatives. The reaction is preferably carried out using an activator. Specific examples of activators usable herein include silver salts, such as silver perchlorate and silver carbonate, mercury salts, such as mercuric chloride, zinc salts, such as zinc chloride, tin salts, such as stannous chloride, copper salts, such as cuprous trifluoromethanesulfonate, Lewis acids, such as boron trifluoride etherate, trimethylsilyl trifluoromethanesulfonate, tin tetrachloride, silicon tetrafluoride, titanium tetrafluoride, zirconocene dichloride, and hafnocene dichloride, oxidizing agents, such as N-bromosuccinimide, bromine, and N-iodosuccinimide-trifluoromethanesulfonic acid, and alkylating agents, such as methyl trifluoromethanesulfonate and dimethylmethylthiosulfonium trifluoromethanesulfonate (DMTST). These activators may be used alone or in combination according to the kind of the glycosyl donor.

Further, in the segment structure represented by formula (I), when moiety A-U- is O-glycoside or S-glycoside, for example, the derivative may be a compound having at one end an unprotected functional group, a functional group, which has been properly protected by a selectively removable protective group, or a precursor easily convertible to the functional group, the functional group being to be bound to the drug, and having at the other end a halogen atom or an eliminating group, for example, alkylsulfonic ester, such as methanesulfonyloxy, or arylsulfonic ester, such as p-toluenesulfonyloxy. Specific examples thereof include methyl 9-(p-toluenesulfonyloxy)nonanoate, methyl 9-bromo nonanoate, 8-bromo-1-octanol, 8-azido-1-bromooctane, 8-S-acetylthio-1-bromooctane. In this case, 1-hydroxysugar or 1-thiosugar, of which the hydroxyl group has been properly protected or unprotected, may be reacted in an inert solvent (for example, N,N-dimethylformamide, water, methanol, toluene, or acetone) in the presence of an inorganic base, such as sodium hydroxide, sodium hydrogencarbonate, or potassium carbonate, or an organic base, such as triethylamine or pyridine.

In the segment structure represented by formula (I), when moiety A-U- is N-glycoside, for example, the lipid derivative may be a compound having at one end an unprotected functional group, a functional group, which has been properly protected by a selectively removable protective group, or a precursor easily convertible to the functional group, the functional group being to be bound to the drug, and having at the other end amino in a free form or in the form of a salt with a suitable acid. Specific examples thereof include amines, such as 8-aminooctanoic acid and 8-amino-1-octanol, salts of these amines with inorganic acids, such as hydrochloric acid or bromic acid, or salts of these amines with organic acids, such as p-toluenesulfonic acid or trifluoroacetic acid. The glycosyl, together with the lipid derivative, may be reacted in a solvent, an alcohol, such as hydrous or anhydrous methanol or ethanol, tetrahydrofuran, or N,N-dimethylformamide at room temperature or under reflux with heating, optionally in the presence of an acid catalyst.

The renal targeting drug represented by formula (IV) according to the present invention may be produced preferably by the following process. Specifically, the reaction of the drug carrier represented by formula (III) according to the present invention with a drug is preferably carried out as follows.

At the outset, if necessary, hydroxyl groups of the sugar in the drug carrier represented by formula (III) according to the present invention are protected. Further, when Y is in a protected state, the protective group is removed. When Y is a precursor, the precursor is converted to a functional group to be bound to the drug. Thereafter, according to the kind of groups present in the drug, if necessary, other functional groups are protected, followed by reaction with the drug carrier represented by formula (III).

For example, when the drug carrier represented by formula (III) is bound to the drug through an ester bond,

the drug carrier represented by formula (III), wherein Y represents carboxyl, is reacted with a hydroxyl-containing drug. The reaction may be carried out in an inert solvent (for example, methylene chloride, tetrahydrofuran, toluene, or N,N-dimethylformamide) optionally together with 4-dimethylaminopyridine, for example, by dehydrocondensation using a dehydrocondensating agent, such as N,N'-dicyclohexylcarbodiimide or 1,1'-carbonyldiimidazole, or by active esterification using an active ester prepared from N-hydroxysuccinimide or the like. Alternatively, the drug carrier represented by formula (III), wherein Y represents hydroxyl, may be reacted with a carboxyl-containing drug, for example, by the dehydrocondensation or active esterification as described above.

Likewise, when the drug carrier represented by formula (III) is bound to the drug through an ester bond, the drug carrier represented by formula (III), wherein Y represents carboxyl, may be reacted with a drug derivative having a halogen atom or an eliminating group, for example, alkylsulfonic ester, such as methanesulfonyloxy, or arylsulfonic ester, such as p-toluenesulfonyloxy, in the presence of an inorganic base, such as sodium hydrogencarbonate or potassium carbonate, or an organic base, such as triethylamine or pyridine, in an inert solvent (for example, N,N-dimethylformamide, water, or methanol). Alternatively, the drug carrier represented by formula (III), wherein Y represents a halogen atom or an eliminating group, for example, alkylsulfonic ester, such as methanesulfonyloxy, or arylsulfonic ester, such as p-toluenesulfonyloxy, may be reacted with a carboxyl-containing drug by the nucleophilic displacement reaction as described above.

When the drug carrier represented by formula (III) is bound to the drug through a thioester bond or an amido bond, the drug carrier represented by formula (III), wherein Y represents carboxyl, may be reacted with a thiol- or amino-containing drug by the dehydrocondensation or active esterification as described above. Further, when Y in the drug carrier represented by formula (III) represents thiol or amino, the drug carrier may be reacted with a carboxyl-containing drug.

When the drug carrier represented by formula (III) is bound to the drug through a carbonic ester bond or a urethane bond, the drug carrier represented by formula (III), wherein Y represents hydroxyl, may be reacted with triphosgene, 1,1'-carbonyldiimidazole or the like in an inert solvent (for example, methylene chloride, tetrahydrofuran, toluene, or N,N-dimethylformamide), followed by reaction with a hydroxyl- or amino-containing drug. Likewise, when the drug carrier represented by formula (III) is bound to the drug through a carbonic ester bond or a urethane bond, a hyroxyl-containing drug may be reacted with triphosgene, 1,1'-carbonyldiimidazole or the like in an inert solvent (for example, methylene chloride, tetrahydrofuran, toluene, or N,N-dimethylformamide), followed by reaction with the drug carrier represented by formula (III) wherein Y represents hydroxyl or amino.

When the drug carrier represented by formula (III) is bound to the drug through an imino bond (including a Schiff base), the drug carrier represented by formula (III), wherein Y represents aldehyde, may be reacted with an amino-containing drug in the presence of an acid catalyst, such as hydrochloric acid, p-toluenesulfonic acid, or acetic acid, in an inert solvent (for example, methanol, chloroform, methylene chloride, tetrahydrofuran, or N,N-dimethylformamide). Alternatively, when the drug carrier represented by formula (III) is bound to the drug through an wino bond (including a Schiff base), the drug carrier represented by formula (III), wherein Y represents amino, may be reacted with an aldehyde-containing drug in the presence of an acid catalyst, such as hydrochloric acid, p-toluenesulfonic acid, or acetic acid, in an inert solvent (for example, methanol, chloroform, methylene chloride, tetrahydrofuran, or N,N-dimethylformamide).

When the drug carrier represented by formula (III) is bound to the drug through an ether bond, the drug carrier represented by formula (III), wherein Y represents a halogen atom or an eliminating group, for example, alkylsulfonic ester, such as methanesulfonyloxy, or arylsulfonic ester, such as p-toluenesulfonyloxy, may be reacted with a hydroxyl-containing drug in the presence of a base, such as sodium hydride or potassium t-butoxide, in an inert solvent (for example, N,N-dimethylformamide, tetrahydrofuran, or acetonitrile). Alternatively, when the drug carrier represented by formula (III) is bound to the drug through an ether bond, the drug carrier represented by formula (III), wherein Y represents hydroxyl, may be reacted with a drug containing a halogen atom or an eliminating group, for example, alkylsulfonic ester, such as methanesulfonyloxy, or arylsulfonic ester, such as p-toluenesulfonyloxy, in the presence of a base, such as sodium hydride or potassium t-butoxide, in an inert solvent (for example, N,N-dimethylformamide, tetrahydrofuran, or acetonitrile).

When the drug carrier represented by formula (III) is bound to the drug through a urea bond, the drug carrier represented by formula (III), wherein Y represents amino, may be reacted with triphosgene, 1,1'-carbonyldiimidazole or the like in an inert solvent (for example, methylene chloride, tetrahydrofuran, toluene, or N,N-dimethylformamide), followed by reaction with an amino-containing drug. Alternatively, the drug carrier represented by formula (III) is bound to the drug through a urea bond, an amino-containing carrier may be reacted with triphosgene, 1,1'-carbonyldiimidazole or the like in an inert solvent (for example, methylene chloride, tetrahydrofuran, toluene, or N,N-dimethylformamide), followed by reaction with the drug carrier represented by formula (III) wherein Y represents amino.

The renal targeting drug represented by formula (IV), wherein X represents -NH- or -N<, may be synthesized by reacting the drug carrier represented by formula (III), wherein Y represents a halogen atom or an eliminating group, for example, alkylsufonic ester, such as methanesulfonyloxy, or arylsulfonic ester, such as p-toluenesulfonyloxy, with a

primary or secondary amino-containing drug in the presence of an inorganic base, such as sodium hydrogencarbonate or potassium carbonate, or an organic base, such as triethylamine or pyridine, in an inert solvent (for example, N,N-dimethylformamide, water, or methanol). Alternatively, the drug carrier represented by formula (III), wherein Y represents aldehyde, and a primary or secondary amino-containing drug may be reacted with a reducing agent, such as sodium cyanoboron hydride or sodium triacetoxyboron hydride, optionally in-the presence of a suitable acid catalyst (for example, hydrochloric acid, p-toluenesulfonic acid, or acetic acid), in an inert solvent (for example, methanol, chloroform, methylene chloride, tetrahydrofuran, or N,N-dimethylformamide). Likewise, the renal targeting drug represented by formula (IV), wherein X represents -NH- or -N<, may be synthesized by reacting the drug carrier represented by formula (III), wherein Y represents primary or secondary amino, with a drug containing a halogen atom or an eliminating group, for example, alkylsufonic ester, such as methanesulfonyloxy, and arylsulfonic ester, such as p-toluenesulfonyloxy, by the nucleophilic displacement reaction described above. Alternatively, this renal targeting drug may be obtained from the drug carrier represented by formula (III), wherein Y represents primary or secondary amino, and an aldehyde-containing drug by the reductive amination described above.

When the drug carrier represented by formula (III) is bound to the drug through an N-Mannich base type bond, the drug carrier represented by formula (III), wherein Y represents amino, may be reacted with an aldehyde, such as formaldehyde and acetaldehyde, and a hydroxyl-, thiol-, amino, or activated ester-containing drug, or a drug converted to a reactive derivative of carboxylic acid, such as a mixed acid anhydride derivative and a halogen derivative, optionally in the presence of an acid catalyst, such as hydrochloric acid, p-toluenesulfonic acid, and acetic acid, in an inert solvent (for example, methylene chloride, tetrahydrofuran, or N,N-dimethylformamide). Alternatively, when the drug carrier represented by formula (III) is bound to the drug through an N-Mannich base type bond, an amino-containing drug may be reacted with an aldehyde, such as formaldehyde or acetaldehyde, and the drug carrier represented by formula (III), wherein Y represents hydroxyl, thiol, amino, or active ester, or the drug carrier, represented by formula (III), converted to a reactive derivative of carboxylic acid, such as a mixed acid anhydride derivative or a halogen-derivative, in the presence of an acid catalyst, such as hydrochloric acid, p-toluenesulfonic acid, or acetic acid, in an inert solvent (for example, methylene chloride, tetrahydrofuran, or N,N-dimethylformamide).

In formula (IV-a), when X represents -CO-J-O- wherein J represents an amino acid residue, the renal targeting drug may be produced by subjecting a hydroxyl-containing drug and an amino acid having a properly protected N end (specifically, an amino acid derivative, such as N-t-butoxycarbonyl-L-glycine, N-t-butoxycarbonyl-L-alanine, N-t-butoxycarbonyl-L-leucine, N-t-butoxycarbonyl-L-isoleucine) to the ester bond formation reaction described above to give an amino acid-drug conjugate, selectively removing the protective group of the conjugate at its N end, and reacting the resultant amino with the drug carrier represented by formula (III), wherein Y represents carboxyl, to form an amido bond.

Preferably, the compounds synthesized by the above processes are purified by a conventional method, for example, recrystallization, reprecipitation, silica gel chromatography, or column chromatography on an adsorbent resin. When a protective group is present in the final stage, this purification is carried out after the removal of the protective group.

Compounds represented by formulae (III-a) and (IV-a)

According to another aspect of the present invention, there are provided novel compounds represented by formulae (III-a) and (IV-a).

In the formula (III-a), A* represents 2-deoxy-D-glucosyl, 5-deoxy-5-thio-D-glucosyl, or 5-amino-5-deoxy-D-glucosyl. The bond between A* and U, that is, the glycoside linkage, is preferably β-linkage. Y* represents carboxyl, methoxycarbonyl, hydroxyl, thiol, amino, aldehyde, sulfonic ester, or a halogen atom. U and V are as defined above in connection with formulae (I) and (III), and preferred examples thereof include those described above in connection with formulae (I) and (III).

In formula (IV-a), A, U, and V are as defined above in connection with formulae (I) and (III), and preferred examples thereof include those described above in connection with formulae (I) and (III). More specifically, A preferably represents D-glucosyl, D-mannosyl, 2-deoxy-D-glucosyl, 5-deoxy-5-thio-D-glucosyl, or 5-amino-5-deoxy-D-glucosyl, V preferably represents a $C_{6-18}$ aromatic hydrocarbon or a $C_{1-18}$ straight-chain or branched aliphatic hydrocarbon, more preferably a $C_{3-15}$ straight-chain aliphatic hydrocarbon. X* represents -CONH-, -COO-, -OCOO-, or -CO-J-O- wherein J represents an amino acid residue. The amino acid residue represented by J is not particularly limited, and examples thereof include alanine, glycine, leucine, and isoleucine residues.

Z* represents an arginine vasopressin, oxytocin, tryptamine, doxorubicin, dexamethasone, or 7-nitrobenz-2-oxa-1,3-diazole residue.

In formula (IV-a), however, when U represents O, Z does not represent arginine vasopressin or oxytocin. That is, compounds, wherein U represents O with Z representing arginine vasopressin or oxytocin, are excluded from the compounds represented by formula (IV-a) according to the present invention.

The compounds represented by formulae (III-a) and (IV-a) may be synthesized as described above in connection with the production of the drug carrier and the renal targeting drug respective represented by formulae (III) and (IV).

## EXAMPLES

The following Examples and Tests further illustrate the present invention, but are not intended to limit it.
The following abbreviations are used in the following examples.
AVP: arginine vasopressin, Oxy: oxytocin, DXR: doxorubicin, DEX: dexamethasone, Glc: glucopyranose, Gal: galactopyranose, Man: mannopyranose, All: allopyranose, Xyl: xylopyranose, HOBt: 1-hydroxybenzotriazole, WSCI: 1-ethyl-3-(3-dimethylaminopropyl)carbodiimide, NBD: 7-nitrobenz-2-oxa-1,3-diazole, NBD-Cl: 4-chloro-7-nitrobenz-2-oxa-1,3-diazole, 2dGlu: 2-deoxyglucose, DCC: 1,3-dicyclohexylcarbodiimide, HOSu: N-hydroxysuccinimide, DMF: N,N-dimethylformamide, TFA: trifluoroacetic acid, THF: tetrahydrofuran, and DMAP: 4-dimethylaminopyridine.
Structures of compounds synthesized in the following examples are summarized as follows. Example Nos. and structures of the compounds as drug carriers represented by formula (III) according to the present invention are shown in Table 1 below.

Table 1

| Example | R1 | R2 | R3 | R4 | R5 | R6 | T | U | V | Y |
|---|---|---|---|---|---|---|---|---|---|---|
| 1 | H | OH | OH | H | OH | $CH_2OH$ | O | O | $(CH_2)_8$ | COOMe |
| 2 | H | OH | OH | H | OH | $CH_2OH$ | O | O | $(CH_2)_8$ | COOH |
| 6 | OH | H | OH | H | OH | $CH_2OH$ | O | O | $(CH_2)_8$ | COOH |
| 9 | H | H | OH | H | OH | $CH_2OH$ | O | O | $(CH_2)_8$ | COOMe |
| 10 | H | H | OH | H | OH | $CH_2OH$ | O | O | $(CH_2)_8$ | COOH |
| 17 | H | OH | OH | H | OH | $CH_2OH$ | O | S | $(CH_2)_8$ | COOMe |
| 18 | H | OH | OH | H | OH | $CH_2OH$ | O | S | $(CH_2)_8$ | COOH |
| 21 | H | OH | OH | H | OH | $CH_2OH$ | O | S | $(CH_2)_{11}$ | COOMe |
| 22 | H | OH | OH | H | OH | $CH_2OH$ | O | S | $(CH_2)_{11}$ | COOH |
| 28 | OH | H | OH | H | OH | $CH_2OH$ | O | S | $(CH_2)_8$ | COOMe |
| 29 | H | OH | H | OH | OH | $CH_2OH$ | O | S | $(CH_2)_8$ | COOMe |
| 30 | H | OH | OH | H | OH | H | O | S | $(CH_2)_8$ | COOMe |
| 31 | H | H | OH | H | OH | $CH_2OH$ | O | S | $(CH_2)_8$ | COOMe |
| 32 | H | OH | OH | H | OH | $CH_2OH$ | S | S | $(CH_2)_8$ | COOMe |
| 33 | H | OH | OH | H | OH | $CH_2OH$ | O | NH | $(CH_2)_7$ | COOMe |
| 34 | H | OH | OH | H | OH | $CH_2OH$ | O | S | $(CH_2)_8$ | OH |
| 35 | H | OH | OH | H | OH | $CH_2OH$ | O | S | $(CH_2)_8$ | SH |
| 36 | H | OH | OH | H | OH | $CH_2OH$ | O | S | $(CH_2)_8$ | $NH_2$ |
| 37 | H | OH | OH | H | OH | $CH_2OH$ | O | S | $CH_2$ | COOMe |
| 38 | H | OH | OH | H | OH | $CH_2OH$ | O | S | $(CH_2)_{15}$ | COOMe |
| 39 | H | OH | OH | H | OH | $CH_2OH$ | O | S | $CH(C_6H_{13})C_{10}H_{20}$ | COOMe |
| 40 | H | OH | OH | H | OH | $CH_2OH$ | O | S | $p-C_6H_4$ | COOMe |
| 45 | H | OH | OH | H | OH | $CH_2OH$ | NH | S | $(CH_2)_8$ | COOMe |
| 46 | H | OH | OH | H | OH | $CH_2OH$ | O | S | $(CH_2)_5$ | COOMe |
| 47 | H | OH | OH | H | OH | $CH_2OH$ | O | S | $(CH_2)_5$ | COOH |

Example Nos. and structures of the compounds as kidney-selective drugs represented by formula (IV) according to the present invention are shown in Table 2 below.

Table 2

| Example | R1 | R2 | R3 | R4 | R5 | R6 | T | U | V | Y | Z |
|---|---|---|---|---|---|---|---|---|---|---|---|
| 3 | H | OH | OH | H | OH | $CH_2OH$ | O | O | $(CH_2)_8$ | CONH | AVP |
| 7 | OH | H | OH | H | OH | $CH_2OH$ | O | O | $(CH_2)_8$ | CONH | AVP |
| 8 | OH | H | OH | H | OH | $CH_2OH$ | O | O(α) | $(CH_2)_8$ | CONH | AVP |
| 11 | H | H | OH | H | OH | $CH_2OH$ | O | O | $(CH_2)_8$ | CONH | AVP |
| 12 | H | H | OH | H | OH | $CH_2OH$ | O | O | $(CH_2)_8$ | CONH | AVP*1 |
| 13 | H | OH | OH | H | OH | $CH_2OH$ | O | O | $(CH_2)_8$ | CONH | Oxy |
| 14 | H | OH | OH | H | OH | $CH_2OH$ | O | O | $(CH_2)_8$ | CONH | Oxy*2 |
| 15 | OH | H | OH | H | OH | $CH_2OH$ | O | O | $(CH_2)_8$ | CONH | Oxy |
| 16 | OH | H | OH | H | OH | $CH_2OH$ | O | O | $(CH_2)_8$ | CONH | Oxy*2 |
| 19 | H | OH | OH | H | OH | $CH_2OH$ | O | S | $(CH_2)_8$ | CONH | AVP |
| 20 | H | OH | OH | H | OH | $CH_2OH$ | O | S | $(CH_2)_8$ | CONH | AVP*1 |
| 23 | H | OH | OH | H | OH | $CH_2OH$ | O | S | $(CH_2)_{11}$ | CONH | AVP |
| 24 | H | OH | OH | H | OH | $CH_2OH$ | O | S | $(CH_2)_{11}$ | CONH | AVP*1 |
| 25 | H | OH | OH | H | OH | $CH_2OH$ | O | S | $(CH_2)_8$ | CONH | $(CH_2)_2$-NBD |
| 26 | H | OH | OH | H | OH | $CH_2OH$ | O | S | $(CH_2)_8$ | CONH | Tryptamine |
| 27 | H | OH | OH | H | OH | $CH_2OH$ | O | S | $(CH_2)_8$ | CONH | Tryptamine |
| 41 | H | OH | OH | H | OH | $CH_2OH$ | O | S | $(CH_2)_8$ | COO | DXR |
| 42 | H | OH | OH | H | OH | $CH_2OH$ | O | S | $(CH_2)_8$ | CONH | DXR |
| 43 | H | OH | OH | H | OH | $CH_2OH$ | O | S | $(CH_2)_8$ | COO | $(CH_2)_2$-NBD |
| 44 | H | OH | OH | H | OH | $CH_2OH$ | O | S | $(CH_2)_8$ | OCOO | $(CH_2)_2$-NBD |
| 48 | H | OH | OH | H | OH | $CH_2OH$ | O | S | $(CH_2)_5$ | CONH | AVP |
| 49 | H | OH | OH | H | OH | $CH_2OH$ | O | S | $(CH_2)_5$ | CONH | AVP*1 |
| 50 | H | OH | OH | H | OH | $CH_2OH$ | O | S | $(CH_2)_8$ | COO | DEX |
| 51 | H | OH | OH | H | OH | $CH_2OH$ | O | S | $(CH_2)_5$ | COO | DEX |
| 52 | H | OH | OH | H | OH | $CH_2OH$ | O | S | $(CH_2)_8$ | CONH-Gly-COO | DEX |
| 53 | H | OH | OH | H | OH | $CH_2OH$ | O | S | $(CH_2)_8$ | CONH-Ala-COO | DEX |
| 54 | H | OH | OH | H | OH | $CH_2OH$ | O | S | $(CH_2)_8$ | CONH-Leu-COO | DEX |
| 55 | H | OH | OH | H | OH | $CH_2OH$ | O | S | $(CH_2)_8$ | CONH-Ile-COO | DEX |
| 56 | H | OH | OH | H | OH | $CH_2OH$ | O | S | $(CH_2)_5$ | CONH-Gly-COO | DEX |
| 57 | H | OH | OH | H | OH | $CH_2OH$ | O | S | $(CH_2)_5$ | CONH-Ala-COO | DEX |
| 58 | H | OH | OH | H | OH | $CH_2OH$ | O | S | $(CH_2)_5$ | CONH-Leu-COO | DEX |
| 59 | H | OH | OH | H | OH | $CH_2OH$ | O | S | $(CH_2)_5$ | CONH-Ile-COO | DEX |

Further, in the following examples, for easy understanding of structures, the above abbreviations and the like are utilized to show the name of compounds in a simplified form. It appears that the rule of the notation can be easily understood from the name of compounds appended thereto. For example, Cn represents $(CH_2)n$.

Examples 4 and 5 below show synthetic processes for comparison.

Example 1 Glc-O-$C_8$-COOMe(8-methoxycarbonyloctylβ-D-glucopyranoside)

(1) Boron trifluoride etherate (4 ml, 32 mmol) was added to a solution of 1,2,3,4,6-penta-O-acetyl-α-D-glucopyranoside (2.5 g, 8.0 mmol) and 8-methoxycarbonyl octanol (3.0 g) in methylene chloride (30 ml). The mixture was stirred at room temperature for 5 hr. The reaction solution was washed with water and dried over magnesium sulfate. After filtration, the filtrate was concentrated to dryness under the reduced pressure. The oil thus obtained was purified by column chromatography on silica gel (toluene-acetone (9 : 1)) to give 2.45 g (61%) of 8-methoxycarbonyloctyl 2,3,4,6-tetra-O-acetyl-β-D-glucopyranoside.

$[\alpha]_D^{17}$ +3.73° (c = 1.42, chloroform)
$^1$H-NMR ($CDCl_3$) δ: 1.56 (8H, s), 1.57-1.63 (4H, m), 2.01 (3H, s), 2.02 (3H, s), 2.04 (3H, s), 2.09 (3H, s), 2.30 (2H, t, J = 7.6 Hz), 3.46 (1H, dt, J = 9.5, 6.8 Hz), 3.67 (3H, s), 3.69 (1H, ddd, J = 2.4, 4.6, 9.8 Hz, H-5), 3.86 (1H, dt, J = 9.5, 6.4 Hz), 4.15 (1H, dd, J = 12.4, 2.4 Hz, H-6), 4.28 (1H,dd, J = 12.4, 4.6 Hz, H-6), 4.49 (1H, d, J = 8.1 Hz, H-1), 4.98 (1H, dd, J = 9.5, 8.1 Hz, H-2), 5.09 (1H, dd, J = 9.8, 9.5 Hz, H-4), 5.20 (1H, dd, J = 9.5, 9.5 Hz, H-3)

(2) A methanol solution of 28% sodium methoxide (264 mg, 6.64 mmol) was added to a methanol solution (4 ml) of 8-methoxycarbonyloctyl 2,3,4,6-tetra-O-acetyl-β-D-glucopyranoside (1123 mg, 3.32 mmol) obtained in step (1). The mixture was stirred under ice cooling for 4 hr. Acetic acid (0.1ml) was added to the reaction solution, followed by concentration. The oil thus obtained was purified by column chromatography on silica gel (chloroform-methanol (9 : 1)) to give 587 mg (59%) of the title compound.

$[\alpha]_D^{17}$ -12.9° (c = 1.16, methanol)
$^1$H-NMR ($D_2O$) δ: 1.32 (8H, s), 1.59-1.66 (4H, m), 2.40 (2H, t, J = 7.6 Hz), 3.26 (1H, dd, J = 7.8, 9.0 Hz, H-2), 3.39 (1H, dt, J = 9.5, 9.5 Hz, H-4), 3.44-3.46 (1H, m, H-5), 3.49 (1H, dd, J = 9.0, 9.3 Hz, H-3), 3.66-3.70 (1H, m), 3.70 (1H, s), 3.73 (1H, dd, J = 5.9, 12.2 Hz, H-6), 3.90-3.95 (2H, m, H-6, $CH_2O$), 4.46 (1H, d, J = 8.1 Hz, H-1)

Example 2 Glc-O-$C_8$-COOH (9-(β-D-glucopyranosyl)nonanoic acid)

A solution of sodium hydroxide (180 mg, 4.5 mmol) in water (8ml) was added to a solution of 8-methoxycarbonyloctyl β-D-glucopyranoside (754 mg, 2.23 mmol), obtained in Example 1, in a mixture of methanol (8 ml) and THF (4ml). The mixture was stirred at room temperature for 3.5 hr. The reaction solution was concentrated. The concentrate was adjusted to pH 3 by addition of 2 N hydrochloric acid and applied to a Diaion HP-20 column. Elution was carried out with water (300 ml) and acetonitrile (300 ml). The fraction eluted with acetonitrile was concentrated to dryness to give 640 mg(89%) of the title compound.

$[\alpha]_D^{25}$-20.7° (c = 1.13, methanol)
$^1$H-NMR ($D_2O$) δ: 1.34 (8H, s), 1.54-1.65 (4H, m), 2.37 (2H, t, J = 7.6 Hz), 3.27 (1H, dd, J = 8.1, 9.3 Hz, H-2), 3.40 (1H, dd, J = 9.0, 9.8 Hz, H-4), 3.47 (1H, ddd, J = 2.2, 5.9, 9.8 Hz, H-5), 3.50 (1H, dd, J = 9.0, 9.3 Hz, H-3), 3.69 (1H, dt, J = 10.0, 6.8 Hz), 3.73 (1H, dd, J = 5.9, 12.2 Hz, H-6), 3.93 (1H, dd, J = 10.0, 7.1 Hz), 3.27 (1H, dd, J = 2.2, 12.2 Hz, H-6), 4.47 (1H, d, J = 8.1 Hz, H-1)

Example 3 Glc-O-$C_8$-AVP

AVP(40 mg, 0.04 mmol), DCC(31 mg, 0.12 mmol), and HOBt (20 mg, 0.15 mmol) were added to 9-(β-D-glucopyranosyl) nonanoic acid (40 mg, 0.12 mmol) obtained in Example 2. The mixture was stirred in DMF (2 ml) for 20 hr. The reaction solution was concentrated to dryness. The concentrate was filtered, and the filtrate was lyophilized.

The compound thus obtained was purified by HPLC (ODS column) to give a compound comprising a glucose derivative introduced into an AVP fragment at its N end.

$^1$H-NMR ($D_2O$) δ: 1.35 (8H, m), 1.54-1.76 (5H, m), 1.80-1.88 (1H, m), 1.92-2.01 (2H, m), 2.08-2.26 (5H, m), 2.30-2.38 (3H, m), 2.75-2.78 (1H, m), 2.86-2.90 (2H, m), 2.98-3.10 (4H, m), 3.17-3.32 (4H, m), 3.36-3.54 (4H, m), 3.64-3.86 (4H, m), 3.90-3.99 (3H, m), 4.16-4.22 (1H, m), 4.34-4.40 (1H, m), 4.44-4.52 (2H, m), 6.82 (2H, d, J = 8.3 Hz), 6.94 (2H, d,J = 8.3 Hz), 7.28-7.32 (2H, m), 7.38-7.48 (3H, m)

Example 4 Gal-O-$C_8$-COOH (9-(β-D-galactopyranosyl)nonanoic acid)

1 N sodium hydroxide (1.2 ml) was added to a solution of 8-methoxycarbonyloctyl β-D-galactopyranoside (350 mg, 1 mmol), prepared according to the method of Lemieux R.U. et al. (J. Am. Chem. Soc., 97, 4074, 1975), in a mixture

of methanol (5 ml) and THF(4 ml). The mixture was stirred at room temperature for 18 hr. The reaction solution was adjusted to pH 3 by addition of Amberlist IR-120B ($H^+$), followed by filtration. The filtrate was concentrated to dryness to give 327 mg (90%) of the title compound.

$[\alpha]_D^{25}$ -10.3° (c = 1.0, methanol)
$^1$H-NMR ($D_2O$) δ: 1.30-1.44 (8H, m), 1.55-1.66 (4H, m), 2.27 (2H, t, J = 7.0 Hz), 3.45 (1H,dd, J = 3.5, 9.5 Hz), 3.46-3.52 (2H, m), 3.53 (1H, dt, J = 8.5,7.0 Hz), 3.69-3.78 (2H, m), 3.83 (1H, d, J = 3.5 Hz), 3.89 (1H, dt, J = 8.5,7.0 Hz), 4.20 (1H,d, J = 8.0 Hz)

Example 5 Gal-O-$C_8$-AVP

A solution of AVP (80mg, 0.07 mmol), with triethylamine (0.049 ml, 0.35 mmol) added thereto, dissolved in methanol was concentrated to dryness. DMF (3 ml), 9-(β-D-galactopyranosyl)nonanoic acid (50 mg,0.15 mmol) obtained in Example 4, DCC (31 mg,0.15 mmol), and HOBt (20 mg,0.15 mmol) were added thereto, and the mixture was stirred overnight. The reaction solution was concentrated to dryness, followed by filtration. The filtrate was purified by HPLC (ODS column) to give the title compound comprising a galactose derivative introduced into an AVP fragment at its N end.

$[\alpha]_D^{25}$ -79.1° (c = 0.44, $H_2O$)
$^1$H-NMR ($D_2O$) δ: 1.15-1.37 (8H, m), 1.49-1.96 (9H, m), 2.01-2.35 (9H, m), 2.67 (1H, dd, J = 10.0,14.0 Hz), 2.82-2.85 (2H, m), 2.91-3.07 (4H, m), 3.11-3.24 (4H, m), 3.30-3.37 (1H, m), 3.46-3.52 (1H, m), 3.58-3.73 (9H, m), 3.87-3.96(4H, m), 4.11-4.16 (2H, m), 4.30-4.33 (1H, m), 4.34 (2H, d, J = 8.0 Hz) 4.42-4.46 (1H, m), 4.55-4.69 (3H, m), 4.87-4.92 (1H, m), 6.77 (2H, d, J = 8.5 Hz), 7.00 (2H, d, J = 8.5 Hz), 7.25-7.41 (5H, m)

Example 6 Man-O-$C_8$-COOH (9-(β-D-mannopyranosyl)nonanoic acid)

A solution of sodium hydroxide(46 mg, 1.15 mmol) in water (2 ml) was added to a solution of 8-methoxycarbonyloctyl β-D-mannopyranoside (200 mg, 0.57 mmol) in a mixture of methanol (2 ml) and THF (2 ml). The mixture was stirred at room temperature for 255 min. The reaction solution was concentrated. The concentrate was adjusted to pH 2 by addition of 2 N hydrochloric acid and then adsorbed onto a Diaion HP-20 column. Elution was carried out with water (200 ml) and acetonitrile (150 ml). The fraction eluted with acetonitrile was concentrated to dryness to give 187 mg of the title compound.

$[\alpha]_D$ -39.0° (c = 1.1, methanol)
$^1$H-NMR ($D_2O$) δ: 1.33-1.41 (8H, m), 1.62-1.66 (4H, m), 2.41 (2H, t, J = 7.3 Hz), 3.41 (1H, ddd, J = 2.2, 7.1, 12.0 Hz), 3.62 (1H, dd, J = 9.5, 9.8 Hz), 3.67-3.72 (2H, m), 3.78 (1H, dd, J = 6.4, 12.2 Hz), 3.92 (1H, dt, J = 10.0, 6.8 Hz), 3.97 (1H, dd, J = 2.2, 12.2 Hz), 4.02 (1H, d, J = 2.4 Hz), 4.71 (1H, d, J = 1.2 Hz)
IR (KBr): 3406, 2932, 1725 $cm^{-1}$ FAB-MS m/z: 359 ($M+Na^+$), 337 ($MH^+$)

| Elementary analysis: | | |
|---|---|---|
| Calculated for $C_{15}H_{28}O_8 \cdot 1/2\ H_2O$: | C, 52.16; | H, 8.46 |
| Found: | C, 52.43; | H, 8.29 |

Example 7 Man-O-$C_8$-AVP

Triethylamine (0.025 ml) was added to a solution of AVP (50 mg, 0.05 mmol) in methanol (2 ml). The mixture was concentrated to dryness. DMF (2 ml), 9-(β-D-mannopyranosyl)nonanoic acid (50 mg, 0.15 mmol) obtained in Example 6, DCC (41 mg, 0.20 mmol), and HOBt (27 mg, 0.20 mmol) were added to the residue. The mixture was stirred at room temperature for 17 hr. The reaction solution was adjusted to pH 2.0 by addition of 10% TFA and purified by HPLC to give 17 mg of the title compound comprising a mannose derivative introduced into AVP at its N end.

$[\alpha]_D$ -77.8° (c = 0.23, $H_2O$)
$^1$H-NMR ($D_2O$) δ: 1.27-1.39 (8H, m), 1.56-1.73 (5H, m), 1.83-1.86 (1H, m), 1.92-2.00 (2H, m), 2.08-2.25(3H, m), 2.31-2.38 (2H, m), 2.42-2.45 (1H, m), 2.70-2.75 (1H, m), 2.88 (2H, d, J = 6.6 Hz), 2.98-3.10 (3H, m), 3.18-3.28 (3H,

m), 3.36-3.42 (2H, m), 3.59-4.00 (8H, m), 3.81-4.02 (5H, m), 4.17-4.21 (1H, m), 4.36-4.39 (1H, m), 4.48-4.51 (1H, m), 4.93-4.96 (1H, m), 6.83 (2H, d, J = 8.5 Hz), 7.06 (2H, d, J = 8.5 Hz), 7.30 (2H, d, J = 7.6 Hz), 7.40-7.47 (3H, m)
FAB-MS m/z: 1403 ($MH^+$)
Amino acid composition (%) (hydrolyzates in 6 N hydrochloric acid): Gly 0.99, Arg, 1.00, Pro 0.96,Cys 1.81, Asp 1.00, Glu 0.97, Phe 1.01, Tyr 0.91, ammonia 3.25 (recovery of Asp: 80%)

Example 8 Man(α)-O-$C_8$-AVP

Triethylamine (0.025 ml) was added to a solution of AVP (50 mg, 0.05 mmol) in methanol (2 ml). The mixture was concentrated to dryness. DMF (2 ml), 9-(α-D-mannopyranosyl)nonanoic acid (50 mg, 0.15 mmol), DCC(41 mg, 0.20 mmol), and HoBt (27 mg, 0.20 mmol) were added to the residue. The mixture was stirred at room temperature for 20.5 hr. The reaction solution was adjusted to pH 2.0 by addition of 10% TFA and purified by HPLC to give 28 mg of the title compound comprising a mannose derivative introduced into AVP at its N end.

$[\alpha]_D$ -62.9° (c = 0.28, $H_2O$)
$^1$H-NMR ($D_2O$) δ: 1.27-1.40 (8H, m), 1.57-1.80 (5H, m), 1.80-1.90 (1H, m), 1.92-2.02 (2H, m), 2.05-2.24 (5H,m), 2.32-2.40 (3H, m), 2.70-2.75 (1H, m), 2.88 (2H, d, J = 6.6 Hz), 2.98-3.10 (3H, m), 3.18-3.28 (3H, m), 3.36-3.40 (1H, m), 3.53-3.58 (2H, m), 3.64-3.85 (7H, m), 3.90-3.97 (4H, m), 4.17-4.20 (4H, m), 4.36-4.38 (1H, m), 4.47-4.50 (1H, m), 6.82 (2H, d, J = 8.6 Hz), 7.06 (2H, d, J = 8.6 Hz), 7.30 (2H, d, J = 7.1 Hz), 7.44-7.46 (3H, m)
FAB-MS m/z:1403 ($MH^+$)
Amino acid composition (%) (hydrolysates in 6 N hydrochloric acid): Gly 0.98, Arg, 0.99, Pro 0.99, Cys 1.83, Asp 1.00, Glu0.97, Phe 1.02, Tyr 0.92, ammonia 3.21 (recovery of Asp: 80%)

Example 9 2dGlc-O-$C_8$-COOMe(8-methoxycarbonyloctyl 2-deoxy-β-D-glucopyranoside)

(1) A solution of 3,4,6-tri-O-acetyl-D-glucal (12.17 g, 44.7 mmol), N-iodosuccinimide (16.07 g, 1.6 eq), and 8-methoxycarbonyl octanol (15.53 g, 1.85 eq) in acetonitrile (100 ml) was stirred for 6 hr. The reaction solution was distributed in chloroform and a 10% aqueous $Na_2S_2O_3$ solution. The organic layer was dried over $MgSO_4$ and then filtered. The filtrate was concentrated to dryness. The residue was purified by column chromatography on silica gel (toluene) to give a 1 : 5 mixture of the title compound and 8-methoxycarbonyloctyl 3,4,6-tri-O-acetyl-2-deoxy-2-iodo-α-D-mannopyranoside (10.04 g, 66.0%). Purification was again carried out by column chromatography on silica gel to isolate 8-methoxycarbonyloctyl 3,4,6-tri-O-acetyl-2-deoxy-2-iodo-β-D-glucopyranoside as the target compound.

$[\alpha]_D$ +37.9° (c = 1.08, chloroform)
$^1$H-NMR ($CDCl_3$) δ: 1.32-1.42 (8H, m), 1.52-1.67 (4H, m), 2.01 (3H, s), 2.08 (3H, s), 2.09 (3H, s), 2.30 (2H, t, J = 7.6 Hz), 3.53 (1H, dt, J = 9.3, 6.8 Hz), 3.67 (3H, s), 3.72 (1H, ddd, J = 2.4, 4.9, 10.0 Hz), 3.89 (1H, dt, J = 9.3, 6.4 Hz), 3.89 (1H, dd, J = 2.4, 12.2 Hz), 4.12 (1H, dd, J = 2.4, 12.5 Hz), 4.30 (1H, dd, J = 4.9, 12.5 Hz), 4.59 (1H, d, J = 9.0 Hz), 4.96 (1H, dd, J = 9.0, 10.0 Hz), 5.29 (1H, dd, J = 9.0, 11.2 Hz)
IR (KBr): 1756 $cm^{-1}$ FAB-MS m/z: 587 ($MH^+$)
3,4,6-Tri-O-acetyl-2-deoxy-2-iodo-α-D-mannopyranoside was instrumentally analyzed. The results were as follows.

$[\alpha]_D$ +15.8° (c = 1.50, chloroform)
$^1$H-NMR ($CDCl_3$) δ: 1.31-1.38 (8H, m), 1.53-1.64 (4H, m), 2.06 (3H, s), 2.09 (3H, s), 2.12 (3H, s), 2.31 (2H, t, J = 7.6 Hz), 3.45 (1H, dt, J = 9.8, 6.6 Hz), 3.65 (1H, dt, J = 9.8, 6.8 Hz), 3.67 (3H, s) , 4.01 (1H, ddd, J = 2.4, 4.9, 10.0 Hz), 4.16 (1H, dd, J = 2.4, 12.2 Hz), 4.24 (1H, dd, J = 4.9, 12.2 Hz), 4.53 (1H, dd, J = 1.2, 4.4 Hz), 4.65 (1H, dd, J = 4.4, 9.5 Hz), 5.16 (1H, s), 5.37 (1H, dd, J = 9.8, 9.8 Hz)
IR (KBr): 1746 $cm^{-1}$ FAB-MS m/z: 587 ($MH^+$)

(2) Tributyltin hydride (0.583 ml, 1.4 eq) was added to a solution of 8-methoxycarbonyloctyl 3,4,6-tri-O-acetyl-2-deoxy-2-iodo-β-D-glucopyranoside (909 mg, 1.55 mmol), obtained in step (1), in benzene (15 ml). The mixture was stirred at 60°C for 105 min. The reaction solution was concentrated. The concentrate was dissolved in acetonitrile. The acetonitrile layer was washed with hexane and then concentrated to dryness. The residue was purified by column chromatography on silica gel (toluene-ethyl acetate (20 : 1)) to give 642 mg (90%) of 8-methoxycarbonyloctyl 3,4,6-tri-O-acetyl-2-deoxy-β-D-glucopyranoside.

$[\alpha]_D$ -20.1° (c = 1.02, chloroform)
$^1$H-NMR ($CDCl_3$) δ: 1.24-1.30 (8H, m), 1.51-1.64 (4H, m), 1.71-1.78 (1H, m), 2.03 (3H, s), 2.04 (3H, s), 2.08 (3H,s), 2.28-2.33 (1H, m), 2.30 (2H, t, J = 7.6 Hz), 3.45 (1H, dt, J = 9.5, 6.8 Hz), 3.59 (1H, ddd, J = 2.4, 4.9, 9.5 Hz), 3.67

(3H, s), 3.87 (1H, dt, J = 9.5, 6.6 Hz), 4.11 (1H, dd, J = 2.4, 12.2 Hz), 4.31 (1H, dd, J = 4.9, 12.2 Hz), 4.55 (1H, dd, J = 2.0, 9.5 Hz), 4.97-5.05 (2H, m)
IR (KBr): 1742 $cm^{-1}$ FAB-MS m/z: 461 ($MH^+$)

(3) A methanol solution (28%, 0.17 ml) of sodium methoxide was added under ice cooling to a solution of 8-methoxycarbonyloctyl 3,4,6-tri-O-acetyl-2-deoxy-β-D-glucopyranoside (642 mg, 1.4 mmol), obtained in step (2), in methanol (10 ml). The mixture was stirred for 260 min. Acetic acid (0.05 ml) was added to the reaction solution. The mixture was concentrated to dryness. The residue was purified by column chromatography on silica gel (chloroform-methanol (9 : 1)) to give 446 mg (95%) of the title compound.

$[\alpha]_D$ -44.6° (c = 1.00, methanol)
$^1$H-NMR ($D_2O$) δ: 1.29-1.33 (8H, m), 1.46-1.53 (1H, m), 1.56-1.65 (4H, m), 2.25-2.28 (1H, m), 2.38 (2H, t, J = 7.6 Hz), 3.27 (1H, dd, J = 9.0, 9.8 Hz), 3.38 (1H, dd, J = 7.3, 8.8 Hz), 3.63-3.75 (3H, m), 3.89-3.94 (2H, m), 4.72 (1H, d, J = 9.8 Hz)
IR (KBr): 3420, 1738 $cm^{-1}$ FAB-MS m/z: 335 ($MH^+$)

| Elementary analysis: | | |
|---|---|---|
| Calculated for $C_{16}H_{30}O_7$: | C, 57.38; | H, 7.88 |
| Found: | C, 57.28; | H, 8.22 |

Example 10 <u>2dGlc-O-$C_8$-COOH (9(2-deoxy-β-D-glucopyranosyl)-nonanoic acid)</u>

A solution of sodium hydroxide (93 mg, 2.3 mmol) in water (3 ml) was added to a solution of 8-methoxycarbonyloctyl 2-deoxy-β-D-glucopyranoside (393 mg, 1.17 mmol), obtained in Example 9, in methanol (3 ml). The mixture was stirred at room temperature for 6 hr. The reaction solution was concentrated. The concentrate was adjusted to pH 2 by addition of 2 N hydrochloric acid. The resultant precipitate was collected to give 249 mg (66%) of the title compound.

$[\alpha]_D$ -29.9° (c = 1.01, methanol)
$^1$H-NMR ($D_2O$) δ: 1.35-1.43 (8H, m), 1.61-1.70 (4H, m), 2.73-2.82 (2H, m), 3.33 (1H, dd, J = 8.8, 9.8 Hz), 3.43 (1H, dd, J = 9.0, 9.5 Hz), 3.47 (1H, ddd, J = 2.0, 5.6, 9.5 Hz), 3.51 (1H, dd, J = 8.8, 9.0 Hz), 3.73 (1H, dd, J = 5.6, 12.4 Hz), 3.92 (1H, dd, J = 2.0, 12.4 Hz), 4.54 (1H, d, J = 9.8 Hz)
IR (KBr): 3400, 1738, 1712 $cm^{-1}$ FAB-MS m/z: 321 ($MH^+$)

| Elementary analysis: | | |
|---|---|---|
| Calculated for $C_{15}H_{28}O_7$: | C, 56.16; | H, 8.81 |
| Found: | C, 56.16; | H, 9.04 |

Example 11 <u>2dGlc-O-$C_8$-AVP</u>

Triethylamine (0.020 ml) was added to a solution of AVP (40 mg, 0.04 mmol) in methanol (2 ml). The mixture was concentrated to dryness. DMF (2 ml), 9-(2-deoxy-β-D-glucopyranosyl)nonanoic acid (40 mg, 0.12 mmol) obtained in Example 10, DCC (33 mg, 0.16 mmol), and HOBt (22 mg, 0.16 mmol) were added to the residue. The mixture was stirred at room temperature for 18 hr. The reaction solution was adjusted to pH 2.0 by addition of 10% TFA and then purified HPLC to give 15 mg of the title compound.

$[\alpha]_D$ -77.8° (c = 0.23, $H_2O$)
$^1$H-NMR ($D_2O$) δ: 1.20-1.34 (8H, m), 1.44-2.00 (8H, m), 2.04-2.36 (7H, m), 2.66-2.72 (1H, m), 2.85 (2H, d, J = 6.1 Hz), 2.89-3.08 (4H, m), 3.13-3.38 (6H, m), 3.69-3.97 (9H, m), 4.13-4.17 (1H, m), 4.32-4.36 (5H, m), 4.44-4.47 (1H, m), 6.79 (2H, d, J = 8.0 Hz), 7.02 (2H, d, J = 8.0 Hz), 7.27 (2H, d, J = 7.3 Hz), 7.37-7.44 (3H, m)

FAB-MS m/z: 1386 ($MH^+$)

Amino acid composition (%) (hydrolysates in 6 N hydrochloric acid): Gly 0.95, Arg, 0.97, Pro 0.98, Cys 1.57, Asp 1.00, Glu 0.99, Phe 1.02, Tyr 0.86, ammonia 3.05 (recovery of Asp: 81%)

Example 12 $^3$H-2dGlc-O-$C_8$-AVP

A methanol solution of AVP (60 μl; concentration 0.56 mg/ml) and a methanol solution of triethylamine (50 μl; concentration 70 μl/ml) were added to a 0.05M acetic acid-ethanol (3 : 7) solution (0.25 ml) of $^3$H-AVP (3.25 μg, 250 μCi). The mixture was concentrated to dryness. A DMF solution of 9-(2-deoxy-β-D-glucopyranosyl)nonanoic acid obtained in Example 10 (7 μl; concentration 1.5 mg/0.1 ml), a DMF solution of WSCI (6.2 μl; concentration 5.1 mg/0.5 ml), and a DMF solution of HOBt (7.2 μl; concentration 3.1 mg/0.5 ml) were added to the residue. The mixture was stirred at room temperature for 19 hr. The reaction solution was added to 50 μl of 0.05% TFA and then purified by HPLC to give a tritium-labeled product in an amount corresponding to 31.4 μg. The specific radioactivity was 6.34 Ci/ mmol, and the amount of the product synthesized was 168.0 μCi.

Example 13 Glc-O-$C_8$-Oxy

(1) 9-(β-D-Glucopyranosyl)nonanoic acid (150mg, 0.45 mmol), DCC(78 mg, 0.68 mmol), and HOSu (110 mg, 0.53 mmol) were stirred in dioxane (8 ml) for 20 hr. The reaction solution was filtered, followed by concentration to dryness. The residue was purified by column chromatography on silica gel (chloroform-methanol(9 : 1)) to give 105 mg of an N-hydroxysuccinylimide ester of 9-(β-D-glucopyranosyl)nonanoic acid as the target compound.

$[\alpha]_D$ -19.3° (c = 1.07, methanol)

$^1$H-NMR ($CO_3OD$) δ: 1.33-1.48 (8H, m), 1.64-1.67 (2H, m), 1.73-1.76 (2H, m), 2.65 (2H, t, J = 7.1 Hz), 2.86 (4H, s), 3.20 (1H, dd, J = 8.3, 8.5 Hz), 3.29-3.38 (3H, m), 3.57 (1H, dt, J = 9.3, 7.6 Hz), 3.70 (1H, dd, J = 4.9, 11.7 Hz), 3.87-3.94 (2H, m), 4.28 (1H, d, J = 7.8 Hz)

IR (KBr): 3464, 2932, 1822, 1788, 1730 $cm^{-1}$ FAB-MS m/z: 434 ($MH^+$)

| Elementary analysis: | | | |
|---|---|---|---|
| Calculated for $C_{19}H_{31}NO_1 \cdot 1/2\ H_2O$: | C, 51.58; | H, 7.20; | N, 3.17 |
| Found: | C, 37.04; | H, 7.11; | N, 3.05 |

(2) A methanol solution of triethylamine was added to a solution of oxytocin (45 mg, 0.04 mmol). The mixture was concentrated to dryness. DMF (2 ml) and a DMF solution (2 ml) of an N-hydroxysuccinylimide ester of 9-(β-D-glucopyranosyl)nonanoic acid (60 mg, 0.14 mmol) obtained in step (1) were added to the residue. The mixture was stirred at room temperature for 20 hr. The reaction solution was adjusted to pH 2.0 by addition of 10% TFA and then purified by HPLC to give 30 mg of the title compound.

$[\alpha]_D$ -80.0° (c = 0.19, methanol) FAB-MS m/z: 1326 ($MH^+$)

Amino acid composition (%)(hydrolyzates in 6 N hydrochloric acid): Asp 1.00, Cys 1.86, Glu 0.99, Gly 1.00, Ile 0.97, Leu 1.03, Pro 1.00, Tyr 0. 91, ammonia 2.94 (recovery of Asp: 89%)

Example 14 $^3$H-Glc-O-$C_8$-Oxy

A methanol solution of oxytocin (50 μl; concentration 1.1 mg/2 ml) and a methanol solution of triethylamine (10 μl; concentration 70 μl/ml) were added to an ethanol-water (8 : 2) solution (0.25 ml) of $^3$H-oxytocin (6.5 μg, 250 μCi). The mixture was concentrated to dryness. A DMF solution of an N-hydroxysuccinylimide ester of 9-(β-D-glucopyranosyl)nonanoic acid obtained in Example 13 (20 μl; concentration 2.2 mg/0.1 ml) was added to the residue. The mixture was stirred at room temperature for 19 hr. The reaction solution was added to 70 μl of 0.05% TFA and then purified by HPLC to give a tritium-labeled product in an amount corresponding to 15.8 μg (chemical yield 47%). The specific radioactivity was 9.02 Ci/ mmol, and the amount of the product synthesized was 107.6 μCi (radiochemical yield 43%).

Example 15 Man-O-$C_8$-Oxy

Triethylamine (0.031 ml) was added to a solution of oxytocin (40mg, 0.036 mmol) in methanol (2 ml). The mixture was concentrated to dryness. DMF (2 ml) and a DMF solution (2 ml) of an N-hydroxysuccinylimide ester of 9-(β-D-mannopyranosyl)nonanoic acid (50 mg, 0.12 mmol) were added to the residue. The mixture was stirred at room temperature for 21 hr. The reaction solution was adjusted to pH 2.0 by addition of 10% TFA and then purified by HPLC to give 30 mg of the title compound.

Example 16 $^3$H-Man-O-$C_8$-Oxy

A methanol solution of oxytocin (50 µl; concentration 1.1 mg/2 ml) and a methanol solution of triethylamine (10 µl; concentration 70 µl/ml) were added to an ethanol-water (8 : 2) solution (0.25 ml) of $^3$H-oxytocin (6.5 µg, 250 µCi). The mixture was concentrated to dryness. A DMF solution of an N-hydroxysuccinylimide ester of 9-(β-D-glucopyranosyl)nonanoic acid obtained in Example 13 (20 µl; concentration 2.2 mg/0.1 ml) was added to the residue. The mixture was stirred at room temperature for 19 hr. The reaction solution was added to 70 µl of 0.05% TFA and then purified by HPLC to give a tritium-labeled product. The specific radioactivity was 4.53 Ci/ mmol, and the amount of the product synthesized was 87.5 µCi.

Example 17 Glc-S-$C_8$-COOMe (8-methoxycarbonyloctyll-thio-β-D-glucopyranoside)

(1) Methyl 9-hydroxynonanoate (1.16 g, 6.17 mmol), triphenylphosphine (2.43 g, 9.26 mmol), and carbon tetrabromide (3.07 g, 9.26 mmol) were stirred in toluene (5 ml) under ice cooling for 3 hr. The reaction solution was filtered, followed by concentration to dryness . The residue was purified by column chromatography on silica gel (toluene-ethyl acetate (98 : 2)) to give 1.37 g of methyl 9-bromononanoate.

$^1$H-NMR ($CDCl_3$) δ: 1.35-1.40 (6H, m), 1.48-1.50 (2H, m), 1.64-1.70 (2H, m), 1.91 (2H, tt, J = 7.6, 6.8 Hz), 2.37 (2H, t, J = 7.6 Hz), 3.46 (2H, t, J = 6.8 Hz), 3.73 (3H, s)

(2) 2-(2,3,4,6-Tetra-O-acetyl-1-thio-β-D-glucopyranosyl)thiopseudourea hydrobromide (1287 mg, 2.70 mmol) synthesized by the method of S. Saito and T. Tsuchiya (Chem. Pharm. Bull., 33, 503, 1985), methyl 9-bromononanoate (814 mg, 3.24 mmol) obtained in step (1), $K_2CO_3$ (386 mg, 2.80 mmol), and $NaHSO_3$ (257 mg, 2.46 mmol) were stirred in acetone (30 ml)/water (30 ml) at room temperature for 19 hr. The reaction solution was distributed in chloroform and water. The organic layer was dried over $MgSO_4$, filtered, and then concentrated to dryness. The residue was purified by column chromatography on silica gel (toluene-ethyl acetate (9:1)) to give 980 mg of 8-methoxycarbonyloctyl 2,3,4,6-tetra-O-acetyl-1-thio-β-D-glucopyranoside.

$[\alpha]_D$ -26.4° (c = 1.01,chloroform)
$^1$H-NMR ($CDCl_3$) δ: 1.29-1.38 (8H, m), 1.54-1.63 (4H, m), 2.01 (3H, s), 2.03 (3H, s), 2.06 (3H, s), 2.08 (3H, s), 2.30 (2H, t, J = 7.6 Hz), 2.61-2.71 (2H, m), 3.67 (3H, s), 3.71 (1H, ddd, J = 2.4, 4.9, 9.8 Hz), 4.14 (1H, dd, J = 2.4, 12.5 Hz), 4.25 (1H, dd, J = 4.9, 12.5 Hz), 4.47 (1H, d, J = 10.3 Hz), 5.03 (1H, dd, J = 9.5, 9.8 Hz), 5.08 (1H, dd, J = 9.8, 9.8 Hz), 5.22 (1H, dd, J = 9.3, 9.5 Hz)
IR (KBr): 1744, 1440, 1374, 1230 $cm^{-1}$ FAB-MS m/z: 535 ($MH^+$)

| Elementary analysis: | | |
|---|---|---|
| Calculated for $C_{24}H_{38}O_{11}S \cdot 1/2\ H_2O$: | C, 53.03; | H, 7.23 |
| Found: | C, 53.30; | H, 7.46 |

(3) A methanol solution of sodium methoxide (28%, 0.23 ml) was added to a solution of 8-methoxycarbonyloctyl 2,3,4,6-tetra-O-acetyl-1-thio-β-D-glucopyranoside (900 mg, 1.9 mmol), obtained in step (2), in methanol (10 ml) under ice cooling. The mixture was stirred for 2.5 hr. Acetic acid (0.067 ml) was added to the reaction solution. The mixture was concentrated to dryness. The residue was purified by column chromatography on silica gel (chloroform-methanol (9 : 1)) to give 522 mg of the title compound.

$[\alpha]_D$ -44.6° (c = 1.00, methanol)

$^1H$-NMR ($D_2O$) δ: 1.35-1.44 (8H, m), 1.62-1.71 (4H, m), 2.43 (2H, t, J = 7.6 Hz), 2.74-2.83 (2H, m), 3.35 (1H, dd, J = 8.8, 9.8 Hz), 3.44-3.54 (3H, m), 3.73 (3H, s), 3.76 (1H, dd, J = 4.2, 12.2 Hz), 3.93 (1H, dd, J = 2.2, 12.2 Hz), 4.55 (1H, d, J = 10.0 Hz)
IR (KBr): 3384, 1732 $cm^{-1}$ FAB-MS m/z: 367 ($MH^+$)

Example 18 Glc-S-$C_8$-COOH (9-(1-thio-β-D-glucopyranosyl)nonanoic acid)

An aqueous solution (2 ml) of sodium hydroxide (68 mg, 1.7 mmol) was added to a methanol solution (2 ml) of 8-methoxycarbonyloctyl 1-thio-β-D-glucopyranoside (260 mg, 0.85 mmol) obtained in Example 17. The mixture was stirred at room temperature for 6 hr. The reaction solution was concentrated. The concentrate was adjusted to pH about 2 by addition of 2 N hydrochloric acid. The resultant precipitate was collected by filtration to give 187 mg of the title compound.

$[\alpha]_D$ -45.4° (c = 1.00, methanol)
$^1H$-NMR ($D_2O$) δ: 1.35-1.43 (8H, m), 1.61-1.70 (4H, m), 2.73-2.82 (2H, m), 3.33 (1H, dd, J = 8.8, 9.8 Hz), 3.43 (1H, dd, J = 9.0, 9.5 Hz), 3.47 (1H, ddd, J = 2.0, 5.6, 9.5 Hz), 3.51 (1H, dd, J = 8.8, 9.0 Hz), 3.73(1H, dd, J = 5.6, 12.4 Hz), 3.92 (1H, dd, J = 2.0, 12.4 Hz), 4.54 (1H, d, J = 9.8 Hz)
IR (KBr): 3400, 1710 $cm^{-1}$ FAB-MS m/z: 353 ($MH^+$)

| Elementary analysis: | | |
|---|---|---|
| Calculated for $C_{15}H_{28}O_7S \cdot 1/4H_2O$: | C, 50.47; | H, 8.04 |
| Found: | C, 50.85; | H, 8.52 |

Example 19 Glc-S-$C_8$-AVP

Triethylamine (0.025 ml) was added to a methanol solution (2 ml) of AVP (50 mg, 0.05 mmol). The mixture was concentrated to dryness. DMF (2 ml), 9-(1-thio-β-D-glucopyranosyl)nonanoic acid (50 mg, 0.14 mmol) obtained in Example 18, DCC (41 mg, 0.20 mmol), and HOBt (27 mg, 0.20 mmol) were added to the residue. The mixture was stirred at room temperature for 19.5 hr. The reaction solution was adjusted to pH 2.0 by addition of 10% TFA and then purified by HPLC to give 24 mg of the title compound comprising a glucose derivative introduced into AVP at its N end.

$[\alpha]_D$ -83.2° (c = 0.19, $H_2O$)
$^1H$-NMR ($D_2O$) δ: 1.24-1.40 (8H, m), 1.54-1.71 (5H, m), 1.80-1.90 (1H, m), 1.94-2.03 (2H, m), 2.06-2.34 (6H, m), 2.69-2.78 (3H, m),2.87 (1H, d, 3 = 7.6 Hz), 2.96-3.09 (3H, m), 3.17-3.26 (3H, m), 3.31-3.52 (5H, m), 3.70-3.96 (6H, m), 4.16-4.19 (1H, m), 4.34-4.38 (1H, m), 4.46-4.52 (2H, m), 4.59-4.74 (3H, m), 4.93-4.94 (1H, m), 6.81 (2H, d, J = 8.6 Hz), 7.04 (2H, d, 3 = 8.6 Hz), 7.29 (2H, d, J = 8.1 Hz), 7.39-7.46 (3H, m).
FAB-MS m/z: 1403 ($MH^+$)

Example 20 $^3H$-Glc-S-$C_8$-AVP

A methanol solution of AVP (70 μl; concentration 1.1 mg/2 ml) and a methanol solution of triethylamine (50 μl; concentration 70 μl/ml) were added to a 0.05 M acetic acid -ethanol (3 : 7) solution (0.25 ml) of $^3H$-AVP (4.25 μg, 250 μCi). The mixture was concentrated to dryness. A DMF solution of 9-(1-thio-β-D-glucopyranosyl)nonanoic acid (7 μl; concentration 1.0 mg/0.05 ml) obtained in Example 18, a DMF solution of WSCI (7 μl; concentration 1.7 mg/0.2 ml), and a DMF solution of HOBt (5 μl; concentration 1.1 mg/0.1 ml) were added to the residue. The mixture was stirred at room temperature for 19 hr. The reaction solution was added to 50 μl of 0.05% TFA, and the mixture was purified by HPLC to give a tritium-labeled product in an amount corresponding to 13.8 μg. The specific radioactivity was 2.89 Ci/ mmol, and the amount of the product synthesized was 112.0 μCi.

Example 21 Glc-S-$C_{11}$-COOMe (11-methoxycarbonylundecyl 1-thio- glucopyranoside)

(1) Methyl 12-hydroxydodecanoate (2.30 g, 10.0 mmol), triphenylphosphine (3.93 g, 1.5 eq), carbon tetrabromide (4.97 g, 1.5 eq) were stirred in toluene (10 ml) at room temperature for 3 hr. The reaction solution was filtered, followed by concentration to dryness. The residue was purified by column chromatography on silica gel (toluene-ethyl

acetate (99 : 1)) to give 2.65 g (90%) of methyl 12-bromododecanoate.

$^1$H-NMR ($CDCl_3$) δ: 1.20-1.35 (12H, m), 1.39-1.43 (2H, m), 1.56-1.63 (2H, m), 1.82-1.88 (2H, m), 2.30 (2H, t, J = 7.6 Hz), 3.41 (2H, t, J = 7.6 Hz), 3.73 (3H, s)
IR (KBr): 1742 $cm^{-1}$ EI-MS m/z: 293 ($M^+$)
HRMS. Calculated for $C_{13}H_{25}O_2Br$: 292.1038
Found: 292.1045

(2) 2-(2,3,4,6-Tetra-O-acetyl-1-thio-β-D-glucopyranosyl)thiopseudourea hydrobromide (3.51 g, 7.37 mmol) synthesized by the method of S. Saito and T. Tsuchiya (Chem. Pharm. Bull., 33, 503, 1985), methyl 12-bromododecanoate (2.59 g, 8.84 mmol), $K_2CO_3$ (1.04 g), and $NaHSO_3$ (0.70 g) were stirred in acetone (70 ml)/water (70 ml) at room temperature for 16.5 hr. The reaction solution was distributed in chloroform and water. The organic layer was dried over $MgSO_4$ and then filtered, followed by concentration to dryness. The residue was purified by column chromatography on silica gel (toluene-ethyl acetate (95 : 5)) to give 2.38 g (57%) of 11-methoxycarbonylundecyl 2,3,4,6-tetra-O-acetyl-1-thio-β-D-glucopyranoside.

$[\alpha]_D$ -26.3° (c = 1.02, chloroform)
$^1$H-NMR ($CDCl_3$) δ: 1.26-1.37 (14H, m), 1.52-1.63 (4H, m), 2.01 (3H, s), 2.03 (3H, s), 2.06 (3H, s), 2.08 (3H, s), 2.30 (2H, t, J = 7.6 Hz), 2.63-2.70 (2H, m), 3.67 (3H, s), 3.69-3.72 (1H, m), 4.14 (1H, dd, J = 2.4, 12.5 Hz), 4.24 (1H, dd, J = 5.1, 12.5 Hz), 4.47 (1H, d, J = 10.0 Hz), 5.03 (1H, dd, J = 9.3, 10.0 Hz), 5.09 (1H, dd, J = 9.5, 9.8 Hz), 5.22 (1H, dd, J = 9.3, 9.5 Hz)
IR (KBr): 1748, 1734 $cm^{-1}$ FAB-MS m/z: 577 ($MH^+$)

| Elementary analysis: | | |
|---|---|---|
| Calculated for $C_{27}H_{44}O_{11}S$: | C, 56.23; | H, 7.69 |
| Found: | C, 56.10; | H, 7.73 |

(3) A methanol solution of sodium methoxide (28%, 0.30 ml) was added under ice cooling to a solution of 11-methoxycarbonylundecyl 2,3,4,6-tetra-O-acetyl-1-thio-β-D-glucopyranoside (1.447 g, 2.5 mmol), obtained in step (2), in methanol (20 ml) and THF (10 ml). The mixture was stirred for 5 hr. Acetic acid (0.089 ml) was added to the reaction solution. The mixture was concentrated to dryness. The residue was purified by column chromatography on silica gel (chloroform-methanol (95 : 5)) to give 400 mg of the title compound.

$[\alpha]_D$ -37.9° (c = 1.00, methanol: chloroform = 1:1)
$^1$H-NMR ($CD_3OD$) δ: 1.30-1.42 (8H, m), 1.58-1.64 (4H, m), 2.31 (2H, t, J = 7.6 Hz), 2.67-2.76 (2H, m), 3.19 (1H, dd, J = 8.6, 9.5 Hz), 3.26-3.35 (3H, m), 3.64 (3H, s), 3.65 (1H, dd, J = 5.4, 12.0 Hz), 3.85 (1H, dd, J = 2.2, 12.0 Hz), 4.33 (1H, d, J = 9.8 Hz)
IR (KBr): 1740 $cm^{-1}$ FAB-MS m/z: 409 ($MH^+$)

| Elementary analysis: | | |
|---|---|---|
| Calculated for $C_{19}H_{36}O_7S$: | C, 55.86; | H, 8.88 |
| Found: | C, 55.56; | H, 8.92 |

Example 22 Glc-S-$C_{11}$-COOH (12-(1-thio-β-D-glucopyranosyl)dodecanoic acid

An aqueous solution (8 ml) of sodium hydroxide (80 mg, 2.0 mmol) and THF (4 ml) were added to a methanol solution (8 ml) of 11-methoxycarbonylundecyl 1-thio-β-D-glucopyranoside (400 mg, 1.0 mmol). The mixture was stirred at room temperature for 6 hr. The reaction solution was concentrated. The concentrate was adjusted to pH 2 by addition of 2 N hydrochloric acid. The resultant precipitate was collected by filtration to give 380 mg (96%) of the title compound.

$[\alpha]_D$ -37.6° (c = 1.02, methanol)
$^1$H-NMR (CD,OD) δ: 1.27-1.42 (14H, m), 1.57-1.64 (4H, m), 2.27 (2H, t, J = 7.6 Hz), 2.67-2.75 (2H,m), 3.19 (1H, dd, J = 8.6, 9.5 Hz), 3.25-3.36 (3H, m), 3.66 (1H, dd, J = 5.4, 12.0 Hz), 3.85 (1H, dd, J = 2.0, 12.0 Hz), 4.34 (1H, d, J = 9.8 Hz)
IR (KBr): 1726 $cm^{-1}$ FAB-MS m/z: 395 ($MH^+$)

| Elementary analysis: | | |
|---|---|---|
| Calculated for $C_{18}H_{34}O_7S$: | C, 54.79; | H, 8.69 |
| Found: | C, 54.76; | H, 8.64 |

Example 23 Glc-S-$C_{11}$-AVP

Triethylamine (0.020 ml) was added to a methanol solution (2 ml) of AVP (40 mg, 0.04 mmol). The mixture was concentrated to dryness. DMF (2 ml), 12-(1-thio-β-D-glucopyranosyl)dodecanoic acid (50 mg, 0.14 mmol) obtained in Example 22, DCC (33 mg, 0.16 mmol), HOBt (22 mg, 0.16 mmol) were added to the residue. The mixture was stirred at room temperature for 19.5 hr. The reaction solution was adjusted to pH 2.0 by addition of 10% TFA and then purified by HPLC to give 13 mg of the title compound comprising a glucose derivative introduced into AVP at its N end.

$[\alpha]_D$ -83.2 (c = 0.19, $H_2O$)
$^1$H-NMR ($D_2O$) δ: 1.30-1.38 (14H, m), 1.52-1.71 (5H, m), 1.79-1.82 (1H, m), 1.91-1.97 (2H, m), 2.00-2.34 (6H, m), 2.68-2.80 (3H, m), 2.85 (1H, d, J = 6.6 Hz), 2.95-3.07(3H, m), 3.14-3.24 (3H, m), 3.29-3.51 (5H, m), 3.69-3.97 (6H, m), 4.14-4.17 (1H, m), 4.32-4.35 (1H, m), 4.44-4.51 (2H, m), 4.57-4.75 (3H, m), 6.79 (2H, d, J = 8.1 Hz), 7.01 (2H, d, J = 8.1 Hz), 7.26 (2H, d, J = 7.3 Hz), 7.36-7.44 (3H, m)
FAB-MS m/z: 1460 ($MH^+$)
Amino acid composition (%) (hydrolyzates in 6 N hydrochloric acid): Gly 0.95, Arg, 1.00, Pro 1.01, Cys 1.32, Asp 1.00, Glu 1.01, Phe 0.99, Tyr0.93, ammonia 3.12 (recovery of Asp: 93%)

Example 24 $^3$H-Glc-S-$C_{11}$-AVP

A methanol solution of AVP (54 μl; concentration 0.56 mg/ml) and a methanol solution of triethylamine (50 μl; concentration 70 μl/ ml) were added to a 0.05 M acetic acid-ethanol (3 : 7) solution (0.22 ml) of $^3$H-AVP (2.86 μg, 220 μCi). The mixture was concentrated to dryness. A DMF solution of 12-(1-thio-β-D-glucopyranosyl)dodecanoic acid (5.85 μl; concentration 4.0 mg/0.2 ml) obtained in Example 22, a DMF solution of WSCI (5.59 μl; concentration 3.0 mg/0.4 ml), and a DMF solution of HOBt (8.91 μl; concentration 2.0 mg/0.3 ml) were added to the residue. The mixture was stirred at room temperature for 19 hr. The reaction solution was added to 50 μl of 0.05% TFA. The mixture was purified by HPLC to give a tritium-labeled product in an amount corresponding to 2.46 μg. The specific radioactivity was 3.32 Ci/mmol, and the amount of the product synthesized was 55.9 μCi.

Example 25 Glc-S-$C_8$-CONH-$C_2$-NBD

NBD-Cl (900 mg, 4.5 mmol), N-Boc-ethylenediamine (0.178 ml, 1.13 mmol), and $NaHCO_3$ (287 mg) were stirred in methanol (30 ml) at 55°C for 4 hr. The reaction solution was distributed in chloroform and water. The organic layer was dried over $MgSO_4$ and then filtered. The filtrate was concentrated to dryness. The residue was purified by column chromatography on silica gel (toluene-ethyl acetate (85 : 15)) to give 220 mg (67%) of a red amorphous compound. The red amorphous compound (50 mg, 0.15 mmol) and anisole (0.133 ml) were stirred in TFA (2 ml) at room temperature for 2.5 hr. The reaction solution was concentrated to dryness. Methanol (4 ml) and triethylamine (0.2 ml) were added to the residue, followed by concentration to dryness, and this procedure was repeated twice. DMF (2 ml), 9-(1-thio-β-D-glucopyranosyl)nonanoic acid (67 mg, 0.21 mmol) obtained in Example 18, DCC (47 mg, 0.30 mmol), and HOBt (47 mg, 0.30 mmol) were added to the residue. The mixture was stirred at room temperature for 16 hr. The reaction solution was adjusted to pH 2.0 by addition of 10% TFA and then purified by HPLC to give 60 mg of the title compound.

$^1$H-NMR ($CD_3OD$) δ: 1.25-1.36 (8H, m), 1.53-1.60 (4H, m), 2.17 (1H, t, J = 7.6 Hz), 2.64-2.74 (2H, m), 3.19 (1H, dd, J = 8.8, 9.5 Hz), 2.64-2.74 (2H, m), 3.19 (1H, dd, J = 8.8, 9.5 Hz), 3.26-3.36 (2H, m), 3.54 (1H, t, J = 5.9 Hz),

3.63-3.67 (1H, m), 3.65 (1H, dd, J = 5.4, 12.0 Hz), 3.85 (1H, dd, J = 1.5, 12.0 Hz), 4.34 (1H, d, J = 9.5 Hz), 6.42 (1H, d, J = 8.8 Hz), 8.53(1H, d, J = 8.8 Hz)
IR (KBr): 1622, 1588 $cm^{-1}$ FAB-MS m/z: 558 ($MH^+$)

| Elementary analysis: | | | |
|---|---|---|---|
| Calculated for $C_{23}H_{35}O_9N_5S \cdot 9/4H_2O$: | C, 45.49; | H, 6.72; | N, 11.53 |
| Found: | C, 45.61; | H, 6.72; | N, 11.53 |

Example 26 Glc-S-$C_8$-Tryptamine

Tryptamine (32 mg, 0.2 mmol), 9-(1-thio-β-D-glucopyranosyl)nonanoic acid (88 mg, 0.30 mmol) obtained in Example 18, DCC (82 mg, 0.40 mmol), and HOBt (54 mg, 0.40 mmol) were stirred in DMF (2 ml) at room temperature for 18 hr. The reaction solution was adjusted to pH 2.0 by addition of 10% TFA and then purified by HPLC to give 20 mg of the title compound comprising a glucose derivative introduced into tryptamine.

$^1H$-NMR ($D_2O$) δ: 1.06-1.10 (2H, m), 1.16-1.22 (2H, m), 1.30-1.36 (2H, m), 1.40-1.46 (2H, m), 1.60-1.64 (2H, m), 2.14 (1H, t, J = 7.3 Hz), 2.69-2.78 (2H, m), 3.02 (1H, t, J = 6.6 Hz), 3.31 (1H, dd, J = 9.0, 9.8 Hz), 3.39-4.42 (2H, m), 4.49 (1H, d, J = 10.0 Hz), 7.18 (1H, dd, J = 7.1, 7.3 Hz), 7.25-7.28 (2H, m), 7.52 (1H, d, J = 8.3 Hz), 7.71 (1H, d, J = 8.1 Hz)
IR (KBr): 1634, 1546 $cm^{-1}$ FAB-MS m/z: 495 ($MH^+$)

| Elementary analysis: | | | |
|---|---|---|---|
| Calculated for $C_{25}H_{36}O_6\ N_2S \cdot 3/2\ H_2O$: | C, 57.78; | H, 7.56; | N, 5.39. |
| Found: | C, 57.61; | H, 7.21; | N, 5.39 |

Example 27 $^3H$-Glc-S-$C_8$-Tryptamine

A methanol solution of tryptamine (55 µl; concentration 1.43 mg/ml) was added to an ethanol solution (0.25 ml) of $^3H$-tryptamine (2.4 µg, 250 µCi). The mixture was concentrated to dryness. A DMF solution of 9-(1-thio-β-D-glucopyranosyl)nonanoic acid obtained in Example 18 (8.1 µl; concentration 2.2 mg/0.1 ml), a DMF solution of WSCI (7.3 µl; concentration 3.2 mg/0.2 ml), and a DMF solution of HOBt (3.3 µl; concentration 2.2 mg/0.1 ml) were added to the residue. The mixture was stirred at room temperature for 19 hr. The reaction solution was added to 50 µl of 0.05% TFA and then purified by HPLC to give a tritium-labeled product in an amount corresponding to 1.80 µg. The specific radioactivity was 7.12 Ci/mmol, and the amount of the product synthesized was 29.35 µCi.

Example 28 Man-S-$C_8$-COOMe (8-methoxycarbonyloctyl 1-thio-β-D-mannopyranoside)

(1) A solution of 3.42 g (8.76 mmol) of D-mannose pentaacetate in dichloromethane (30 ml) was cooled to 0°C, and 10 ml of a solution of 25% hydrogen bromide in acetic acid was added thereto. The mixture was stirred at room temperature for 2 hr. After the completion of the reaction, the reaction solution was extracted with chloroform, followed by washing with water and an aqueous $NaHCO_3$ solution. The washed product was dried over $Na_2SO_4$ and concentrated under the reduced pressure at a temperature of 30°C or below. Tetrabutylammonium thioacetate prepared from 2.43 g (8.76 mmol) of tetrabutylammonium chloride and 1 g (8.76 mmol) of potassium thioacetate was added to 80 ml of a solution of the resultant oil in toluene. The mixture was stirred overnight at room temperature. The solvent was removed by distillation. The residue was extracted with chloroform and washed with water. After drying ($Na_2SO_4$), the washed product was concentrated under the reduced pressure. The concentrate was purified by column chromatography on silica gel (300 cc, ethyl acetate-hexane = 1 : 3) and recrystallized from dichloromethane-ether to give 596.5 mg of 2,3,4,6-tetra-O-acetyl-1-S-acetyl-1-thio-β-D-mannopyranose.

$[\alpha]_D^{24}$ -24.4° (c = 0.66, chloroform)

$^1$H-NMR ($CDCl_3$) δ: 5.50 (1H, s), 5.49 (1H, d, J = 4.4 Hz), 5.26 (1H, t, J = 10.0 Hz), 5.15 (1H, dd, J = 3.3, 10.0 Hz), 4.27 (1H; dd, J = 5.3, 12.4 Hz), 4.12 (1H, dd, J = 2.3, 12.3 Hz), 3.82 (1H, ddd, J = 2.3, 5.3, 9.9 Hz), 2.37 (3H, s), 2.19-1.98 (12H, s)

(2) A methanol solution (5 ml) of 0.5 g (1.23 mmol) of 2,3,4,6-tetra-O-acetyl-1-S-acetyl-1-thio-β-D-mannose obtained in step (1) was cooled to -30°C. A methanol solution (2 ml) of 28.3 mg (1.23 mmol) of sodium was added thereto. The mixture was stirred in an argon atmosphere at -20°C for 30 min and then concentrated under the reduced pressure. The concentrate was dissolved in DMF (4 ml). A solution of 0.55 g (1.60 mmol) of tosylated methyl 9-hydroxynonanoate in DMF (4 ml) was added to the solution. The mixture was stirred at room temperature overnight. The reaction solution was then extracted with ethyl acetate and washed with water. The washed product was dried over $Na_2SO_4$ and then concentrated under the reduced pressure. The concentrate was purified by column chromatography on silica gel (ethyl acetate-hexane = 1 : 2) to give 612 mg of 8-methoxycarbonyloctyl 2,3,4,6-tetra-O-acetyl-1-thio-β-D-mannopyranoside. Deprotection was carried out with sodium methoxide in methanol, followed by recrystallisation from methanol-ether to give the title compound.

FAB-MS m/z: 367 ($MH^+$)
$[\alpha]_D^{22}$ -55.8° (c = 1.24, chloroform-methanol = 1:1)
$^1$H-NMR ($CD_3OD$) δ: 4.70(1H, s), 3.87 (1H,d, J = 3.3 Hz), 3.85(1H, d, J = 3.3 Hz), 3.85 (1H, dd, J = 11.9 Hz, 2.4 Hz), 3.71 (1H, dd, J = 11.9 Hz, 5.5 Hz), 3.65 (3H, s), 3.58 (1H, t, J = 9.5 Hz), 3.47(1H, dd, J = 9.4 Hz, 3.5 Hz), 3.23 (1H, ddd, J = 9.6 Hz, 5.6 Hz, 2.4 Hz), 2.71 (2H,m), 2.31 (2H, t), 1.63 (4H, m), 1.32 (8H, m)

Example 29 <u>All-S-$C_8$-COOMe (8-methoxycarbonyloctyl 1-thio-β-D-allopyranoside)</u>

(1) A DMF suspension (8 ml) of potassium thioacetate (1.6 g, 14.0 mmol) was added to a solution of a tosylation product (2.4 g, 7.00 mmol) prepared from methyl 9-hydroxynonanoate. The mixture was stirred at room temperature overnight. The reaction solution was then extracted with ethyl acetate, followed by washing with water. After drying over $NaSO_4$, the product was concentrated under the reduced pressure. The concentrate was purified by column chromatography on silica gel (ethyl acetate-hexane = 1 : 20) to give 1.38g of methyl 9-S-acetylthiononanoate. A methanol solution (6 ml) of methyl 9-S-acetylthiononanoate (600 mg, 2.44 mmol) thus obtained was cooled to -10 °C. A methanol solution (6ml) of sodium (56mg, 2.44mmol) were added thereto. The mixture was stirred in an argon atmosphere at -5°C for 20 min, and then neutralized with an ion exchange resin Amberlyst 15. The resin was removed by filtration. The filtrate was concentrated under the reduced pressure to give 479 mg of methyl 9-mercaptononanoate.

$[\alpha]_D^{22}$ -0.03° (c = 0.62, chloroform)
$^1$H-NMR($CDCl_3$) δ: 3.67(3H, s), 2.52 (2H, q, J = 7.4 Hz), 2.30(2H, t, J = 7.6 Hz), 1.60 (4H, m), 1.34 (8H, m)

(2) A solution of β-D-allopyranose pentaacetate and methyl 9-mercaptononanoate (135 mg, 0.66 mmol) obtained in step (1) in dichloromethane solution (2 ml) was cooled to 0°C. Boron trifluoride etherate (81 μl, 0.66 mmol) was added thereto. The mixture was stirred at room temperature for 4 hr. After the completion of the reaction, the reaction solution was extracted with chloroform. The extract was washed with an $NaHCO_3$ solution and water, dried over $Na_2SO_4$, and concentrated under the reduced pressure. The concentrate was purified by column chromatography on silica gel (50 cc, ethyl acetate-hexane = 1 : 3) to give 125 mg of 8-methoxycarbonyloctyl 2,3,4,6-tetra-O-acetyl-1-thio-β-D-allopyranoside.

(3) 8-Methoxycarbonyloctyl 2,3,4,6-tetra-O-acetyl-1-thio-β-D-allopyranoside obtained in step (2) was deprotected in methanol with sodium methoxide and recrystallised from methanol-ether to give 51 mg of the target compound.

Rf = 0.54 (chloroform-methanol = 5 : 1), m.p. 66-68 °C
FAB-MS m/z: 367 ($MH^+$)
$[\alpha]_D^{22}$ -22.2° (c = 0.44, chloroform-methanol = 1:1)
$^1$H-NMR ($CD_3OD$) δ: 4.69 (1H, d, J = 9.8 Hz), 4.06 (1H, t, J = 2.8 Hz), 3.82 (1H, m), 3.65 (3H, s) ,3.63 (2H, m), 3.47 (1H,dd,J = 9.6 Hz,3.0 Hz),3.36 (1H, dd, J = 9.9 Hz, 2.7 Hz),2.70 (2H, m),2.31 (2H, t)1.61 (4H, m),1.32 (8H, m)

Example 30 <u>Xyl-S-$C_8$-COOMe (8-methoxycarbonyloctyl 1-thio-β-D-xylopyranoside</u>

A solution of a tetraacetate, prepared from 200 mg (1.33 mmol) of D-xylose, and 408 mg (2.00 mmol) of methyl 9-mercaptononanoate in dichloromethane (6 ml) was cooled to 0°C. Boron trifluoride etherate (245 μl, 2.00 mmol) was added thereto. The mixture was stirred at room temperature overnight. The reaction solution was extracted with chloroform. The extract was washed with an aqueous $NaHCO_3$ solution and water, dried over $Na_2SO_4$, and concentrated under the reduced pressure. The concentrate was purified by column chromatography on silica gel (120 cc, ethyl acetate-hexane = 1 : 3) to give 186 mg of 8-methoxycarbonyloctyl 2,3,4-tri-O-acetyl-1-thio-β-D-xylopyranoside. This compound was deprotected in methanol with sodium methoxide and recrystallised from methanol-ether to give 101 mg of the title compound.

Rf = (chloroform-methanol = 5 : 1), m.p. 78-80 °C
FAB-MS m/z:337 ($MH^+$)
$[\alpha]_D^{24}$ -39.7° (c = 0.70, chloroform-methanol = 1 : 1)
$^1$H-NMR ($CD_3OD$) δ: 4.29 (1H, d, J = 9.4 Hz),3.91 (1H, dd, J = 11.4 Hz,5.3 Hz), 3.65 (3H, s),3.49 (1H, m), 3.30 (1H, t, J = 8.6 Hz), 3.20 (1H, t, J = 10.8 Hz),3.18 (1H, t, J = 9.0 Hz),2.66 (2H, m), 2.31 (2H, t), 1.61 (4H, m), 1.36 (8H, m)

Example 31 2dGlc-S-$C_8$-COOMe (8-methoxycarbonyloctyl 2-deoxy-1-thio-β-D-glucopyranoside)

In the same manner as in Example 30, the title compound was obtained from 563 mg (1.69 mmol) of 1,3,4,6-tetra-O-acetyl-2-deoxy-D-glucopyranose and 519 mg (2.54 mmol) of methyl 9-mercaptononanoate.

Rf = 0.54 (chloroform-methanol = 5 : 1), m.p. 90-92°C
FAB-MS m/z: 351 ($MH^+$)
$[\alpha]_D^{23}$ -48.1° (c = 1.11, chloroform-methanol = 1:1)
$^1$H-NMR ($CD_3OD$) δ: 4.63 (1H, dd, J = 11.8 Hz,1.8 Hz), 3.85 (1H, dd, J = 12.1 Hz, 2.1 Hz), 3.68 (1H, dd, J = 11.8 Hz, 5.1 Hz),3.65 (3H, s),3.56 (1H, m), 3.20 (2H, m), 2.71 (2H, m), 2.31 (2H, t), 2.14 (1H, ddd, J = 12.7 Hz, 5.3 Hz, 1.8 Hz), 1.59 (4H, m), 1.32 (8H, m)

Example 32 5S-Glc-S-$C_8$-COOMe (8-methoxycarbonyloctyl 5-deoxy-1,5-dithio-β-D-glucopyranoside)

In the same manner as in Example 30, the title compound was obtained from a pentaacetate prepared from 40 mg (0.20 mmol) of 5-thio-D-glucose and 72 mg (0.30 mmol) of methyl 9-mercaptononanoate.

Rf = 0.24 (chloroform-methanol = 5 : 1), m.p. 62-64 °C
ES-MS m/z: 405 $(M+Na)^+$
$[\alpha]_D^{22}$ -12.2° (c = 0.82, chloroform-methanol = 1 : 1)
$^1$H-NMR ($CD_3OD$) δ: 3.92 (1H,dd,J = 11.4 Hz, 3.7 Hz), 3.72 (1H,dd,J = 11.5 Hz, 6.5 Hz), 3.71 (1H, d, J = 10.3 Hz), 3.65 (3H, s), 3.50 (1H, dd, J = 10.3 Hz, 9.0 Hz), 3.36 (1H, dd, J = 10.3 Hz, 8.7 Hz), 3.16 (1H, t, J = 8.9 Hz), 2.89 (1H, ddd, J = 10.2 Hz, 6.5 Hz, 3.7 Hz), 2.73 (2H, m), 2.32 (2H, t), 1.61 (4H, m), 1.32 (8H, m)

Example 33 Glc-NH-$C_7$ -COOMe (N-(7-methoxycarbonyl)heptyl-β-D-glucopyranosylamine)

D-glucose (1 g, 5.55 mmol) and 0.88 g (5.55 mmol) of 8-aminooctanoic acid were dissolved in 50 ml of methanol. The solution was stirred in an argon atmosphere at 70°C overnight. The reaction solution was concentrated under the reduced pressure and adsorbed onto Diaion HP-20(500 cc). Elution was then carried out with water-methanol = 1 : 1. The eluate was purified by gel filtration column chromatography (LN-20: 300 cc, methanol). The resultant syrup was added to a methanol solution (3 ml), and 1.39 g of trimethylsilyldiazomethane (10% hexane solution) was added thereto. The mixture was stirred overnight and concentrated under the reduced pressure. The concentrate was adsorbed onto Diaion HP-20 (500 cc). Elution was then carried out with water-methanol = 3 : 1 to give the title compound.

Rf = 0.50 (butanol-acetic acid-water = 2 : 1 : 1)
TS-MS m/z: 336 ($MH^+$)
$[\alpha]_D^{23}$ -29.7° (c = 0.52, methanol-water = 1 : 1)
$^1$H-NMR ($CD_3OD$) δ: 4.00 (1H, dd, J = 12.2 Hz, 1.4 Hz), 3.89 (1H, m), 3.78 (1H, dd, J = 9.6 Hz, 3.5 Hz), 3.65 (3H, s), 3.59 (1H, dd, J = 12.3 Hz, 1.8 Hz), 2.63 (2H, m), 2.31 (2H, t), 1.61(2H, m), 1.51 (2H, m), 1.34 (6H, m)

Example 34 Glc-S-$C_9$-OH (8-hydroxyoctyl 1-thio-β-D-glucopyranoside)

A DMF solution (2 ml) of 8-boromooctanol (230 mg, 1.10 mmol) was added to a solution of 218 mg (1.00 mmol) of a sodium salt of 1-thio-β-D-glucose in a mixture of water (5 ml) with DMF (3 ml). The mixture was stirred at room temperature overnight. After the completion of the reaction, the reaction solution was concentrated under the reduced pressure. The concentrate was purified by column chromatography on silica gel (chloroform-methanol = 10 : 1) to give 67 mg of the title compound.

Rf = 0.28 (chloroform-methanol = 5 : 1)
ES-MS m/z: 325 ($MH^+$)
$[\alpha]_D^{27}$ -51.9° (c = 0.11, chloroform-methanol = 1 : 1)
$^1$H-NMR ($CD_3OD$) δ: 4.34 (1H, d, J = 9.8 Hz), 3.85 (1H, dd, J = 12.1 Hz, 2.0 Hz), 3.65 (1H, dd, J = 12.1 Hz, 5.5 Hz), 3.54 (2H, t, J = 6.6 Hz), 3.35 (1H, t, J = 8.6 Hz), 3.30 (1H, t, J = 9.8 Hz), 3.27 (1H, m), 3.19 (1H, dd, J = 9.8 Hz, 8.6 Hz), 2.72 (2H, m), 1.64 (2H, m), 1.52 (2H, m), 1.38 (8H, m)

Example 35 Glc-S-$C_8$-SH (8-mercaptooctyl 1-thio-β-D-glucopyranoside

1,8-Dibromooctane (5.44 g, 20.0 mmol) was dissolved in 30 ml of DMF. Potassium thioacetate (1.14 g, 9.98 mmol) was added to the solution. The mixture was stirred at room temperature for 8 hr. The reaction solution was extracted with ethyl acetate. The extract was washed with water, dried over $Na_2SO_4$, and concentrated under the reduced pressure. The concentrate was purified by column chromatography on silica gel (600 cc, ethyl acetate-hexane = 1 : 50) to give a thioacetyl derivative. In the same manner as in Example 34, a thioglycoside derivative was obtained from the thioacetyl derivative and a sodium salt of 1-thio-β-D-glucose. A catalytic amount of sodium methoxide was added to a methanol solution of the thioglycoside derivative in an argon atmosphere. The mixture was stirred at room temperature for 30 min. The reaction solution was neutralized with an ion exchange resin (Amberlyst 15) and concentrated under the reduced pressure. The concentrate was adsorbed onto Diaion HP-20 (100 cc). Elution was carried out with water-methanol (1 : 5) to give the title compound.

Rf = 0.54 (chloroform-methanol = 5 : 1) EI-MS m/z: 340$(M)^+$
$[\alpha]_D^{27}$ -61.6° (c = 0.10,chloroform-methanol = 1 : 1)
$^1$H-NMR ($CD_3OD$) δ: 4.34 (1H, d, J = 9.8 Hz), 3.85 (1H, dd, J = 12.1 Hz, 2.0 Hz), 3.65 (1H, dd, J = 11.9 Hz, 5.3 Hz), 3.32 (2H, m), 3.19 (1H, t, J = 9.2 Hz), 2.71 (2H, m), 2.49 (2H, t, J = 7.0 Hz), 1.61 (4H, m), 1.37 (8H, m)

Example 36 Glc-S-$C_8$-$NH_2$ (8-aminooctyl 1-thio-β-D-glucopyranoside)

Sodium azide (98 mg, 1.50 mmol) was added to a DMF solution (3 ml) of 209 mg (1.00 mmol) of 8-bromooctanol. The mixture was stirred at room temperature for 6 hr and then extracted with ethyl acetate. The extract was washed with water, dried over $Na_2SO_4$, and concentrated under the reduced pressure. The concentrate was dissolved in a mixed solvent composed of dichloromethane (2 ml) and pyridine (1 ml). p-Toluenesulfonyl chloride (285 mg, 1.50 mmol) was added thereto. The mixture was stirred at room temperature overnight. The reaction solution was extracted with chloroform. The extract was washed with 2 N hydrochloric acid and water, dried over $Na_2SO_4$, and concentrated under the reduced pressure to give 285 mg of a tosylation product. In the same manner as in Example 34, a thioglycoside derivative was obtained from this tosylation product and a sodium salt of 1-thio-β-D-glucose 191 mg (0.88 mmol). An ethanol solution (6 ml) of 100 mg of the thioglycoside derivative was catalytically hydrogenated in the presence of a Lindlar catalyst. The catalyst was removed by filtration and concentrated under the reduced pressure. The concentrate was purified by gel filtration column chromatography (LH-20: 200cc, methanol) to give the title compound.

ES-MS m/z: 324 ($MH^+$)
$[\alpha]_D^{27}$ -89.7° (c = 0.06, chloroform-methanol = 1 : 1)
$^1$H-NMR ($CD_3OD$) δ: 4.34 (1H, d, J = 9.8 Hz), 3.85 (1H, dd, J = 12.1 Hz, 2.0 Hz), 3.65 (1H, dd, J = 11.9 Hz, 5.3 Hz), 3.33 (3H, m), 3.19 (1H, t, J = 9.0 Hz), 2.72 (4H, m), 1.67 (2H, m), 1.52 (2H, m), 1.37 (8H, m)

Example 37 Glc-S-$C_1$-COOMe (methoxycarbonylmethyl 1-thio-β-D-glucopyranoside)

In the same manner as in Example 34, 182 mg of the title compound was obtained from 87 μl (0.92 mmol) of methyl bromoacetate and 200 mg (0.92 mmol) of a sodium salt of 1-thio-β-D-glucose.

FAB-MS m/z: 269 ($MH^+$), m.p. 100-102°C
$[\alpha]_D^{23}$ -52.1° (c = 0.89, chloroform-methanol = 1 : 1)
$^1$H-NMR ($CD_3OD$) δ: 4.49 (1H, d), 3.85 (1H, d), 3.73 (3H, s), 3.62 (2H, m), 3.34 (5H, m)

Example 38 Glc-S-$C_{15}$-COOMe (15-methoxycarbonyl-pentadecanoyl 1-thio-β-D-glucopyranoside)

Methyl 16-hydroxyhexadecanoate (200 mg, 0.70 mmol) was dissolved in a mixed solvent composed of dichloromethane (3 ml) and pyridine (3 ml). p-Toluenesulfonyl chloride (0.2 g, 1.05 mmol) was added to the solution. The mixture was stirred at room temperature overnight. The reaction solution was extracted with chloroform. The extract was washed with 2 N hydrochloric acid and water, dried over $Na_2SO_4$, and concentrated under the reduced pressure to give a tosylation product. In the same manner as in Example 34, 60 mg of the title compound was obtained from the tosylation product and 107 mg (0.49 mmol) of a sodium salt of 1-thio-β-D-glucose.

FAB-MS m/z: 465 ($MH^+$)
$[\alpha]_D^{23}$ -35.1° (c = 0.34, chloroform-methanol = 1 : 1)
$^1$H-NMR ($CD_3OD$) δ: 4.34 (1H, d, J = 9.4 Hz), 3.85 (1H, dd, J = 11.9 Hz, 2.0 Hz), 3.65 (4H, m), 3.20 (1H, t, J = 9.0 Hz), 3.33 (3H, m), 2.72 (2H, m), 2.31 (2H, t), 1.62 (4H, m), 1.29 (22H, m)

Example 39 Glc-S-CH(-$C_6$)-$C_{10}$-COOMe (methyl 12-(1-thio-β-D-glucopyranosyl)stearate)

Trimethylsilyldiazomethane (10% hexane solution) (2.28 g, 2.00 mmol) was added to a solution of 0.3 g (1.00 mmol) of 12-hydroxystearic acid in methanol (2 ml). The mixture was stirred at room temperature for 4 hr. The reaction solution was concentrated under the reduced pressure to give a methyl ester compound. In the same manner as in Example 38, 71.6 mg of the title compound was obtained from the methyl ester compound.

Rf = 0.59 (chloroform-methanol = 5 : 1)
TS-MS m/z: 510 $(M+NH_4)^+$
$[\alpha]_D^{24}$ -34.9 (c = 1.39, chloroform)
$^1$H-NMR ($CD_3OD$) δ: 4.38 (1H, d, J = 9.7 Hz), 3.83 (1H, dd, J = 11.9, 2.4 Hz), 3.67 (1H, dd, J = 11.8 Hz, 5.1 Hz), 3.65 (3H, s), 3.35 (2H, m), 3.24 (1H, m), 3.17 (1H, m), 2.95 (1H, m), 2.31 (2H, t), 1.60 (6H, m), 1.44 (2H, m), 1.30 (20H,s), 0.90 (3H, t)

Example 40 Glc-S-$C_1$-Ph-COOMe (p-(carboxymethyl)benzyl 1-thio-β-D-glucopyranoside)

A DMF solution (3 ml) of 0.33 g (1.51 mmol) of p-bromomethylbenzoic acid was added to a solution of 300 mg (1.37 mmol) of a sodium salt of 1-thio-β-D-glucose in a mixture of 3 ml of water with 3 ml of DMF. The mixture was stirred at room temperature overnight. The reaction solution was concentrated under the reduced pressure. The concentrate was dissolved in 3 ml of methanol. Trimethylsilyldiazomethane (10% hexane solution)(2.35g, 2.06 mmol) was added thereto. The mixture was stirred at room temperature overnight. The reaction solution was concentrated under the reduced pressure. The concentrate was purified by column chromatography on silica gel (100 cc, chloroform-methanol = 10 : 1) to give 109.4 mg of the title compound as an acicular crystal.

Rf = 0.38 (chloroform-methanol = 5 : 1),
EI-MS m/z: 344$(M)^+$
$[\alpha]_D^{24}$ -104.5° (c = 0.45, $CHCl_3$-$CH_3OH$ = 1 : 1) m.p. 163-165°C
$^1$H-NMR ($CD_3OD$) δ: 7.96 (2H, dd), 7.49 (2H, d), 4.15 (1H, d, J = 9.2 Hz), 4.10 (1H, d, J = 13.1 Hz), 3.89 (1H, d, J = 13.1 Hz), 3.90 (3H, s), 3.67 (1H, dd, J = 12.1, 5.9 Hz), 3.25 (5H, m)

Example 41 Glc-S-$C_8$-COO-DXR (14-[9-(1-thio-β-D-glucopyranosyl)nonanoyl]doxorubicin)

9-(1-Thio-β-D-glucopyranosyl)nonanoic acid (193 mg, 0.55 mmol) obtained in Example 18 and 378 mg (0.55 mmol) of 14-bromodaunomycin were dissolved in 5 ml of DMF. The solution was cooled to 0°C. N,N-Diisopropylethylamine (115 μl (0.66 mmol) was added thereto. The mixture was stirred at room temperature overnight. Diisopropyl ether was added thereto. The resultant red oil was adsorbed onto Diaion HP-20 (100 cc). Elution was carried out with water-methanol (1 : 3). The eluate was purified by column chromatography (LH-20: 100 cc, methanol) to give 77 mg of the title compound.

Rf = 0.41 (butanol-acetic acid-water = 4 : 1 : 1)
FAB-MS m/z:878($MH^+$)
$[\alpha]_D^{20}$ 159.3° (c = 0.01, chloroform-methanol = 1 : 1)
$^1$H-NMR ($CD_3OD$ - $CDCl_3$) δ: 7.95 (1H, broad d), 7.82 (1H, broad t), 7.51 (1H, broad d), 5.49 (1H, broad d), 5.34 (1H, d, J = 17.7 Hz), 5.14 (1H, s)5.12 (1H, d, J = 18.0 Hz), 4.36 (1H, d, J = 9.8 Hz), 4.29 (1H, q, J = 6.7 Hz), 4.06

(3H, s), 3.86 (1H, dd, J = 11.9 Hz, 2.4 Hz), 3.69 (2H, m), 3.55 (1H, m), 3.41-3.29 (3H, m), 3.23 (1H, t, J = 9.2 Hz), 3.13 (1H, d, J = 19.0 Hz), 2.93 (1H, d, J = 18.7 Hz), 2.73 (2H, m), 2.47 (3H, m), 2.13 (1H, m), 2.04 (1H, m), 1.89 (1H, m), 1.67 (4H, m), 1.39 (8H, m), 1.34 (3H, d, J = 6.7 Hz)

Example 42 Glc-S-$C_3$-CONH-DXR (N-[9-(1-thio-β-D-glucopyranosyl)nonanoyl]doxorubicin)

DCC (248 mg, 1.20 mmol) was added to 3 ml of a DMF solution of 210 mg (0.60 mmol) of 9-(1-thio-β-D-glucopyranosyl)nonanoic acid obtained in Example 18. The mixture was stirred for 30 min. A DMF solution (3 ml) of 348 mg (0.60 mmol) of doxorubicin was added thereto. The mixture was further stirred at room temperature overnight. Diisopropyl ether was added thereto. The resultant red oil was purified by column chromatography on silica gel (100 cc, chloroform-methanol = 5 : 1) and then by gel filtration chromatography (LH-20: 300 cc, methanol) to give 40 mg of the title compound.

FAB-MS m/z: 878($MH^+$)
$[\alpha]_D^{22}$ 66.5° (c = 0.01, chloroform-methanol = 1 : 1)
$^1$H-NMR ($CD_3OD$) δ: 7.88 (2H, broad s), 7.48 (1H, broad s), 5.40 (1H, broad s), 5.07 (1H, broad s), 4.75 (2H, s), 4.29 (1H, d, J = 9.7 Hz), 4.27 (1H, m), 4.13 (1H, m), 3.99 (3H, s), 3.82 (1H, dd, J = 12.2 Hz, 1.9 Hz), 3.62 (2H, m), 3.34-3.21 (3H, m), 3.15 (1H, t, J = 9.0 Hz), 3.01 (1H, broad d), 2.86 (1H, broad d), 2.60 (2H, m), 2.34 (1H, m), 2.15 (2H, t), 1.98 (1H, m),1.72 (1H, m),1.53 (4H, m),1.27 (3H, d, J = 6.7 Hz), 1.24 (10H, m)

Example 43 Glc-S-$C_8$-COO-$C_2$-NBD (4-{2-[9-(1-thio-β-D-glucopyranosyl)nonanoyl]oxyethyl}amino-7-nitrobenz-2-oxa-1,3-diazole)

(1) NBD-Cl (1 g, 5.01 mmol) was dissolved in 50 ml of methanol. 2-Bromoethylamine hydrobromide (1.23 g, 6.01 mmol) and 2.09 ml (12.02 mmol) of N,N-diisopropylethylamine were added to the solution. The mixture was stirred at room temperature for 6 hr. The reaction solution was concentrated under the reduced pressure. The concentrate was purified by column chromatography on silica gel (300 cc, toluene-ethyl acetate = 10 : 1) to give 1.08 g of 4-(2-bromoethyl)amino-7-nitrobenz-2-oxa-1,3-diazole.

(2) 4-(2-Bromoethyl)amino-7-nitrobenz-2-oxa-1,3-diazole (80 mg, 0.28 mmol) obtained in step (1) and 98 mg (0.28 mmol) of 9-(1-thio-β-D-glucopyranosyl)nonanoic acid obtained in Example 18 were dissolved in 2 ml of DMF. N,N-Diisopropylethylamine (59 μl, 0.34 mmol) was added thereto. The mixture was stirred at room temperature overnight. The reaction solution was concentrated under the reduced pressure. The concentrate was purified by column chromatography on silica gel (30 cc, chloroform-methanol = 10 : 1) and then by gel filtration chromatography (LH-20: 300 cc, methanol) to give 36.2 mg of the title compound.

FAB-MS m/z: 559 ($MH^+$)
$^1$H-NMR ($CD_3OD$) δ: 8.50 (1H, d, J = 8.7 Hz), 6.44 (1H, d, J = 9.0 Hz), 4.40 (2H, t, J = 5.4 Hz), 4.35 (1H, d, J = 9.8 Hz), 3.86 (1H, dd, J = 11.9 Hz,1.9 Hz), 3.67 (1H, dd, J = 12.1 Hz, 5.1 Hz), 3.39-3.27 (3H, m), 3.20 (1H, dd, J = 9.6 Hz, 8.6 Hz), 2.69 (2H, m), 2.32 (2H, t, J = 9.2 Hz), 1.56 (4H, m), 1.32 (2H, m), 1.20 (6H, m)

Example 44 Glc-S-$C_8$-OCOO-$C_2$-NBD (4-{2-[8-(1-thio-β-D-glucopyranosyl)octyloxycarbonyl]oxyethyl}amino-7-nitrobenz-2-oxa-1,3-diazole)

(1) NBD-Cl (2 g, 10.0 mmol) was dissolved in 80 ml of methanol. An aqueous solution (20 ml) of 0.73 ml (12.0 mmol) of ethanolamine and 2.52g (30.0 mmol) of sodium hydrogencarbonate was added thereto. The mixture was stirred at room temperature overnight. The reaction solution was concentrated under the reduced pressure. The concentrate was purified by column chromatography on silica gel (400 cc, toluene-ethyl acetate = 5 : 1) to give 1.99 g of 4-(2-hydroxyethyl)amino-7-nitrobenz-2-oxa-1,3-diazole.

$^1$H-NMR ($CD_3OD$) δ: 8.52 (1H, d, J = 8.9 Hz), 6.41 (1H, d, J = 8.9 Hz), 3.86 (2H, t, J = 5.5 Hz), 3.68 (2H, broad s).

(2) A catalytic amount of sodium ethoxide was added to 15 ml of a THF solution of 8-bromooctanol (0.5 g, 2.39 mmol) and 0.58 g (3.59 mmol) of 1,1'-carbonyldiimidazole. The mixture was stirred at room temperature for 5 hr. The reaction solution was concentrated under the reduced pressure. The concentrate was purified by column chromatography on silica gel (140 cc, hexane-ethyl acetate = 1 : 2) to give 0.57 g of an imidazolide derivative.

$^1$H-NMR($CDCL_3$) δ: 8.14 (1H, s), 7.43 (1H, m), 7.07 (1H, m), 4.42 (2H, t, J = 6.7 Hz), 3.41 (2H, t, J = 6.8 Hz), 1.83 (4H, m), 1.40 (8H, m).

(3) A THF solution (20 ml) of 0.51 g (2.28 mmol) of 4-(2-hydroxyethyl)amino-7-nitrobenz-2-oxa-1,3-diazole obtained in step (1) and the imidazolide derivative (6.84 mmol) obtained in step (2) was stirred in the presence of a catalytic amount of sodium ethoxide at 70°C for 5 days. The reaction solution was concentrated under the reduced pressure. The concentrate was purified by column chromatography on silica gel (140 cc, hexane-ethyl acetate = 1 : 1) to give 144 mg of 4-[2-(8-bromooctyloxycarbonyl)oxyethyl]amino-7-nitrobenz-2-oxa-1,3-diazole.

$^1$H-NMR($CDCL_3$) δ: 8.50 (1H, d, J = 8.5 Hz), 6.72 (1H, broad t), 6.26 (1H, d, J = 8.7 Hz), 4.51 (2H, t, J = 5.3 Hz), 4.17 (2H, t, J = 6.7 Hz), 3.84 (2H, q, J = 5.4 Hz), 3.41 (2H, t, J = 6.8 Hz), 1.85 (2H, m), 1.67 (2H, m),1.44-1.32 (8H, m).

(4) A DMF solution (3 ml) of 207 mg (0.93 mmol) of a sodium salt of 1-thio-β-D-glucose and 352 mg (0.93 mmol) of tetra-n-butylammonium iodide was added to 144 mg (0.31 mmol) of 4[2-(8-bromooctyloxycarbonyl)oxyethyl]amino-7-nitrobenz-2-oxa-1,3-diazole obtained in step (3). The mixture was stirred at room temperature overnight. Isopropyl ether was added thereto. The resultant yellow oil was purified by column chromatography on silica gel (150 cc, chloroform-methanol = 10 : 1) and then by gel filtration chromatography (LH-20: 300 cc, methanol) to give 86.2 mg of the title compound.

LC-MS m/z(ES): 592$(M+NH_4)^+$
$^1$H-NMR ($CD_3OD$) δ: 8.46 (1H, d, J = 8.7 Hz), 6.38 (1H, d, J = 8.7 Hz),4.40 (2H, t, J = 5.4 Hz),4.31 (1H, d, J = 9.8 Hz), 4.04 (2H t, J = 6.5 Hz), 3.82 (1H, dd, J = 12.1 Hz, 2.1 Hz), 3.62 (1H, dd, J = 12.1 Hz, 5.4 Hz), 3.35-3.26 (3H, m), 3.16 (1H, t, J = 9.1 Hz), 2.66 (2H, m), 1.56 (4H, m), 1.28 (8H, m)

Example 45 5N-Glc-S-$C_8$-CooMe (8-methoxycarbonyloctyl 5-amino-5-deoxy-1-thio-β-D-glucopyranoside hydrochloride)

(1) A methanol solution of 1.90 g (7.75 mmol) of 5-azido-5-deoxy-1,2-O-isopropylidene-α-D-glucofuranose (Carbohydrate Research, 68, 391-395, 1979) was adjusted to pH 2 by addition of a hydrochloric acid-methanol reagent 10 (manufactured by Tokyo Chemical Industry Co., Ltd.), followed by catalytic hydrogenation in the presence of 1 g of palladium-carbon at room temperature for 3 hr. The catalyst was removed by filtration. The filtrate was concentrated under the reduced pressure. The concentrate was dissolved in methanol. A solution of 3.01 g (11.6 mmol) of 9-fluorenylmethyl chloroformate in methylene chloride (30 ml) was added thereto. The mixture was cooled to 0°C. N,N-Diisopropylethylamine (2.70 ml, 15.5 mmol) was added thereto. The mixture was further stirred at 0°C for 2 hr. The reaction solution was concentrated under the reduced pressure. The concentrate was purified by column chromatography on silica gel (500 cc, chloroform-methanol = 80 : 1) to give 2.90 g of 5-deoxy-5-N-(9-fluorenylmethyloxycarbonyl)amino-1,2-O-isopropylidene-α-D-glucofuranose.

$[\alpha]_D^{24}$ -1.1° (c = 0.73, chloroform)
$^1$H-NMR ($CDCL_3$) δ: 7.75 (2H, d, J = 7.5 Hz), 7.56 (2H, d, J = 7.2 Hz), 7.38 (2H, t, J = 7.5 Hz), 7.29 (2H, t, J = 7.1 Hz), 5.92 (1H, d, J = 3.6 Hz), 5.47 (1H, broad t), 4.51 (1H, d, J = 3.6 Hz), 4.40 (2H, d, J = 6.7 Hz), 4.34 (1H, broad s), 4.19 (1H, t, J = 6.7 Hz), 4.13 (1H, broad s), 4.02 (1H, broad s), 3.56 (2H, m), 3.34 (1H, m), 1.47 (3H, s), 1.30 (3H, s)

(2) 5-Deoxy-5-N-(9-fluorenylmethyloxycarbonyl)amino-1,2-O-isopropylidene-α-D-glucofuranose (0.78 g, 1.77 mmol) obtained in step (1) was dissolved in 17 ml of TFA. Water (1.7 ml) was added to the solution. The mixture was allowed to stand at 0°C for 1.5 hr. The solvent was removed by azeotropic distillation with toluene under the reduced pressure. Pyridine (6 ml) and 12 ml of methylene chloride were added to the residue. The mixture was cooled to 0°C. Acetic anhydride (3 ml) was added thereto, and the mixture was allowed to stand overnight. Methanol (20 ml) was added thereto. The mixture was concentrated under the reduced pressure. The concentrate was extracted with chloroform. The extract was washed with 2 N hydrochloric acid and water, dried over $Na_2SO_4$, and concentrated under the reduced pressure. The residue was purified by column chromatography on silica gel (500 cc, ethyl acetate-hexane = 1 : 1) to give 0.82 g of a mixture of a tetraacetate derivative of α-glucopyranose with a tetraacetate derivative of β-glucopyranose.

LC-MS m/z (TS): 405 $(M+NH_4)$

(3) A methylene chloride solution (10 ml) of 0.81 g (1.42 mmol) of the tetraacetate derivative of glucopyranose obtained in step (2) was cooled to 0°C. A solution (5 ml) of 25% hydrogen bromide in acetic acid was added thereto. The mixture was further stirred at 0°C for 4 hr. The reaction solution was extracted with chloroform. The extract was

washed with water and then with an aqueous $NaHCO_3$ solution, dried over $Na_2SO_4$, and concentrated at 30°C or below under the reduced pressure. The concentrate was dissolved in 3 ml of DMF. A DMF solution (7 ml) of 0.32 g (2.84 mmol) of potassium thioacetate was added thereto. The mixture was stirred at room temperature overnight. The reaction solution was extracted with ethyl acetate. The extract was washed with water, dried over $Na_2SO_4$, and concentrated under the reduced pressure. The concentrate was purified by column chromatography on silica gel (160 cc, ethyl acetate-hexane = 1 : 2) to give 0.26 g of a β-S-acetyl derivative.

LC-MS m/z (TS): 603 $(M+NH_4)^+$
$[\alpha]_D^{23}$ 25.2° (c = 0.84, chloroform)
$^1$H-NMR($CDCl_3$) δ: 7.77 (2H, d, J = 7.4 Hz), 7.61 (2H, t, J = 8.2 Hz), 7.40 (2H, t, J = 7.4 Hz), 7.29 (2H, q, J = 6.9 Hz), 5.27 (1H, t, J = 9.2 Hz), 5.25 (1H, d, J = 10.3 Hz),5.14 (1H, broad s), 5.11 (1H, t, J = 9.7 Hz), 4.97 (1H, t, J = 9.7 Hz), 4.37 (2H, d, J = 7.2 Hz), 4.24 (2H, t, J= 7.1 Hz),3.74 (1H, m), 3.53 (1H, m), 3.30 (1H, m), 3.20 (1H, t), 2.40 (3H, s), 2.07-2.00 (9H, 3s)

(4) The β-S-acetyl derivative (70 mg, 0.12 mmol) obtained in step (3) was dissolved in a mixed solvent composed of methylene chloride (1 ml) and methanol (1 ml). The solution was cooled to -50 °C, and 120 μl of a methanol solution (1 mmol/ml) of sodium was added thereto. The mixture was stirred in an argon atmosphere at -50°C to -30°C for 30 min. The reaction solution was neutralized with an ion exchange resin Amberlyst 15. The resin was removed by filtration. The filtrate was concentrated under the reduced pressure. A solution of 61 mg (0.18 mmol) of a tosylation product of methyl 9-hydroxynonanoate in DMF (1 ml) and 31 μl (0.18 mmol) of N,N-diisopropylethylamine were added to the residue. The mixture was stirred at room temperature for 5 hr. The reaction solution was extracted with ethyl acetate. The extract was washed with water, dried over $Na_2SO_4$, and concentrated under the reduced pressure. The concentrate was purified by column chromatography on silica gel (ethyl acetate-hexane = 1 : 2) to give 70.7 mg of a thioglycoside derivative of nojirimycin.

FAB-MS m/z: 714 $(MH^+)$
$[\alpha]_D^{23}$ -2.3° (c = 0.48, chloroform)
$^1$H-NMR($CDCL_3$) δ: 7.77 (2H, d, J = 7.4 Hz), 7.60 (2H, t, J = 7.3 Hz), 7.40 (2H, m), 7.33 (2H, m), 5.21 (1H, t, J = 9.4 Hz), 5.25 (1H, d, J = 10.3 Hz), 5.16 (1H, broad t), 5.01 (1H, t, J = 9.6 Hz), 4.94 (1H, t, J = 9.6 Hz),4.46 (1H, d, J = 9.8 Hz), 4.43 (1H, m), 4.35 (1H, m), 4.22 (1H, t, J = 6.9 Hz), 3.66 (3H, s), 3.59 (2H, m), 3.32 (1H, m), 2.66 (2H, m), 2.27 (2H, t), 2.07-2.00 (9H, 3s), 1.59 (4H ,m), 1.28 (8H, m)

(5) Sodium methoxide was added to a methanol solution (2 ml) of 50 mg (0.07 mmol) of the thioglycoside derivative of nojirimycin obtained in step (4). The mixture was allowed to stand at 0°C for 5 hr. The reaction solution was rendered acidic by addition of a hydrochloric acid-methanol reagent 10 (manufactured by Tokyo Chemical Industry Co., Ltd.) and then concentrated under the reduced pressure. The concentrate was purified by gel filtration chromatography (LH-20: 50 cc, methanol) to give 21.8 mg of the title compound.

LC-MS m/z (ES): 366 $(MH^+)$
$[\alpha]_D^{22}$ -27.6° (c = 0.45, chloroform-methanol = 1:1),
$^1$H-NMR ($CD_3OD$) δ: 4.44 (1H, d, J = 9.8 Hz), 3.68 (3H, s), 3.55 (1H, dt, J = 9.2 Hz, 3.3 Hz), 3.41 (1H, t, J = 8.8 Hz), 3.40 (1H, dd, J = 13.0 Hz, 3.0 Hz), 3.26 (1H, dd, J = 9.6 Hz, 8.8 Hz), 3.24 (1H, t, J = 8.9 Hz), 3.06 (1H, dd, J = 13.2 Hz, 8.9 Hz), 2.78 (2H, m), 2.35 (2H, t, J = 7.4 Hz), 1.66 (4H, m), 1.41 (8H, m)

Example 46 <u>Glc-S-$C_5$-COOMe (5-methoxycarbonylpentyl 1-thio-β-D-glucopyranoside)</u>

(1) 2-(2,3,4,6-Tetra-O-acetyl-1-thio-β-D-glucopyranosyl)thiopseudourea hydrobromide (5.15 g, 10.8 mmol), ethyl 5-bromopentanoate (2.31 g, 1.2 eq), $K_2CO_3$ (1.54 g), and $NaHSO_3$ (1.03 g) were stirred in acetone (100 ml)-water (100 ml) for 19 hr. The reaction solution was distributed in chloroform and water. The organic layer was dried over $MgSO_4$ and filtered. The filtrate was concentrated to dryness. The residue was purified by column chromatography on silica gel (toluene-ethyl acetate = 9 :1) to give 4.32 g (82%) of 5-ethoxycarbonylpentyl 2,3,4,6-tetra-O-acetyl-1-thio-β-D-glucopyranoside as a colorless oil.

$[\alpha]_D$ -30.2° (c = 1.45, chloroform)
$^1$H-NMR($CDCL_3$) δ: 1.26 (2H, t, J = 7.1 Hz), 1.40-1.45 (2H, m), 1.59-1.67 (4H, m), 2.01 (3H, s), 2.03 (3H, s), 2.06 (3H, s), 2.08 (3H, s), 2.29 (2H, t, J = 7.6 Hz), 2.62-2.73 (2H, m), 3.71 (1H, ddd, J = 2.4, 4.9, 10.0 Hz), 4.14 (2H, q, J = 7.1 Hz), 4.15 (1H, dd, J = 2.4, 12.2 Hz), 4.26 (1H, dd, J = 4.9, 12.2 Hz), 4.48 (1H, d, J = 10.3 Hz), 5.03 (1H, dd, J = 9.5, 10.0 Hz), 5.08 (1H, dd, J = 9.5, 10.0 Hz), 5.22 (1H, dd, J = 9.3, 9.5 Hz).

IR (KBr): 1758 $cm^{-1}$. FAB-MS m/z: 507 ($MH^+$)

(2) A methanol solution of sodium methoxide (28%, 0.39 ml) was added under ice cooling to a solution of 5-ethoxycarbonylpentyl 2,3,4,6-tetra-O-acetyl-1-thio-β-D-glucopyranoside (1.04 g, 3.25 mmol), obtained in step (1), in methanol (15 ml). The mixture was stirred for 4.5 hr. Acetic acid (0.115 ml) was added to the reaction solution, followed by concentration to dryness. The residue was purified by column chromatography on silica gel (chloroform-methanol = 9 : 1) to give 1045 mg (99%) of the title compound as a colorless solid.

$[\alpha]_D$ -47.8° (c = 1.03, methanol).
$^1$H-NMR ($D_2O$) δ: 1.35-1.44 (8H, m), 1.62-1.71 (4H, m), 2.43 (2H, t, J = 7.6 Hz), 2.74-2.83 (2H, m), 3.35 (1H, dd, J = 8.8, 9.8 Hz), 3.44-3.54 (3H, m), 3.73 (3H, s), 3.76 (1H, dd, J = 4.2, 12.2 Hz), 3.93 (1H, dd, J = 2.2, 12.2 Hz), 4.55 (1H,d, J = 10.0 Hz).
IR (KBr): 3472, 1712 $cm^{-1}$. FAB-MS m/z: 325 ($MH^+$)

| Elementary analysis: | | |
|---|---|---|
| Calculated for $C_{15}H_{28}O_7S \cdot 1/4H_2O$: | C, 50.47; | H, 8.04 |
| Found : | C, 50.85; | H, 8.52. |

Example 47 Glc-S-$C_5$-COOH (6-(1-thio-β-D-glucopyranosyl)hexanoic acid)

A solution of sodium hydroxide (231 mg, 2 eq) in water (10 ml) and THF (5 ml) were added to a solution of 5-methoxycarbonylpentyl 1-thio-β-D-glucopyranoside (940 mg, 2.89 mmol) in methanol(10 ml). The mixture was stirred at room temperature for 4 hr. The reaction solution was concentrated. The concentrate was adjusted to pH about 2 by addition of 2 N hydrochloric acid and then adsorbed onto a Diaion HP-20 column. Elution was then carried out with water (200 ml) and acetonitrile (150 ml). The fraction eluted with acetonitrile was concentrated to dryness to give 628 mg (70%) of the title compound as a colorless solid.

$[\alpha]_D$ -48.4° (c = 1.01, methanol)
$^1$H-NMR ($D_2O$) δ: 1.42-1.48 (2H, m), 1.61-1.71 (4H, m), 2.41 (2H, t, J = 7.3 Hz), 2.71-2.83 (2H, m), 3.32 (1H, dd, J = 9.3, 9.5 Hz), 3.40-3.51 (2H, m),3.73 (1H, dd, J = 5.6, 12.5 Hz), 3.92 (1H, dd, J = 2.2, 12.5 Hz), 4.53 (1H, d, J = 9.8 Hz)
IR (KBr): 3456, 1724 $cm^{-1}$. FAB-MS m/z: 311 ($MH^+$)

| Elementary analysis: | | |
|---|---|---|
| Calculated for $C_{13}H_{24}O_7S$: | C, 48.13; | H, 7.46 |
| Found: | C, 47.78; | H, 7.61 |

Example 48 Glc-S-$C_5$-AVP

Triethylamine (0.020 ml) was added to a solution of AVP (40 mg, 0.04 mmol) in methanol (2 ml). The mixture was concentrated to dryness. DMF (2 ml), 6-(1-thio-β-D-glucopyranosyl)hexanoic acid (40 mg, 0.14 mmol) obtained in Example 47, DCC (33 mg, 0.16 mmol), and HOBt (22 mg, 0.16 mmol) were added to the residue. The mixture was stirred at room temperature for 24 hr. The reaction solution was adjusted to pH 2.0 by addition of 10% TFA and then purified by HPLC to give 13 mg of the title compound comprising a glucose derivative introduced into AVP at its N end.

$[\alpha]_D$ -83.2° (c = 0.19, $H_2O$)
$^1$H-NMR ($D_2O$) δ: 1.24-1.40 (2H, m), 1.54-1.72 (5H, m), 1.79-1.85 (1H, m), 1.88-2.01 (2H, m), 2.04-2.35 (6H, m), 2.66-2.84 (3H, m), 2.85 (1H, d, J = 6.8 Hz), 2.94-3.07 (3H, m), 3.15-3.25 (3H, m), 3.29-3.51 (5H, m), 3.67-3.98 (6H, m), 4.14-4.17 (1H, m), 4.33-4.35 (1H, m), 4.44-4.52 (2H, m), 4.60-4.71 (3H, m), 4.93-4.94 (1H, m), 6.80 (2H, d, J = 8.5 Hz), 7.03 (2H, d, J = 8.5 Hz), 7.27 (2H, d, J = 7.1 Hz), 7.37-7.44 (3H, m).

FAB-MS m/z: 1376 ($MH^+$).

Amino acid composition (%) (hydrolysates in 6 N hydrochloric acid): Gly 0.95, Arg, 0.97, Pro 0.98, Cys 1.57, Asp 1.00, Glu 1.00, Phe 1.02, Tyr 0.86, ammonia 3.05 (recovery of Asp 75%)

Example 49 $^3$H-Glc-S-$C_5$-AVP

A methanol solution of AVP (70 μl; concentration 0.56 mg/ml) and a methanol solution of triethylamine (50 μl; concentration 70 μl/ml) were added to a 0.05 M acetic acid-ethanol (3 : 7) solution (0.25 ml) of $^3$H-AVP (4.25 μg, 250 μCi). The mixture was concentrated to dryness. A DMF solution of 6-(1-thio-β-D-glucopyranosyl)hexanoic acid (8 μl; concentration 5.0 mg/0.4 ml) obtained in Example 47, a DMF solution of WSCI (5.8 μl; concentration 5.6 mg/0.5 ml), and a DMF solution of HOBt (6.2 μl; concentration 7.4 mg/1 ml) were added to the residue. The mixture was stirred at room temperature for 19 hr. The reaction solution was added to 50 μl of 0.05% TFA, and the mixture was purified by HPLC to give a tritium-labeled product in an amount corresponding to 2.34 μg. The specific radioactivity was 6.16 Ci/mmol, and the amount of the product synthesised was 105.0 μCi.

Example 50 Glc-S-$C_8$-DEX

DCC (825 mg, 4.0 mmol) was added under ice cooling to a DMF solution (10 ml) of dexamethasone (392 mg, 1.0 mmol), Glc-S-$C_8$-COOH (352 mg, 2.0 mmol) obtained in Example 18, and DMAP (73 mg, 0.6 mmol). The mixture was stirred at room temperature for 24 hr. The insolubles were removed by filtration. The organic solvent was removed by distillation under the reduced pressure. The residue was fractionated by column chromatography on silica gel (ethyl acetate : methanol = 12 : 1) to give 177 mg (0.24 mmol, yield 24%) of the title compound, dexamethasone esterified at the 21-position.

FAB-MS m/z: 727 ($MH^+$)

$^1$H-NMR (DMSO-$d_6$) δ: 7.30 (1H, d, J = 10 Hz), 6 .22 (1H, d, J = 10 Hz), 6.01 (1H, s), 5.40 (1H, d, J = 4.5 Hz), 5.15 (1H, s), 5.05 (1H, d, J = 5.8 Hz), 5.01 (1H, d, J = 18 Hz), 5.00 (1H, d, J = 4.7 Hz), 4.92 (1H, d, J = 4.9 Hz), 4.79 (1H, d, J = 18 Hz), 4.46 (1H, t, J = 5.6 Hz), 4.21 (1H, d, J = 9.5 Hz), 4.16 (1H, m), 3.64 (1H, m), 3.43 (1H, m), 3.2-3.0 (3H, m), 2.98 (1H, m), 2.87 (1H, m), 2.6(3H, m), 2.4-2.1 (6H, m), 1.77 (1H, m), 1.7-1.45 (5H, m), 1.49 (3H, s), 1.4-1. 2 (10H, m), 1.07 (1H, m), 0.88 (3H, s), 0.79 (3H, d, J = 7.0 Hz)

The above procedure was repeated, except that a tritium-labeled dexamethasone was used. Thus, $^3$H-labeled Glc-S-$C_8$-DEX was obtained.

Example 51 Glc-S-$C_8$-DEX

The procedure of Example 50 was repeated, except that Glc-S-$C_5$-COOH obtained in Example 47 was used instead of Glc-S-$C_8$-COOH described in Example 50. Thus, 112 mg (0.16 mmol, yield 33%) of the title compound was obtained from dexamethasone (196 mg, 0.5 mmol).

FAB-MS m/z:685 ($MH^+$)

$^1$H-NMR (DMSO-$d_6$) δ: 7.30 (1H, d, J = 10 Hz), 6 .22 (1H, d, J = 10 Hz), 6.01 (1H, s), 5.40 (1H, d, J = 4.5 Hz), 5.15 (1H, s), 5.05 (1H, d, J = 5.8 Hz), 5.01 (1H, d, J = 17 Hz), 5.00 (1H, d, J = 4.7 Hz), 4.92 (1H, d, J = 4.9 Hz), 4.80 (1H, d, J = 17 Hz), 4.47 (1H, t, J = 5.6 Hz), 4.21 (1H, d, J = 9.5 Hz), 4.16 (1H, m), 3.64 (1H, m), 3.43 (1H, m), 3.2-3.0 (3H, m), 2.98 (1H, m), 2.87 (1H, m), 2.6 (3H, m), 2.4-2.1 (6H, m), 1.77 (1H, m), 1.7-1.45 (5H, m), 1.49 (3H, s), 1.4-1.3 (4H, m), 1.07 (1H, m), 0.88 (3H, s), 0.79 (3H, d, J = 7.0 Hz)

Example 52 Glc-S-$C_8$-Gly-DEX

DCC (62 mg, 0.3 mmol) was added under ice cooling to a DMF solution (10 ml) of dexamethasone (98 mg, 0.25 mmol), BocGly (53 mg, 0.3 mmol), and DMAP (11 mg, 0.09 mmol). The mixture was stirred at room temperature for 24 hr. The insolubles were removed by filtration. The organic solvent was removed by distillation under the reduced pressure. The residue was fractionated by column chromatography on silica gel (chloroform : ethyl acetate = 5 : 3) to give BocGly-DEX. TFA (5 ml) was added to BocGly-DEX. The mixture was stirred for 15 min. The organic solvent was removed by distillation under the reduced pressure to give Gly-DEX. DCC (62 mg, 0.3 mmol) was added under ice cooling to Gly-DEX and a DMF solution (6 ml) of Glc-S-$C_8$-COOH (106 mg, 0.3 mmol) obtained in Example 18 and HOBt (47 mg, 0.3 mmol). The mixture was stirred at room temperature for 24 hr. Thereafter, the insolubles were removed by filtration. The organic solvent was removed by distillation under the reduced pressure. The residue was fractionated by

column chromatography on silica gel (chloroform : methanol = 5 : 1) to give 90 mg (0.11 mmol, yield from dexamethasone 47%) of the title compound.

FAB-MS m/z:784 ($MH^+$)
$^1$H-NMR (DMSO-$d_6$) δ: 8.28 (1H, t, J = 5.8 Hz), 7.30 (1H, d, J = 10 Hz), 6.22 (1H, d, J = 10 Hz), 6.01 (1H, s), 5.40 (1H, d, J = 4.3 Hz), 5.19 (1H, s), 5.10 (1H, d, J = 17 Hz), 5.05 (1H, d, J = 5.8 Hz), 5.00 (1H, d, J = 4.6 Hz), 4.91 (1H, d, J = 4.9 Hz), 4.82 (1H, d, J = 17 Hz), 4.46 (1H, t, J = 5.6 Hz), 4.22 (1H, d, J = 9.5 Hz), 4.17 (1H, m), 3.98 (1H, dd, J = 18, 5.8 Hz), 3.90 (1H, dd, J = 18, 5.8 Hz), 3.64 (1H, m), 3.43 (1H, m), 3.2-3.0 (1H, m), 2.98 (1H, m), 2.89 (1H, m), 2.6 (3H, m), 2.4-2.1 (6H, m), 1.78 (1H, m), 1.7-1.45 (5H, m), 1.49 (3H, s), 1.4-1.2 (10H, m), 1.07 (1H, m), 0.88 (3H, s), 0.79 (3H, d, J = 7.0 Hz)

Example 53 Glc-S-$C_8$-Ala-DEX

The procedure of Example 52 was repeated, except that BocAla was used instead of BocGly in Example 52. Thus, 65 mg (0.082 mmol, yield 38%) of the title compound was obtained from dexamethasone (84 mg, 0.21 mmol).

FAB-MS m/z: 798 ($MH^+$)
$^1$H-NMR (DMSO-$d_6$) δ: 8.23 (1H, d, J = 7.3 Hz), 7.30 (1H, d, J = 10 Hz), 6.23 (1H, d, J = 10 Hz), 6.01 (1H, s), 5.41 (1H, d, J = 4.3 Hz), 5.16 (1H, s), 5.06 (1H, d, J = 5.8 Hz), 5.00 (1H, d, J = 4.9 Hz), 5.00 (1H, d, J = 17 Hz), 4.91 (1H, d, J = 4.9 Hz), 4.86 (1H, d, J = 17 Hz), 4.46 (1H, t, J = 5.8 Hz), 4.35 (1H, m), 4.22 (1H, d, J = 9.8 Hz), 4.15 (1H, m), 3.65 (1H, m), 3.41 (1H, m), 3.2-3.0 (3H, m), 2.96 (1H, m), 2.88 (1H, m), 2.6 (3H, m), 2.4-2.1 (6H, m), 1.77 (1H, m), 1.7-1.45 (5H, m), 1.49 (3H, s), 1.4-1.2 (10H, m), 1.36 (3H, d, J = 7.3 Hz), 1.07 (1H, m), 0.88 (3H, s), 0.79 (3H, d, J = 7.0 Hz)

Example 54 Glc-S-$C_8$-Leu-DEX

The procedure of Example 52 was repeated, except that BocLeu was used instead of BocGly in Example 52. Thus, 67 mg (0.08 mmol, yield 35%) of the title compound was obtained from dexamethasone (90 mg, 0.23 mmol).

FAB-MS m/z: 840 ($MH^+$)
$^1$H-NMR (DMSO-$d_6$) δ: 8.18 (1H, d, J = 7.9 Hz), 7.30 (1H, d, J = 10 Hz), 6.23 (1H, d, J = 10 Hz), 6.01 (1H, s), 5.41 (1H, d, J = 4.6 Hz), 5.15 (1H, s), 5.06 (1H, d, J = 5.8 Hz), 5.00 (1H, d, J = 4.6 Hz), 5.00 (1H, d, J = 17 Hz), 4.91 (1H, d, J = 4.9 Hz), 4.86 (1H, d, J = 17 Hz), 4.46 (1H, t, J = 5 .5 Hz), 4.38 (1H, m), 4.22 (1H, d, J = 9.5 Hz), 4.15 (1H, m), 3.65 (1H, m), 3.42 (1H, m), 3.2-3.0 (3H, m), 2.98 (1H, m), 2.87 (1H, m), 2.6 (3H, m), 2.4-2.1 (6H, m), 1.77 (1H, m), 1.75 -1.45 (8H, m), 1.49 (3H, s), 1.4-1.2 (10H, m), 1.07 (1H, m), 0.92 (3H, d, J = 6.4 Hz), 0.87 (3H, s), 0.86 (3H, d, J = 6.4 Hz), 0.79 (3H, d, J = 7.0 Hz)

Example 55 Glc-S-$C_8$-Ile-DEX

The procedure of Example 52 was repeated, except that BocIle was used instead of BocGly in Example 52. Thus, 65 mg (0.077 mmol, yield 30%) of the title compound was obtained from dexamethasone (101 mg, 0.26 mmol).

FAB-MS m/z: 840 ($MH^+$)
$^1$H-NMR (DMSO-$d_6$) δ: 8.06 (1H, d, J = 7.5 Hz), 7.30 (1H, d, J = 10 Hz), 6.23 (1H, d, J = 10 Hz), 6.01 (1H, s), 5.41 (1H, d, J = 4.6 Hz), 5.16 (1H, s), 5.06 (1H, d, J = 5.8 Hz), 5.00 (1H, d, J = 4.7 Hz), 5.00 (1H, d, J = 17 Hz), 4.91 (1H, d, J = 4.9 Hz), 4.86 (1H, d, J = 17 Hz), 4.46 (1H, t, J = 5.6 Hz), 4.38 (1H, m), 4.22 (1H, d, J = 9.5 Hz), 4.15 (1H, m), 3.65 (1H, m), 3.42 (1H, m), 3.2-3.0 (3H, m), 2.98 (1H, m), 2.87 (1H, m), 2.6 (3H, m), 2.4-2.1 (6H, m), 1.85 (1H, m), 1.77 (1H, m), 1.7-1.45 (7H, m), 1.49 (3H, s), 1.4-1.2 (10H, m), 1.07 (1H, m), 0.94 (3H, d, J = 6.0 Hz), 0.88 (3H, s), 0.86 (3H, t, J = 6.0 Hz), 0.79 (3H, d, J = 7.0 Hz)

Example 56 Glc-S-$C_8$-Gly-DEX

The procedure of Example 52 was repeated, except that Glc-S-$c_5$-COOH obtained in Example 47 was used instead of Glc-S-$C_8$-COOH in Example 52. Thus, 50 mg (0.067 mmol, yield 30%) of the title compound was obtained from dexamethasone (88 mg, 0.22 mmol).

FAB-MS m/z: 742($MH^+$)
$^1$H-NMR (DMSO-$d_6$) δ: 8.30 (1H, t, J = 5.8 Hz), 7.30 (1H, d, J = 10 Hz), 6.22 (1H, d, J = 10 Hz), 6.01(1H, s), 5.40

(1H, d, J = 4.3 Hz), 5.19 (1H, s), 5.10 (1H, d, J = 17 Hz), 5.08 (1H, d, J = 4.5 Hz), 5.02 (1H, d, J = 3 Hz), 4.93 (1H, d, J = 4.3 Hz), 4.82 (1H, d, J = 17 Hz), 4.48 (1H, t, J = 5.5 Hz), 4.23 (1H, d, J = 9.8 Hz), 4.15 (1H, m), 3.98 (1H, dd, J = 18, 5.8 Hz), 3.89 (1H, dd, J = 18, 5.8 Hz), 3.66 (1H, m), 3.41 (1H, m), 3.2-3.0 (3H, m), 2.98 (1H, m), 2.88 (1H, m), 2.6 (3H, m), 2.4-2.1 (6H, m), 1.78 (1H, m), 1.7-1.45 (5H, m), 1.49 (3H, s), 1.4-1.3 (4H, m), 1.07 (1H, m), 0.88 (3H, s), 0.79 (3H, d, J = 7 Hz)

Example 57 Glc-S-$C_5$-Ala-DEX

The procedure of Example 56 was repeated, except that BocAla was used instead of BocGly in Example 56. Thus, 74 mg (0.098 mmol, yield 45%) of the title compound was obtained from dexamethasone (86 mg, 0.22 mmol).

FAB-MS m/z:756 ($MH^+$)
$^1$H-NMR (DMSO-$d_6$) $\delta$: 8.23 (1H, d, J = 7.3 Hz), 7.30 (1H, d, J = 10 Hz), 6.23 (1H, d, J = 10 Hz), 6.0 1 (1H, s), 5.40 (1H, d, J = 4.3 Hz), 5.16 (1H, s), 5.06 (1H, d, J = 5.8 Hz), 5.00 (1H, d, J = 4.9 Hz), 5.00 (1H, d, J = 17 Hz), 4.91 (1H, d, J = 4.9 Hz), 4.86 (1H, d, J = 17 Hz), 4.46 (1H, t, J = 5 .8 Hz), 4.35 (1H, m), 4.22 (1H, d, J = 9.8 Hz), 4.15 (1H, m), 3.65 (1H, m), 3.41 (1H, m), 3.2-3.0 (3H, m), 2.96 (1H, m), 2.88(1H, m), 2.6 (3H, m), 2.4-2.1 (6H, m), 1.77(1H, m), 1.7- 1.45 (5H, m), 1.49 (3H, s), 1.4-1.3 (4H, m), 1.36(3H, d, J = 7.3 Hz), 1.07 (1H, m), 0.88 (3 H, s), 0.79(3H, d, J = 7.0 Hz)

Example 58 Glc-S-$C_5$-Leu-DEX

The procedure of Example 56 was repeated, except that BocLeu was used instead of BocGly in Example 56. Thus, 95 mg (0.12 mmol, yield 43%) of the title compound was obtained from dexamethasone (110 mg, 0.28 mmol).

FAB-MS m/z: 798($MH^+$)
$^1$H-NMR (DMSO-$d_6$) $\delta$: 8.20 (1H, d, J = 7.5 Hz), 7.30 (1H, d, J = 10 Hz), 6.23(1H, d, J = 10 Hz), 6.01 (1H, s), 5.41 (1H, d, J = 4.5 Hz), 5.16 (1H, s), 5.06 (1H, d, J = 5.8 Hz), 5.00 (1H, d, J = 4.6 Hz), 5.00 (1H, d, J = 17 Hz), 4.91 (1H, d, J = 4.9 Hz), 4.86 (1H, d, J = 17 Hz), 4.46 (1H, t, J = 5.5 Hz), 4.39 (1H, m), 4.22 (1H, d, J = 9.5 Hz), 4.15 (1H, m), 3.65 (1H, m), 3.42 (1H, m), 3.2-3.0 (3H , m), 2.99 (1H, m), 2.88 (1H, m), 2.6 (3H, m), 2.4-2.1 (6H, m), 1.77 (1H, m), 1.75-1.45 (8H, m), 1.49 (3H, s), 1.4-1.3 (4H, m), 1. 07 (1H, m), 0.92 (3H, d, J = 6.4 Hz), 0.88 (3H, s), 0.86 (3H, d, J = 6.4 Hz), 0.79 (3H, d, J = 7.0 Hz)

Example 59 Glc-S-$C_5$-Ile-DEX

The procedure of Example 56 was repeated, except that BocIle was used instead of BocGly in Example 56. Thus, 66 mg (0.083 mmol, yield 32%) of the title compound was obtained from dexamethasone (102 mg, 0.26 mmol).

FAB-MS m/z: 798 ($MH^+$)
$^1$H-NMR (DMSO-$d_6$) $\delta$: 8.06 (1H, d, J = 7.5 Hz), 7.30 (1H, d, J = 10 Hz), 6.23 (1H, d, J = 10 Hz), 6.01 (1H, s), 5.41 (1H, d, J = 4.5 Hz), 5.16 (1H, s), 5.06 (1H, d, J = 5.8 Hz), 5.00 (1H, d, J = 4. 7 Hz), 5.00 (1H, d, J = 17 Hz), 4.91 (1H, d, J = 4.9 Hz), 4.86 (1H, d, J = 17 Hz), 4.46 (1H, t, J = 5.6 Hz), 4.38 (1H, m), 4.22 (1H, d, J = 9.5 Hz), 4.15 (1H, m), 3.65 (1H, m), 3.42 (1H, m), 3.2-3.0 (3H, m), 2.98 (1H, m), 2.87 (1H, m), 2.6 (3H, m), 2.4-2.1 (6H, m), 1.85 (1H, m), 1.77 (1H, m), 1.7-1.45 (7H, m), 1.49 (3H, s), 1.4-1.3 (4H, m), 1.07 (1H, m), 0.94 (3H, d, J = 6.6 Hz), 0.88 (3H, s), 0.87 (3H, t, J = 6.5 Hz), 0.79 (3H, d, J = 7.0 Hz)

The above procedure was repeated, except that a tritium-labeled dexamethasone was used. Thus, $^3$H-labeled Glc-S-$C_5$-Ile-DEX was obtained.

Example 60 4dGlc-S-$C_8$ (n-octyl 4-deoxy-1-thio-β-D-glucopyranoside)

(1) At the outset, 3.82 g (6.96 mmol) of 2-(trimethylsilyl)ethyl 2,3-di-O-benzyl-4,6-O-benzylidene-β-D-glucopyranoside and a TNF solution (100 ml) of 10 g of powdery 3A molecular sieve were stirred at room temperature for 30 min. Thereafter, 4.37 g (69.6 mmol) of sodium cyanoboron hydride was added thereto. The mixture was further stirred at room temperature for one hr. A ether solution saturated with hydrogen chloride was gradually added until foaming ceased. After the completion of the reaction was confirmed, the reaction solution was neutralized with triethylamine, and the solids were removed by filtration. The filtrate was concentrated to under the reduced pressure. Chloroform was added to the concentrate. The mixture was washed with water, dried over $Na_2SO_4$, and concentrated under the reduced pressure. The concentrate was purified by column chromatography on silica gel (500 cc, ethyl acetate-hexane = 1 : 3)

to give 3.48 g (yield 91%) of 2-(trimethylsilyl)ethyl 2,3,6-tri-O-benzyl-β-D-glucopyranoside.

(2) 2-(Trimethylsilyl)ethyl 2,3,6-tri-O-benzyl-β-D-glucopyranoside (1.00 g, 1.82 mmol) obtained in step (1) was dissolved in a mixed solvent composed of 9 ml of dichloromethane and 9 ml of pyridine. Phenyl chlorothionoformate (1.00 ml, 7.28 mmol) was added thereto. The mixture was stirred at room temperature overnight. Methanol (5 ml) was added thereto, followed by concentration under the reduced pressure. The concentrate was extracted with chloroform. The extract was washed with 2 N hydrochloric acid and water, dried over $Na_2SO_4$, and concentrated under the reduced pressure. The concentrate was purified by column chromatography on silica gel (250 cc, ethyl acetate-hexane = 1 : 10) to give 2-(trimethylsilyl)ethyl 2,3,6-tri-O-benzyl-4-O-phenoxythiocarbonyl-β-D-glucopyranoside.

(3) Tributyltin hydride (4.0 ml, 15.0 mmol) and 123 mg (0.75 mmol) of α,α'-azobisisobutyronitrile (AIBN) were added to a solution of 1.03 g (1.50 mmol) of 2-(trimethylsilyl)ethyl 2,3,6-tri-O-benzyl-4-O-phenoxythiocarbonyl-β-D-glucopyranoside, obtained in step (2), in toluene (50 ml). The mixture was stirred 100°C for 2 hr. After the completion of the reaction, the reaction solution was concentrated under the reduced pressure. The concentrate was purified by column chromatography on silica gel (200 cc, ethyl acetate-hexane = 1 : 10) to give 0.54 g (yield 67%) of 2-(trimethylsilyl)ethyl 2,3,6-trio-O-acetyl-4-deoxy-β-D-glucopyranoside.

(4) Acetic acid (6 ml) and 0.5 g of 10% palladium-carbon were added to a solution of 0.54 g (1.01 mmol) of 2-(trimethylsilyl)ethyl 2,3,6-tri-O-benzyl-4-deoxy-β-D-glucopyranoside obtained in step (3) in ethanol (36 ml), followed by catalytic hydrogenation at 50°C. After the completion of the reaction, the catalyst was removed by filtration. The filtrate was concentrated under the reduced pressure. Pyridine (10 ml) and 5 ml of acetic anhydride were added to the concentrate. The mixture was stirred overnight. Methanol (10 ml) was added thereto. The mixture was then concentrated under the reduced pressure. The concentrate was extracted with chloroform. The extract was washed with 2 N hydrochloric acid and water, dried over $Na_2SO_4$, and concentrated under the reduced pressure. The concentrate was purified by column chromatography on silica gel (250 cc, ethyl acetate-hexane = 1 : 3) to give 2-(trimethylsilyl)ethyl 2,3,6-tri-O-acetyl-4-deoxy-β-D-glucopyranoside.

> $^1$H-NMR ($CDCL_3$) δ ppm: 4.98 (1H, ddd, J = 11.4 Hz, 9.7 Hz, 5.3 Hz), 4.87 (1H, d, J = 9.7 Hz, 7.8 Hz), 4.41 (1H, d, J = 8.0 Hz), 4.19 (1H, dd, J = 11.7 Hz, 5.8 Hz), 4.09 (1H, dd, J = 11.7 Hz, 4.4 Hz), 3.95 (1H, m), 3.74 (1H, m), 3.54 (1H, m), 2.12 (1H, ddd, J = 12.7 Hz, 5.3 Hz, 2.0 Hz), 2.07-2.02 (9H, 3s), 1.59 (1H, dt, J = 12.5 Hz, 11.7 Hz), 0.92 (2H, m)

(5) Acetic anhydride (76 μl, 0.81 mmol) was added to a solution of 21 mg (53.7 μmol) of 2-(trimethylsilyl)ethyl 2,3,6-tri-O-acetyl-4-deoxy-β-D-glucopyranoside obtained in step (4) in toluene (0.5 ml). The mixture was cooled to 0°C. Boron trifluoride etherate (6 μl, 48.3 μmol) was added thereto. The mixture was then stirred at room temperature for 2 hr. After the completion of the reaction, an aqueous sodium hydrogencarbonate solution was added to the reaction solution, followed by separation into an aqueous layer and an organic layer. The aqueous layer was extracted with chloroform. The chloroform layer was combined with the organic layer. The combined layer was then dried over $Na_2SO_4$ and concentrated under the reduced pressure. The concentrate was purified by column chromatography on silica gel (20 cc, ethyl acetate-hexane = 1 : 3) to give 16.5 mg (yield 92%) of 1,2,3,6-tetra-O-acetyl-4-deoxy-D-glucopyranose.

(6) 1,2,3,6-Tetra-O-acetyl-4-deoxy-D-glucopyranose (130 mg, 0.39 mmol) obtained in step (5) was dissolved in 2 ml of methylene chloride. n-Octylmercaptane (0.59 mmol) was added to the solution. The mixture was cooled to 0°C. Boron trifluoride etherate (72 μl, 0.59 mmol) was added thereto. The mixture was stirred at 0°C for 4 hr and then at room temperature for 2 hr. After the completion of the reaction, the reaction solution was extracted with chloroform. The organic layer was washed with an aqueous sodium hydrogencarbonate solution, dried over $Na_2SO_4$, and concentrated under the reduced pressure. The concentrate was purified by column chromatography on silica gel (50 cc, ethyl acetate-hexane = 1 : 5) to give 40.0 mg (yield 24%) of n-octyl 2,3,6-tri-O-acetyl-4-deoxy-1-thio-β-D-glucopyranoside.

> $^1$H-NMR ($CDCL_3$) δppm: 5.02 (1H, m), 4.93 (1H, t, J = 9.6 Hz), 4.41 (1H, d, J = 9.7 Hz), 4.17 (1H, dd, J = 11.8 Hz, 6.0 Hz), 4.10 (1H, dd, J = 11.7 Hz, 4.4 Hz), 3.77 (1H,m), 2.65 (2H, m), 2.17 (1H, ddd, J = 12.6 Hz, 5.1 Hz, 1.9 Hz), 2.08-2.03 (9H, 3S ), 1.62 (1H, dt, J = 12.5 Hz, 11.7 Hz), 1.59 (2H, m), 1.27 (10H, m), 0.88 (3H, t)

(7) n-Octyl 2,3,6-tri-O-acetyl-4-deoxy-1-thio-β-D-glucopyranoside (40 mg, 95.5 μmol) obtained in step (6) was deacetylated with sodium methoxide in methanol. The reaction solution was subjected to gel filtration column chromatography (LH-20; 100 cc). Elution was carried out with methanol to give 18.7 mg (yield 67%) of n -octyl 4-deoxy-1-thio-β-D-glucopyranoside.

> LC-MS m/z: 315 $(M+Na)^+$
> $^1$H-NMR ($CD_3OD$) δ: ppm : 4.27 (1H ,d, J = 9.5 Hz), 3.58-3.49 (4H, m), 3.08 (1H, t, J = 9.1 Hz), 2.68 (2H, m), 1.94 (1H, ddd, J = 12.7 Hz, 5.1 Hz, 1.8 Hz), 1.61 (3H, m), 1.28 (10H, m) , 0.88 (3H, t).

Compounds represented by the following abbreviations were synthesised as described in the above examples, and used in tests conducted below.

Glc-O-$C_8$: Octyl β-D-glucopyranoside
Glc-O-$C_6$: Hexyl β-D-glucopyranoside
Glc-O- $CH_3$: Methyl β-D-glucopyranoside
Glc-S-$C_8$: Octyl 1-thio-β-D-glucopyranoside
Gal-S-$C_8$: Octyl 1-thio-β-D-galactopyranoside
Man-S-$C_8$: Octyl 1-thio-β-D-mannopyranoside
2dGlc-S-$C_8$: Octyl 2-deoxy-1-thio-β-D-glucopyranoside
All-S-$C_8$: Octyl 1-thio-β-D-allopyranoside
Xyl-S-$C_8$: Octyl 1-thio-β-D-xylopyranoside
Glc-S-pNP: p-Nitrophenyl 1-thio-β-D-glucopyranoside
Glc-S-$C(CH_3)_2$-$CH_2$-$C(CH_3)_3$: t-Octyl 1-thio-β-D-glucopyranoside
Glc-S-$CH(C_4H_9)_2$: 5-(1-Thio-β-D-glucopyranosyl)nonan

Test 1

Distribution of sugar-modified AVP in organs

Under ether anaesthesia, rats (male SD rats, 6 to 8 weeks old) were intravenously injected with a specimen (1 nmol/kg), and the concentration of the specimen in plasma up to five min after the administration and the concentration of the specimen in organ 5 min after the administration were measured. Organ samples were processed in an automatic sample combustion equipment, and the total radioactivity were measured with a liquid scintillation counter to determine the drug level. The concentration of drug in organs and the organ clearance were used as organ selectivity index. The organ clearance was determined by the following equation.

Organ clearance = (concentration in organ in 5 min after administration)/(area under plasma concentration-time curve up to 5 min after the administration)

The organ clearance under the above experimental conditions is considered as a measure of the speed at which the compound is incorporated into the organs. The kidney is an organ which functions to perform glomerular filtration for the production of urine. The renal clearance of low-molecular weight compounds, which are likely to undergo glomerular filtration, is generally larger than the clearance for other organs. Further, under the above experimental conditions, it was estimated from the results on $^{14}C$-labeled inulin that the incorporation of about 0.6 ml/min/g was carried out by glomerular filtration. Larger values indicate the incorporation of drugs into the kidney through blood vessels in addition to the glomerular filtration.

The distribution of the sugar-modified AVPs in organs obtained by this test were as shown in Figs. 1 and 2.

For the organs other than the kidney, the transportation of all the sugar-modified AVPs was lower than that in AVP. There was a clear difference in the distribution in the kidney among the sugar-modified AVPs. Specifically, there was high renal selectivity for Glc-O-$C_8$-, Man-O-$C_8$-, and 2dGlc-O-$C_8$-modified AVPs, whereas, for Gal-O-$C_8$- and Man(α)-O-$C_8$-modified AVPs, the renal selectivity was the same as that for AVP.

The above results clearly show that the sugar located at the end is involved in the renal selectivity. Further, the results suggest that the structural requirement of the sugar for recognition in the kidney is such that the C4 hydroxyl group in the pyranose ring is not axial and the $C_2$ hydroxyl group may orient in any direction or may be absent.

Test 2

Autoradiography of sugar-modified AVPs

Sugar-modified AVPs were administered to rats. Five min after the administration, accumulation sites in the kidney were examined by autoradiography. As a result, as compared with Gal-$C_8$-O-AVP and AVP, the concentration of Glc-O-$C_8$-AVP in cortical part and in the outer layer of the outer band in the medulla were clearly higher while the concentration in the medulla was lower.

Test 3

Binding between sugar-modified AVPs and membrane fractions of organs

A membrane fraction was prepared by the method of Stassen F.L. et al. (J. Pharm. Exp. Thera., 223, 50-54, 1982). An experiment on binding was carried out as follows. The membrane fraction prepared above, together with the $^{3}$H-labeled derivative, was incubated in physiological phosphate buffer on ice, followed by ultracentrifugation. The bound product obtained as the sediment by the ultracentrifugation was counted.

As a result, the sugar-modified AVPs, which exhibited renal selectivity in vivo, were bound to the kidney membrane fraction. Scatchard analysis have revealed that the Glc-O-$C_8$-, Man-O-$C_8$-, 2dGlc-O-$C_8$-, and Glc-S-$C_8$-modified AVPs had specific binding activity. The dissociation constant was 55 nM, 14 nM, 28 nM, and 8.6 nM respectively for the Glc-O-$C_8$-, Man-O-$C_8$-, 2dGlc-O-$C_8$-, and Glc-S-$C_8$-modified AVPs.

Test 4

Inhibitory activity against binding between $^{1}$H-Glc-O-$C_8$-AVP and kidney membrane fraction

The inhibitory activity of segmental structures of Glc-O-$C_8$-AVP and the like against binding $^{3}$H-Glc-O-$C_8$-AVP (20 pmol/ml) to the kidney membrane fraction (1 mg/ml) (incubation on ice for 10 min) was studied. The experiment was carried out in a series of ihibition concentrations to determine 50% inhibition concentration ($IC_{50}$: nmol/ml). The results were as shown in Table 3 below.

For Glc, the inhibitory activity was very low, whereas when the alkyl chain length exceeded a given value, the inhibitory activity became high. This suggests that the recognition in the kidney cannot be achieved by sugar alone, but requires both sugar and lipid.

Table 3

| Inhibitory activity against binding of $^{3}$H-Glc-O-$C_8$-AVP to kidney membrane fraction | |
|---|---|
| Sample | $IC_{50}$, nmol/ml |
| Glc-O-$C_8$-AVP | 0.075 |
| Glc-O-$C_8$ | 2.9 |
| Glc-O-$C_6$ | 5.9 |
| Glc-O-$CH_3$ | >2000 |
| Glc | >2000 |
| AVP | 150 |
| Glc-S-$C_8$ | 0.065 |
| Gal-S-$C_8$-S-$C_8$ | 120 |

Test 5

Studies on oxytocin derivatives

To investigate whether or not the enhancement in distribution to the kidney by sugar-lipid modification is inherent in AVP, oxytocin was used as other model peptide and modified with Glc-O-$C_8$- and Man-O-$C_8$-, followed by determination of organ distribution. The organ selectivity was evaluated in the same manner as described above in connection with the sugar-lipid-modified AVPs.

As a result, as with the modified AVPs, the modified Oxys had renal selectivity. In Table 4 below, the results are expressed by average value (n = 3) ± standard error, and the clearance is in ml/min/g.

Table 4

| Organ clearance of oxytocin derivatives | | |
|---|---|---|
| Sample | Renal clearance | Hepatic clearance |
| Oxy | 0.39±0.06 | 0.05±0.01 |
| Glc-O-$C_8$-Oxy | 1.05±0.05 | 0.08±0.00 |
| Man-O-$C_8$-Oxy | 3.59±0.17 | 0.14±0.02 |

Test 6

Renal selectivity of thioglucoside-modified products

Organ distribution was studied on Glc-S-$C_8$ and, in addition, thioglucoside modification products of AVP, tryptamine, NBD as a fluorescent dye, doxorubicin as an anticancer agent and the like. Glc-S-$C_8$ (the labeled product was prepared by catalytic exchange with $^3$H gas; the specific activity 40.7 GBq/mmol), AVP derivatives, and tryptamine derivatives were converted to $^3$H-labeled product and then studied on organ selectivity in the same manner as in Test 1. For the NBD derivatives, an experiment on distribution was carried out under conditions of dose 10 nmol/kg and 3 min. The vital sample was deproteinized with acetonitrile, subjected to separation by reversed phase liquid chromatography, and then quantitatively determined by fluorescence detection. DXR and derivatives thereof were administered at a dose of 50 nmol/kg, and, 3 min after the administration, the distribution was detected in the same manner as used for the NBD derivatives.

As a result, it was found that the thioglucoside derivative of AVP, Glc-S-$C_8$-AVP, has higher renal selectivity than Glc-O-$C_8$-AVP having renal selectivity. Further, Glc-S-$C_{11}$-AVP had higher renal selectivity than Glc-S-$C_8$-AVP. Furthermore, for Glc-S-$C_8$-Tryptamine, Glc-S-$C_8$-CONH-$C_2$-NBD, Glc-S-$C_8$-CONH-DXR, and Glc-S-$C_8$-COO-DXR, the renal clearance was high, and, in addition, the actual level thereof in kidney was higher than the original compounds. This demonstrates that the renal selectivity can be imparted to these compounds by sugar-lipid modification. In Glc-S-$C_8$-COO-DXR, the structure recognizable in the kidney is bound to the drug through an ester bond. This bond is widely known to under hydrolysis in vivo. In fact, the formation of free DXR in plasma and kidney could be confirmed.

Table 5

| Renal selectivity of thioglucoside-modified products | | |
|---|---|---|
| Sample | Renal clearance ml/min/g | Conc. in kidney % of dose/g |
| Glc-S-$C_8$ | 5.13±0.68 | 10.2±0.8 |
| AVP | 0.49±0.04 | 5.3±0.7 |
| Glc-S-$C_8$-AVP | 3.42±0.05 | 29.2±0.9 |
| Glc-S-$C_{11}$-AVP | 4.47±0.33 | 40.2±1.5 |
| Tryptamine | 0.63±0.07 | 3.5±0.3 |
| Glc-S-$C_8$-Tryptamine | 5.72±0.38 | 16.3±1.5 |
| HO-$C_2$-NBD | 0.28±0.03 | 0.8±0.1 |
| Glc-S-$C_8$-CONH-$C_2$-NBD | 3.58±0.44 | 17.7±1.3 |
| DXR | 0.54±0.10 | 1.9±0.2 |
| Glc-S-$C_8$-CONH-DXR | 1.31±0.04 | 5.8±0.6 |
| Glc-S-$C_8$-COO-DXR | 2.73±0.24 | 6.1±0.3 |

For O and S as atoms attached to the glucoside, the above test results lead to the following conclusion. At the outset, according to the experiment on organ distribution using rats, as shown in Table 5 and Fig. 2, the renal clearance

was 3.42 ml/min/g and 2.43 ml/min/g respectively for Glc-S-$C_8$-AVP and Glc-O-$C_8$-AVP. Further, as is apparent from Test 3, for bindability to the kidney membrane fraction, the dissociation constant was 8.6 nM and 55 nM respectively for the Glc-S-$C_8$-thioglucoside derivative and the Glc-O-$C_8$-thioglucoside derivative, indicating that the binding activity of the Glc-S-$C_8$- derivative was about 6 times higher than that of the Glc-O-$C_8$- derivative. Therefore, it is considered that Glc-S-$C_8$- is more likely to be recognised in the kidney than Glc-O-$C_8$-. Further, in Test 4, Glc-S-$C_8$ had higher inhibitory activity than Glc-O-$C_8$. Therefore, S is more preferably as the atom attached to the glucoside.

Test 7

Renal selectivity of modified anti-inflammatory steroid (part 1)

Dexamethasone as a steroidal anti-inflammatory drug was used to examine the usefulness of the compounds of the present invention. SD male rats (seven weeks old) (two groups, groups A and B each consisting of three rats) were provided. The rats of group A were injected with $^3$H-labeled Glc-S-$C_5$-Ile-DEX, while the rats of group B were injected with $^3$H-labeled dexamethasone. In this case, the $^3$H-labeled Glc-S-$C_5$-Ile-DEX and $^3$H-labeled dexamethasone were each dissolved in physiological saline containing a very small amount of ethanol, followed by intravenous injection through caudal vein under non-anesthesia. For both the groups, the dose was 10 nmol/kg.

Five min after the administration, laparotomy was carried out under ether anesthesia, blood was collected from the abdominal artery, and each organ was then extracted. According to the conventional method, the total radioactivity in the organs was measured, and the total concentration of dexamethasone in each organ was determined.

The results were as shown in Table 6 below. As is apparent from the table, the concentration of dexamethasone in the kidney for the rats of group A to which Glc-S-$C_5$-Ile-DEX has been administered was at least 6 times higher than the concentration of dexamethasone in the kidney for the rats of group B to which dexamethasone had been administered. In the other organs, the concentration of dexamethasone was lower for the rats of group A.

Separately, for rats to which the $^3$H-labeled Glc-S-$C_5$-Ile-DEX had been administered, Glc-S-$C_5$-Ile-DEX and free dexamethasone in the kidney were isolated and analyzed. As a result, the production of free dexamethasone in the kidney could be confirmed.

Table 6

| Total conc. of dexamethasone in organ, pmol/g tissue | | |
|---|---|---|
| | Group A | Group B |
| Blood | 5.36±0.83 | 7.61±0.49 |
| Kidney | 143.03±50.59 | 22.36±0.80 |
| Heart | 2.01±0.12 | 14.86±1.05 |
| Lung | 4.09±0.95 | 16.49±1.57 |
| Liver | 50.62±27.37 | 65.90±12.61 |
| Spleen | 2.23±0.60 | 11.04±0.25 |
| Adrenal | 16.17±2.45 | 24.65±0.95 |
| Brain | 0.25±0.08 | 0.94±0.25 |
| Fat | 0.44±0.10 | 2.74±1.12 |

Test 8

Effect of sugars on inhibition of specific binding between $^3$H-Glc-S-$C_8$ and kidney membrane fraction

In order to estimate structural requirement for recognition in the kidney, an experiment was carried out on the effect of various sugars and sugar derivatives on the inhibition of specific -binding between $^3$H-Glc-S-$C_8$ (20 pmol/ml) and the kidney membrane fraction (1 mg protein/ml). The results were as summarized in Table 7.

For four sugars, the order of the inhibitory effect was 2-deoxyglucose ≧ mannose ≧ glucose > galactose, and the inhibitory effect was in agreement with the renal selectivity of the vasopressin derivatives indicated in Test 1. Further, the results on the inhibitory effect of other sugars suggested that the hydroxyl Group at the 2- and 4-positions of

the pyranose ring may be replaced with fluorine. Furthermore, nojirimycin, a compound wherein the oxygen atom in the pyranose ring has been replaced with a nitrogen atom, exhibited high inhibitory effect. This suggests that nitrogen may be present instead of oxygen.

Table 7

| Effect of sugars on inhibition of specific binding between $^3$H-Glc-S-$C_8$ and kidney membrane fraction | | |
|---|---|---|
| Sample | Inhibition (%) | |
| | 20 mM | 100 mM |
| Glucose ` | 16.7 | 38.4 |
| Galactose | 2.5 | 18.9 |
| Mannose | 18.7 | 51.0 |
| 2-Deoxyglucose | 29.2 | 58.3 |
| 2-Fluoro-2-deoxyglucose | 37.8 | 67.9 |
| 3-Fluoro-3-deoxyglucose | 9.7 | 18.7 |
| 4-Fluoro-4-deoxyglucose | 70.2 | 83.9 |
| 6-Fluoro-6-deoxyglucose | 17.8 | 32.5 |
| 1-Deoxynojirimycin | 98.9 | 99.6 |

Test 9

Effect of glycolipid derivatives on inhibition of specific binding between $^3$H-Glc-S-$C_8$ and kidney membrane fraction

Commercially available or synthesized glycolipid derivatives were subjected to an binding inhibition experiment in the same manner as in Test 4. The experiment was carried out in a series of inhibition concentrations to determine 50% inhibition concentration ($IC_{50}$: nmol/ml).

The results were as shown in Table 8 below.

In judging the results, compounds having higher inhibitory effect (that is, lower $IC_{50}$ value) than Glc-O-$C_8$, which is a structure capable of actually imparting renal selectivity by modifying AVP with the structure, was regarded as those which can be brought to a structure recognizable in the kidney. At the outset, regarding the structure of sugar, the inhibitory effect of lipid derivatives of galactose and 4-deoxyglucose is so low that equatorial hydroxyl group of C4 in the pyranose ring is necessary. Since lipid derivatives of mannose and allose are recognized, hydroxyl group of C2 or C3 may be axial or equatorial. Since lipid derivatives of xylose are recognized, C6 carbon and hydroxyl group attached thereto are not indispensable. For the lipid portion, branched chains as well as straight-chain alkyls are recognizable. Further, in addition to aliphatic chains, such as alkyls, structures containing aromatic rings are also recognizable.

Comparison of compounds having a methoxycarbonyl group at the head of the lipid shows that, regarding the atom attached to the glycoside, S or NH had better recognition than O. O in the pyranose ring may be replaced with S or NH.

Table 8

| Effect of glycolipid derivatives on inhibition of specific binding between $^3$H-Glc-S-$C_3$ and kidney membrane fraction | |
|---|---|
| Sample | $IC_{50}$ nmol/ml |
| Glc-S-$C_8$ | 0.078 |
| Glc-O-$C_8$ | 4.6 |
| Gal-S-$C_8$ | 37 |

Table 8 (continued)

| Effect of glycolipid derivatives on inhibition of specific binding between $^3H$-Glc-S-$C_3$ and kidney membrane fraction | |
|---|---|
| Sample | $IC_{50}$ nmol/ml |
| Man-S-$C_8$ | 0.20 |
| 2d-Glc-S-$C_8$ | 0.071 |
| 4d-Glc-S-$C_8$ | 55 |
| All-S-$C_8$ | 1.1 |
| Xyl-S-$C_8$ | 1.5 |
| Glc-S-pNP* | 0.23 |
| Glc-S-$C(CH_3)_2$-$CH_2$-$C(CH_3)_3$ | 1.0 |
| Glc-S-$CH(C_4H_9)_2$ | 1.5 |
| Glc-O-$C_8$-COOMe | 33 |
| Glc-S-$C_8$-COOMe | 0.59 |
| Glc-NH-$C_7$-COOMe | 0.11 |
| 5S-Glc-S-$C_8$-COOMe | 14 |
| 5N-Glc-S-$C_8$-COOMe | 4.7 |

Test 10

Renal selectivity of modified anti-inflammatory steroid (part 2)

(1) Pharmacokinetic test of rats (Glc-S-$C_5$-Ala-DEX)

Dexamethasone as a steroidal anti-inflammatory drug was used to examine the usefulness of the compounds of the present invention. SD male rats (ten weeks old) (two groups, groups A and B each consisting of three rats) were provided. The rats of group A were injected with Glc-S-$C_5$-Ala-DEX, while the rats of group B were injected with dexamethasone. In this case, the Glc-S-$C_5$-Ala-DEX and dexamethasone were each dissolved in physiological saline containing a very small amount of ethanol, followed by intravenous injection through caudal vein under non-anesthesia. For both the groups, the dose was 100 nmol/kg.

Five min after the administration, laparotomy was carried out under ether anesthesia, blood was collected from the abdominal artery. Plasma was fractionated from the collected blood according to the conventional method. Further, the kidney and the liver were extracted. The plasma was deprotenized, and the organs were homogenized and deprotenized, followed by separation by reversed phase liquid chromatography. Thereafter, Glc-S-$C_5$-Ala-DEX and dexamethasone were detected and quantitatively determined by UV detector.

The results were as shown in Table 9 below. As is apparent from the table below, as compared with the group of rats to which dexamethasone had been administered, the total amount of dexamethasone in the kidney was 4.7 times larger for the group of rats to which Glc-S-$C_5$-Ala-DEX had been administered, indicating the renal selectivity. Further, for the concentration of free dexamethasone which is the main body having pharmacological activity, the group of rats to which Glc-S-$C_5$-Ala-DEX had been administered were 4.1 times higher than the group of rats to which dexamethasone had been administered, suggesting that, after accumulation of Glc-S-$C_5$-Ala-DEX in the kidney, dexamethasone was liberated.

Table 9

| | Plasma | Kidney | Liver |
|---|---|---|---|
| Group A (Glc-S-$C_5$-Ala-DEX administered) | | | |
| Concentration of Glc-S-$C_5$-Ala-DEX<br>Concentration of free dexamethasone<br>(Total) | N.D.<br>139.8±17.5<br>(139.8) | 116.7±49.9<br>827.8±73.0<br>(944.5) | 157.0±19.4<br>762.0±67.7<br>(919.0) |
| Group B (dexamethasone administered) | | | |
| Concentration of dexamethasone | 116.5±13.6 | 202.8±39.3 | 929.8±119.2 |
| (For n = 3, average value ± SD: pmol/g tissue) | | | |

(2) Pharmacokinetic test of monkey (Glc-S-$C_8$-CONH-$C_2$-NBD)

In order to investigate the difference in activity of the compounds according to the present invention between animal species, the pharmacokinetic test using monkeys was carried out as follows. Macaca fascicularis (eight years old) (two groups, groups A and B each consisting of two monkeys) were provided. The monkeys of group A were injected with Glc-S-$C_8$-CONH-$C_2$-NBD, while, for comparison, the monkeys of group B were injected with 4-(2-hydroxyethyl)amino-7-nitrobenz-2-oxa-1,3-diazole (synthesis intermediate prepared in Example 44; hereinafter referred to as "HO-$C_2$-NBD"). In this case, Glc-S-$C_8$-CONH-$C_2$-NBD and 4-(2-hydroxyethyl)amino-7-nitrobenz-2-oxa-1,3-diazole were each dissolved in physiological saline containing a very small amount of ethanol, followed by intravenous injection under anesthesia. For both the groups, the dose was 10 nmol/kg.

Five min after the administration, the monkeys were bled to death; and, immediately after that, each organ was extracted. According to the conventional method, the organs were homogenized and deprotenized. Thereafter, the samples were subjected to separation by reversed phase liquid chromatography. Glc-S-$C_8$-CONH-$C_2$-NBD and HO-$C_2$-NBD were detected and quantitatively determined by fluorescence detector.

The results were as shown in Table 10 below. As is apparent from the table, also in the case of monkeys, the renal selectivity can be clearly imparted according to the present invention. This demonstrates that, also in the case of human beings, the kidney selectivity could be imparted according to the present invention.

Table 10

| | Group A Glc-S-$C_8$-CONH-$C_2$-NBD administered | Group B HO-$C_2$-NBD administered |
|---|---|---|
| Plasma | 16.42 | 7.51 |
| Blood | 10.72 | 7.53 |
| Kidney | 280.32 | 2.33 |
| Liver | 13.85 | N.D. |
| Lung | 6.90 | 17.33 |
| Heart | 3.23 | 10.92 |
| Spleen | 0.55 | 2.35 |
| Adrenal | 1.87 | 2.05 |
| (For n = 2, average value: pmol/mol or g tissue) | | |

## Claims

1. A drug carrier having in its molecule a segment structure represented by formula (I), said drug carrier having function to specifically transport a drug supported thereon to a kidney:

A-U-V- (I)

wherein

A represents glycosyl represented by formula (II)

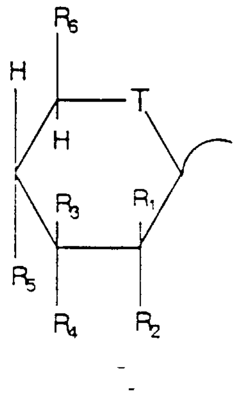

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**2.** The drug carrier according to claim 1, which is represented by formula (III):

A-U-V-Y (III)

wherein

A, U, and V are as defined in claim 1; and
Y represents a segment structure which can support a drug thereon.

**3.** The drug carrier according to claim 2, wherein Y represents a segment structure having a functional group chemically bondable to a group present in the drug.

**4.** The drug carrier according to claim 3, wherein Y represents carboxyl, methoxycarbonyl, hydroxyl, thiol, amino, aldehyde, sulfonic ester, or a halogen atom.

**5.** The drug carrier according to any one of claims 1 to 4, wherein the glycosyl represented by formula (II) represents D-glucosyl, D-mannosyl, or 2-deoxy-D-glucosyl.

**6.** The drug carrier according to any one of claims 1 to 5, wherein V represents a $C_{3-15}$ straight-chain aliphatic hydrocarbon.

**7.** The drug carrier according to any one of claims 1 to 6, wherein U represents S or NH.

**8.** A renal targeting drug having in its molecule a segment structure represented by formula (I):

A-U-V- (I)

wherein

A represents glycosyl represented by formula (II)

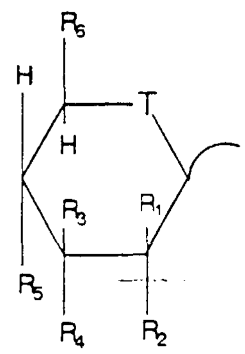

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**9.** The renal targeting drug according to claim 8, which is represented by formula (IV):

A-U-V-X-Z (IV)

wherein

A, U, and V are as defined in claim 1;
X represents a binding group; and
Z represents a drug.

**10.** The renal targeting drug according to claim 9, wherein X is selected from the group consisting of -C(=O)O-, -OC(= O)-, -O(C = O)O-, -C(= O)S-, -SC(= O)-, -C(= O)NH-, -C(= O)N<, -NHC(= O)-, -OC(= O)NH-, -OC(= O)N<, -NHC(= O)O-, -CH = N-, -N = CH-, -O-, -NHC(= O)NH-, -NHC(= O)N<, -NH-, -N<, $-OCH_2O-$, $-OCH(CH_3)O-$, $-SCH_2O-$, $-SCH(CH_3)O-$, $-OCH_2S-$, $-OCH(CH_3)S-$, $-OCH_2NH-$, $-OCH(CH_3)NH-$, $-OCH_2N<$, $-OCH(CH_3)N<$, $-NHCH_2O-$, $-NHCH(CH_3)O-$, $-SCH_2NH-$, $-SCH(CH_3)NH-$, $-SCH(CH_3)N<$, $-C(=O)OCH_2O-$, $-C(=O)OCH(CH_3)O-$, $-C(=O)OCH_2NH-$, $-C(=O)OCH(CH_3)NH-$, $-C(=O)OCH_2N<$, $-C(=O)OCH(CH_3)N<$, $-C(=O)OCH_2S-$, $-C(=O)OCH(CH_3)S-$, $-NHCH_2OC(=O)-$, $-NHCH(CH_3)OC(=O)-$, $-NHC(=O)OCH_2O-$, $-NHC(=O)OCH(CH_3)O-$, $-C(=O)OCH_2OC(=O)NH-$, $-C(=O)OCH(CH_3)OC(=O)NH-$, and -C(= 0)-J-O- wherein J represents an amino acid residue.

**11.** The renal targeting drug according to claim 9, wherein X has a structure represented by $-X_1-X_2-X_3-$ wherein $X_1$ and $X_3$ each independently represent a binding group and $X_2$ represents a spacer structure.

12. The renal targeting drug according to claim 11, wherein $X_1$ and $X_3$ each represent a binding group selected from the group consisting of -C(= O)O-, -OC(= O)-, -O(C = O)O-, -C(=O)S-, -SC(=O)-, -C(=O)NH-, -C(=O)N<, -NHC(= O)-, -OC(= O)NH-, -OC(=O)N<, -NHC(=O)O-, -CH=N-, -N=CH-, -O-, -NHC(= O)NH-, -NHC(=O)N<, -NH-, -N<, -$OCH_2O$-, -$OCH(CH_3)O$-, -$SCH_2O$-, -$SCH(CH_3)O$-, -$OCH_2S$-, -$OCH(CH_3)S$-, -$OCH_2NH$-, -$OCH(CH_3)NH$-, -$OCH_2N$<, -$OCH(CH_3)N$<, -$NHCH_2O$-, -$NHCH(CH_3)O$-, -$SCH_2NH$-, - $SCH(CH_3)NH$-, -$SCH(CH_3)N$<, -C(= O)$OCH_2O$-, -C(= O)$OCH(CH_3)O$-, -C(= O)$OCH_2NH$-, -C(= O)$OCH(CH_3)NH$-, -C(= O)$OCH_2N$<, -C(= O)$OCH(CH_3)N$<, -C(=O)$OCH_2S$-, -C(=O)$OCH(CH_3)S$-, -$NHCH_2OC$(=O)-, -$NHCH(CH_3)OC$(= O)-, -NHC(= O)$OCH_2O$-, -NHC(= O)$OCH(CH_3)O$-, -C(= O)$OCH_2OC$(= O)NH-, -C(= O)$OCH(CH_3)OC$(= O)NH-, and -C(= 0)-J-O- wherein J represents an amino acid residue.

13. The renal targeting drug according to any one of claims 8 to 12, wherein the glycosyl represented by formula (II) represents D-glucosyl, D-mannosyl, or 2-deoxy-D-glucosyl.

14. The renal targeting drug according to any one of claims 8 to 13, wherein V represents a $C_{3-15}$ straight-chain aliphatic hydrocarbon.

15. The renal targeting drug according to any one of claims 8 to 14, wherein U represents S or NH.

16. The renal targeting drug according to claim 9, wherein the glycosyl represented by formula (II) is D-glucosyl, D-mannosyl, or 2-deoxy-D-glucosyl, U represents S, V represents a $C_{3-15}$ straight-chain aliphatic hydrocarbon, X represents -C(O)O- or -CO-J-O- wherein J represents an amino acid residue, and Z represents a steroidal agent bound through its hydroxyl to X.

17. The renal targeting drug according to any one of claims 9 to 16, wherein Z represents a diagnostic drug, an examination drug, a contrast medium, a steroidal agent, an anticancer agent, an immunosuppressive agent, an anticoagulant/antiplatelet, an anti-inflammatory drug, an antihypertensive drug, a diuretic, antidiuretic, a physiologically active peptide, a useful gene, or an anti-sense drug.

18. A chemical modifier for imparting renal selectivity to a drug, comprising the drug carrier according to any one of claims 1 to 7.

19. A compound represented by formula (III-a):

$$A^*\text{-}U\text{-}V\text{-}Y^* \qquad \text{III-a}$$

wherein

A* represents 2-deoxy-D-glucosyl, 5-deoxy-5-thio-D-glucosyl, or 5-amino-5-deoxy-D-glucosyl;
U represents O, S, or NH;
V represents a $C_{6-18}$ aromatic hydrocarbon or a straight-chain or branched $C_{1-18}$ aliphatic hydrocarbon; and
Y* represents carboxyl, methoxycarbonyl, hydroxyl, thiol, amino, aldehyde, sulfonic ester, or a halogen atom.

20. A compound represented by formula (IV-a):

$$A\text{-}U\text{-}V\text{-}X^*\text{-}Z^* \qquad \text{(IV-a)}$$

wherein

A, U, and V are as defined in claim 1;
X* represents -CONH-, -COO-, -OCOO-, or -CO-J-O-wherein J represents an amino acid residue; and
Z* represents a residue of arginine vasopressin, oxytocin, tryptamine, doxorubicin, dexamethasone, 4-(2-hydroxyethyl)amino-7-nitrobenz-2-oxa-1,3-diazole, or 4-(2-aminoethyl)amino-7-nitrobenz-2-oxa-1,3-diazole, provided that compounds, wherein U represents O and Z represents arginine vasopressin or oxytocin, are excluded.

21. The compound according to claim 20, wherein

A represents D-glucosyl, D-mannosyl, 2-deoxy-D-glucosyl, 5-deoxy-5-thio-D-glucosyl, or 5-amino-5-deoxy-D-glucosyl; and
V represents a $C_{6-18}$ aromatic hydrocarbon or a straight-chain or branched $C_{1-18}$ aliphatic hydrocarbon.

**22.** The compound according to any one of claims 19 to 21, wherein V represents a $C_{3-15}$ straight-chain aliphatic hydrocarbon.

**23.** The compound according to any one of clams 19 to 22, wherein U represents S or NH.

**24.** The compound according to claim 20, wherein the glycosyl represented by formula (II) is D-glucosyl, D-mannosyl, or 2-deoxy-D-glucosyl, U represents S, V represents a $C_{3-15}$ straight-chain aliphatic hydrocarbon, X represents -C(O)O- or -CO-J-O- wherein J represents an amino acid residue, and Z represents a steroidal agent bound through its hydroxyl to X.

**25.** A method for specifically transporting a drug to a kidney, comprising the steps of: supporting a drug on a compound which has a segment structure represented by formula (I) and can specifically transport the drug supported thereon to a kidney; and administering the compound to an animal including a human being:

A-U-V- (I)

wherein

A represents glycosyl represented by formula (II)

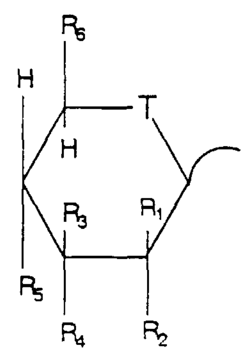

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**26.** The method according to claim 25, wherein the compound is represented by formula (III):

A-U-V-Y (III)

wherein

A, U, and V are as defined in claim 1; and
Y represents a segment structure which can support a drug thereon.

**27.** The method according to claim 26, wherein Y represents a segment structure having a functional group chemically bound to a group present in the drug.

**28.** The method according to claim 27, wherein Y represents carboxyl, methoxycarbonyl, hydroxyl, thiol, amino, aldehyde, sulfonic ester, or a halogen atom.

**29.** The method according to claim 25, wherein the glycosyl represented by formula (II) represents D-glucosyl, D-mannosyl, or 2-deoxy-D-glucosyl.

**30.** The method according to claim 25, wherein V represents a $C_{3-15}$ straight-chain aliphatic hydrocarbon.

**31.** The method according to claim 25, wherein U represents S or NH.

**32.** A method for specifically transporting a drug to a kidney, comprising the step of administering a drug having in its molecule a segment structure represented by formula (I) to an animal including a human being:

$$A\text{-}U\text{-}V\text{-} \qquad (I)$$

wherein

A represents glycosyl represented by formula (II)

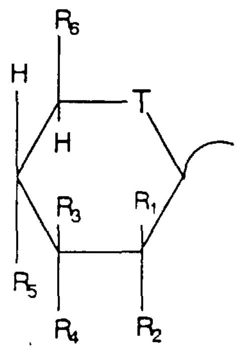

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**33.** The method according to claim 32, wherein the drug is represented by formula (IV):

$$A\text{-}U\text{-}V\text{-}X\text{-}Z \qquad (IV)$$

wherein

A, U, and V are as-defined in claim 1;
X represents a binding group; and
Z represents a drug.

**34.** The method according to claim 33, wherein X is selected from the group consisting of -C(= O)O-, -OC(= O)-, -O(C = O)O-, -C(= O)S-, -SC(= O)-, -C(= O)NH-, -C(= O)N<, -NHC(= O)-, -OC(= O)NH-, -OC(= O)N<, -NHC(= O)O-, -CH = N-, -N = CH-, -O-, -NHC(= O)NH-, -NHC(= O)N<, -NH-, -N<, $-OCH_2O-$, $-OCH(CH_3)O-$, $-SCH_2O-$, $-SCH(CH_3)O-$, $-OCH_2S-$, $-OCH(CH_3)S-$, $- OCH_2NH-$, $-OCH(CH_3)NH-$, $-OCH_2N$<, $-OCH(CH_3)N$<, $-NHCH_2O-$, $-NHCH(CH_3)O-$, $-SCH_2NH-$, $-SCH(CH_3)NH-$, $-SCH(CH_3)N$<, $-C(= O)OCH_2O-$, $-C(= O)OCH(CH_3)O-$, $-C(= O)OCH_2NH-$, $-C(= O)OCH(CH_3)NH-$, $-C(=O)OCH_2N$<, $-C(=O)OCH(CH_3)N$<, $-C(=O)OCH_2S-$, $-C(= O)OCH(CH_3)S-$, $-NHCH_2OC(=O)-$, $-NHCH(CH_3)OC(=O)-$, $-NHC(= O)OCH_2O-$, $-NHC(= O)OCH(CH_3)O-$, $-C(= O)OCH_2OC(= O)NH-$, $-C(= O)OCH(CH_3)OC(= O)NH-$, and -C(= 0)-J-O- wherein J represents an amino acid residue.

**35.** The method according to claim 33, wherein X has a structure represented by $-X_1-X_2-X_3-$ wherein $X_1$ and $X_3$ each independently represent a binding group and $X_2$ represents a spacer structure.

**36.** The method according to claim 35, wherein $X_1$ and $X_3$ each represent a binding group selected from the group consisting of -C(= O)O-, -OC(= O)-, -O(C = O)O-, -C(= O)S-, -SC(= O)-, -C(= O)NH-, -C(= O)N<, -NHC(= O)-, -OC(= O)NH-, -OC(=O)N<, -NHC(=O)O-, -CH=N-, -N=CH-, -O-, -NHC(=O)NH-, -NHC(= O)N<, -NH-, -N<, $-OCH_2O-$, $-OCH(CH_3)O-$, $-SCH_2O-$, $- SCH(CH_3)O-$, $-OCH_2S-$, $-OCH(CH_3)S-$, $-OCH_2NH-$, $-OCH(CH_3)NH-$, $- OCH_2N$<, $-OCH(CH_3)N$<, $-NHCH_2O-$, $-NHCH(CH_3)O-$, $-SCH_2NH-$, $- SCH(CH_3)NH-$, $-SCH(CH_3)N$<, $-C(= O)OCH_2O-$, $-C(= O)OCH(CH_3)O-$, $-C(= O)OCH_2NH-$, $-C(= O)OCH(CH_3)NH-$, $-C(= O)OCH_2N$<, $-C(= O)OCH(CH_3)N$<, $-C(=O)OCH_2S-$, $-C(=O)OCH(CH_3)S-$, $-NHCH_2OC(=O)-$, $-NHCH(CH_3)OC(= O)-$, $-NHC(= O)OCH_2O-$, $-NHC(= O)OCH(CH_3)O-$, $-C(= O)OCH_2OC(= O)NH-$, $-C(= O)OCH(CH_3)OC(= O)NH-$, and -C(= 0)-J-O- wherein J represents an amino acid residue.

**37.** The method according to claim 32, wherein the glycosyl represented by formula (II) represents D-glucosyl, D-mannosyl, or 2-deoxy-D-glucosyl.

**38.** The method according to claim 33, wherein V represents a $C_{3-15}$ straight-chain aliphatic hydrocarbon.

**39.** The method according to claim 33, wherein U represents S or NH.

**40.** The method according to claim 32, wherein Z represents a diagnostic drug, an examination drug, a contrast medium, a steroidal agent, a carcinostatic agent, an immunosuppressive agent, an anticoagulant/antiplatelet, an anti-inflammatory drug, an antihypertensive drug, a diuretic, antidiuretic, a physiologically active peptide, a useful gene, or an anti-sense drug.

**41.** The method according to claim 32, wherein the glycosyl represented by formula (II) is D-glucosyl, D-mannosyl, or 2-deoxy-D-glucosyl, U represents S, V represents a $C_{3-15}$ straight-chain aliphatic hydrocarbon, X represents -C(O)O- or -CO-J-O- wherein J represents an amino acid residue, and Z represents a steroidal agent bound through its hydroxyl to X.

**42.** A method for imparting renal selectivity to a drug, comprising the step of supporting a drug on the drug carrier according to any one of claims 1 to 7.

**43.** Use of a compound having a segment structure represented by formula (I), said compound functioning to specifically transport a drug supported thereon to a kidney, for the production of a drug carrier for specifically transporting the drug to the kidney:

$$A-U-V- \quad (I)$$

wherein

A represents glycosyl represented by formula (II)

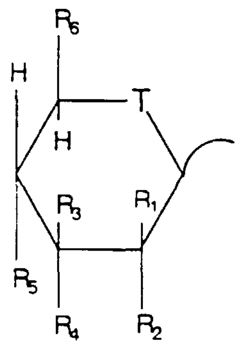

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**44.** Use according to claim 43, wherein the compound is represented by formula (III):

$$A\text{-}U\text{-}V\text{-}Y \qquad (III)$$

wherein

A, U, and V are as defined in claim 1; and
Y represents a segment structure which can support a drug thereon.

**45.** Use according to claim 44, wherein Y represents a segment structure having a functional group chemically bondable to a group present in the drug.

**46.** Use according to claim 45, wherein Y represents carboxyl, methoxycarbonyl, hydroxyl, thiol, amino, aldehyde, sulfonic ester, or a halogen atom.

**47.** Use according to any one of claims 43 to 46, wherein the glycosyl represented by formula (II) represents D-glucosyl, D-mannosyl, or 2-deoxy-D-glucosyl.

**48.** Use according to any one of claims 43 to 47, wherein V represents a $C_{3-15}$ straight-chain aliphatic hydrocarbon.

**49.** Use according to any one of claims 43 to 48, wherein U represents S or NH.

**50.** Use of a comp ound having in its molecule a segment structure represented by formula (I), for the production of a renal targeting drug:

$$A\text{-}U\text{-}V\text{-} \qquad (I)$$

wherein

A represents glycosyl represented by formula (II)

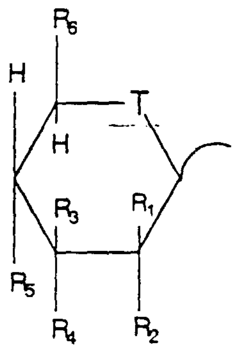

(II)

wherein

T represents O, S, or NH,
one of $R_1$ and $R_2$ represents H with the other representing H, OH, or F,
one of $R_3$ and $R_4$ represents H with the other representing OH,
$R_5$ represents OH or F, and
$R_6$ represents H or $CH_2OH$;

U represents O, S, or NH; and
V represents an optionally substituted $C_{6-18}$ aromatic hydrocarbon or a straight or branched, optionally substituted $C_{1-18}$ aliphatic hydrocarbon.

**51.** Use according to claim 50, wherein the drug is represented by formula (IV):

A-U-V-X-Z (IV)

wherein

A, U, and V are as defined in claim 1;
X represents a binding group; and
Z represents a drug.

**52.** Use according to claim 51, wherein X is selected from the group consisting of -C(= O)O-, -OC(= O)-, -O(C = O)O-, -C(= O)S-, -SC(= O)-, -C(= O)NH-, -C(= O)N<, -NHC(= O)-, -OC(= O)NH-, -OC(= O)N<, -NHC(= O)O-, -CH = N-, -N = CH-, -O-, -NHC(= O)NH-, -NHC(= O)N<, -NH-, -N<, $OCH_2O$-, -$OCH(CH_3)O$-, -$SCH_2O$-, -$SCH(CH_3)O$-, -$OCH_2S$-, -$OCH(CH_3)S$-, - $OCH_2NH$-, -$OCH(CH_3)NH$-, -$OCH_2N$<, -$OCH(CH_3)N$<, -$NHCH_2O$-, - $NHCH(CH_3)O$-, -$SCH_2NH$-, -$SCH(CH_3)NH$-, -$SCH(CH_3)N$<, -C(= $O)OCH_2O$-, -C(= $O)OCH(CH_3)O$-, -C(= $O)OCH_2NH$-, -C(= $O)OCH(CH_3)NH$-, -C(=$O)OCH_2N$<, -C(=$O)OCH(CH_3)N$<, -C(=$O)OCH_2S$-, -C(=$O)OCH(CH_3)S$-, -$NHCH_2OC$(=O)-, -$NHCH(CH_3)OC$(=O)-, -NHC(= $O)OCH_2O$-, -NHC(= $O)OCH(CH_3)O$-, -C(= $O)OCH_2OC$(= O)NH-, -C(= $O)OCH(CH_3)OC$(= O)NH-, and -C(= 0)-J-O- wherein J represents an amino acid residue.

**53.** Use according to claim 51, wherein X has a structure represented by -$X_1$-$X_2$-$X_3$- wherein $X_1$ and $X_3$ each independently represent a binding group and $X_2$ represents a spacer structure.

**54.** Use according to claim 53, wherein $X_1$ and $X_3$ each represent a binding group selected from the group consisting of -C(= O)O-, -OC(= O)-, -O(C = O)O-, -C(= O)S-, -SC(= O)-, -C(= O)NH-, -C(= O)N<, -NHC(= O)-, -OC(= O)NH-, -OC(= O)N<, -NHC(= O)O-, -CH = N-, -N = CH-, -O-, -NHC(= O)NH-, -NHC(= O)N<, -NH-, -N<, -$OCH_2O$-, -$OCH(CH_3)O$-, -$SCH_2O$-, -$SCH(CH_3)O$-, -$OCH_2S$-, -$OCH(CH_3)S$-, -$OCH_2NH$-, -$OCH(CH_3)NH$-, -$OCH_2N$<, -$OCH(CH_3)N$<, -$NHCH_2O$-, -$NHCH(CH_3)O$-, -$SCH_2NH$-, -$SCH(CH_3)NH$-, -$SCH(CH_3)N$<, -C(=$O)OCH_2O$-, -

$C(=O)OCH(CH_3)O-$, $-C(=O)OCH_2NH-$, $-C(= O)OCH(CH_3)NH-$, $-C(= O)OCH_2N<$, $-C(= O)OCH(CH_3)N<$, $-C(= O)OCH_2S-$, $-C(= O)OCH(CH_3)S-$, $-NHCH_2OC(= O)-$, $-NHCH(CH_3)OC(= O)-$, $-NHC(= O)OCH_2O-$, $-NHC(= O)OCH(CH_3)O-$, $-C(= O)OCH_2OC(= O)NH-$, $-C(= O)OCH(CH_3)OC(= O)NH-$, and -C(= 0)-J-O- wherein J represents an amino acid residue.

**55.** Use according to any one of claims 50 to 54, wherein the glycosyl represented by formula (II) represents D-glucosyl, D-mannosyl, or 2-deoxy-D-glucosyl.

**56.** Use according to any one of claims 50 to 55, wherein V represents a $C_{3-15}$ straight-chain aliphatic hydrocarbon.

**57.** Use according to any one of claims 50 to 56, wherein U represents S or NH.

**58.** Use according to any one of claims 51 to 57, wherein Z represents a diagnostic drug, an examination drug, a contrast medium, a steroidal agent, an anticancer agent, an immunosuppressive agent, an anticoagulant/antiplatelet, an anti-inflammatory drug, an antihypertensive drug, adiuretic, antidiuretic, a physiologically active peptide, a useful gene, or an anti-sense drug.

**59.** Use according to any one of claims 51 to 58, wherein the glycosyl represented by formula (II) is D-glucosyl, D-mannosyl, or 2-deoxy-D-glucosyl, U represents S, V represents a $C_{3-15}$ straight-chain aliphatic hydrocarbon, X represents -C(O)O- or -CO-J-O- wherein J represents an amino acid residue, and Z represents a steroidal agent bound through its hydroxyl to X.

**60.** Use of the drug carrier according to any one of claims 1 to 7, as a chemical modifier for imparting renal selectivity to a drug.

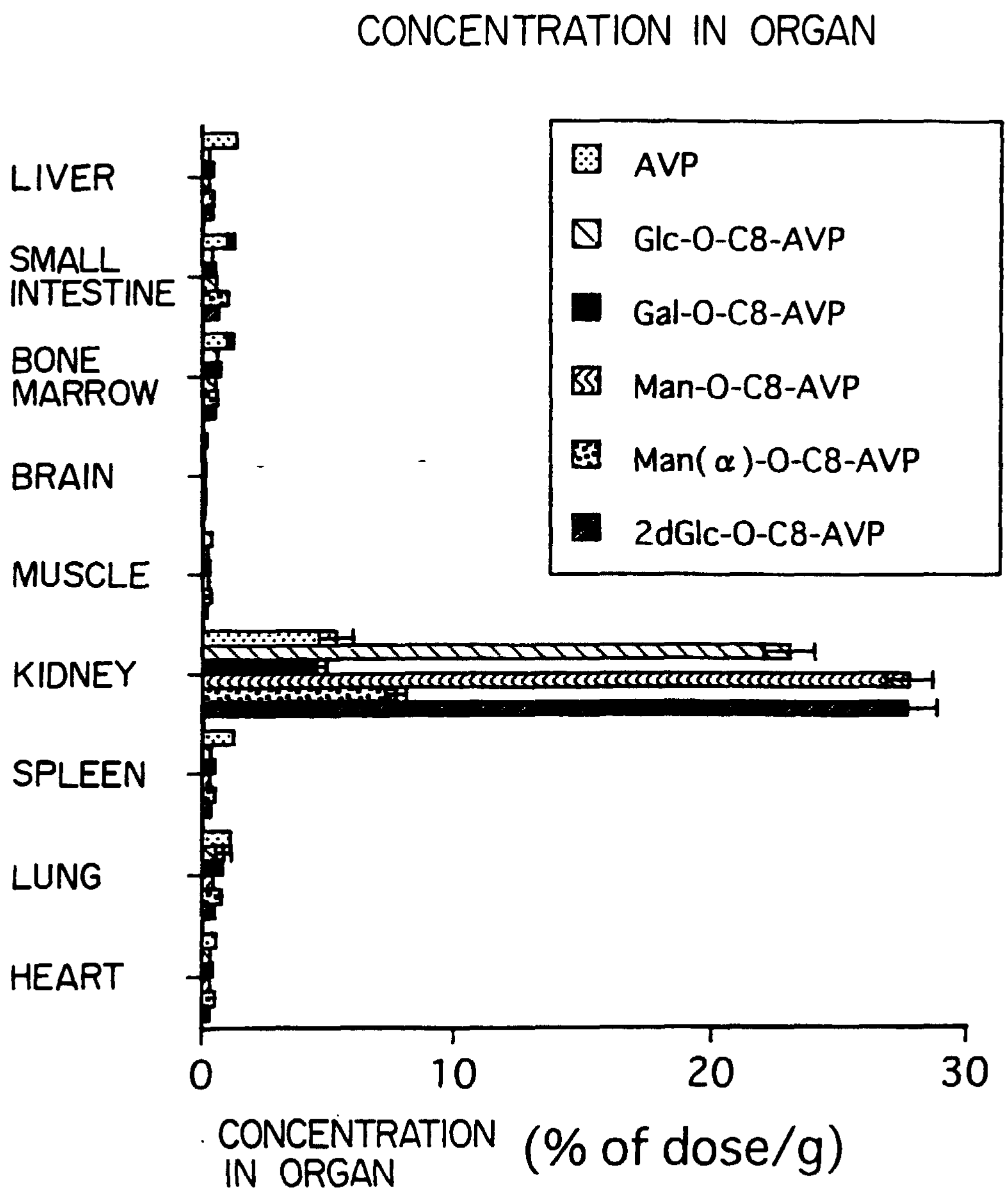
CONCENTRATION IN ORGAN
LIVER
SMALL INTESTINE
BONE MARROW
BRAIN
MUSCLE
KIDNEY
SPLEEN
LUNG
HEART
AVP
Glc-O-C8-AVP
Gal-O-C8-AVP
Man-O-C8-AVP
Man(α)-O-C8-AVP
2dGlc-O-C8-AVP
0
10
20
30
CONCENTRATION IN ORGAN (% of dose/g)

F I G. 1

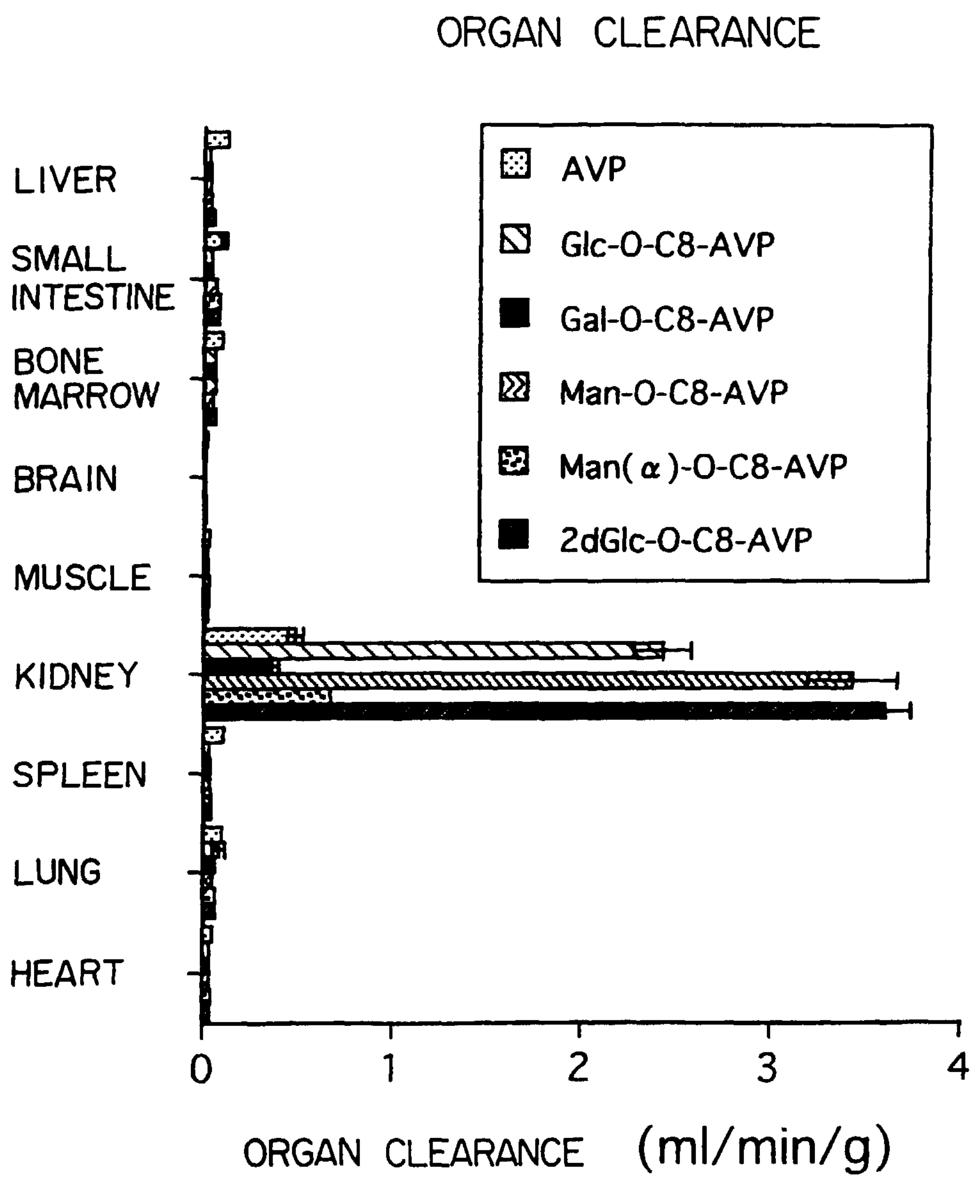
ORGAN CLEARANCE
LIVER
SMALL INTESTINE
BONE MARROW
BRAIN
MUSCLE
KIDNEY
SPLEEN
LUNG
HEART
AVP
Glc-O-C8-AVP
Gal-O-C8-AVP
Man-O-C8-AVP
Man(α)-O-C8-AVP
2dGlc-O-C8-AVP
0
1
2
3
4
ORGAN CLEARANCE (ml/min/g)

FIG. 2

## INTERNATIONAL SEARCH REPORT

International application No. PCT/JP97/03642

**A. CLASSIFICATION OF SUBJECT MATTER**

Int. $Cl^6$ A61K47/26

According to International Patent Classification (IPC) or to both national classification and IPC

**B. FIELDS SEARCHED**

Minimum documentation searched (classification system followed by classification symbols)

Int. $Cl^6$ A61K47/26

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 57-181095, A (Merck & Co.), November 8, 1982 (08. 11. 82) & US, 4386026, A & EP, 63373, A | 1 - 60 |
| A | JP, 3-2875454, A (Research Development Corp. of Japan), December 18, 1991 (18. 12. 91)(Family: none) | 1 - 60 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| November 26, 1997 (26. 11. 97) | December 9, 1997 (09. 12. 97) |
| Name and mailing address of the ISA/ Japanese Patent Office | Authorized officer |
| Facsimile No. | Telephone No. |

Form PCT/ISA/210 (second sheet) (July 1992)